# EUROPEAN PATENT APPLICATION

(11) **EP 4 653 433 A1**
(43) Date of publication of application: **26.11.2025**
(21) Application number: 24744397.1
(22) Date of filing: 19.01.2024
(51) Int. Cl.: C07D 405/14, C07D 405/04, A61K 31/506, A61P 35/00

(54) **CRYSTAL FORM OF ALK INHIBITOR OR SALT AND SOLVATE THEREOF, PREPARATION METHOD THEREFOR, AND USE THEREOF**

(30) Priority: 20.01.2023 CN 202310073722; 05.01.2024 CN 202410021102
(71) Applicant: Ascentage Pharma (Suzhou) Co., Ltd., Suzhou, Jiangsu 215127 (CN); Ascentage Pharma Group Corp Limited, Hong Kong 999077 (CN)
(72) Inventor: LIN, Yanqiong, Suzhou, Jiangsu 215127 (CN); LI, Weidong, Suzhou, Jiangsu 215127 (CN); WU, Yanan, Suzhou, Jiangsu 215127 (CN); WU, Tianzhu, Suzhou, Jiangsu 215127 (CN)
(74) Representative: AWA Denmark A/S
(86) International application number: PCT/CN2024/073239
(87) International publication number: WO 2024/153228

(57) **Abstract**

Provided is a crystal form of a compound represented by formula I or a salt thereof, wherein the crystal form is one equivalent of mesylate crystal form A of the compound represented by formula I, crystal form D of the compound represented by formula I, one equivalent of maleate crystal form A of the compound represented by formula I, one equivalent of tartrate crystal form C of the compound represented by formula I, one equivalent of citrate crystal form B of the compound represented by formula I, or one equivalent of succinate crystal form D of the compound represented by formula I. The crystal form has one or more of the following advantages: high crystallinity, good stability, low hygroscopicity, high solubility, and high bioavailability.

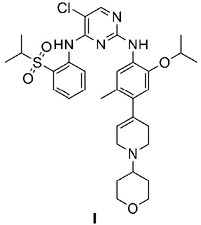

## Description

The present application claims priority to Chinese Patent Application No. 2023100737229 filed on January 20, 2023 and Chinese Patent Application No. 2024100211025 filed on January 5, 2024, which are incorporated herein by reference in their entirety.

### TECHNICAL FIELD

The present disclosure relates to the field of pharmaceutical chemistry, and particularly to a crystal form of an ALK inhibitor or a salt or solvate thereof, a preparation method therefor, and use thereof.

### BACKGROUND

The compound represented by formula I (APG-2449) is disclosed in WO2018044767A1. APG-2449 is a potent focal adhesion kinase (FAK)/ROS1/anaplastic lymphoma kinase (ALK) triple-kinase inhibitor with high oral bioavailability for treating cancers. APG-2449 exhibits anti-tumor activity in multiple xenograft tumor models. Preclinical tumor model studies have shown that APG-2449 can overcome resistance developed by the first-generation ALK inhibitor and shows a synergistic effect with EGFR inhibitors (particularly the newly approved third-generation EGFR inhibitor, AZD9291/osimertinib) in EGFRT790M-mutant NSCLC xenograft tumor models.

### SUMMARY

In order to overcome the defects of low bioavailability, poor stability, relatively high hygroscopicity, and poor solubility of an amorphous form of the compound represented by formula I, provided is a crystal form of an ALK inhibitor or a salt or solvate thereof. The crystal form has one or more of the following advantages: high crystallinity, good stability, low hygroscopicity, high solubility, and high bioavailability.

The present disclosure solves the above technical problems by the following technical solutions.

The present disclosure provides a crystal form A of the compound represented by formula I, and an X-ray powder diffraction (XRPD) pattern of the crystal form has at least three, at least four, at least five, at least six, or at least seven characteristic peaks at the following 2θ angles: 5.494°±0.2°, 7.553°±0.2°, 8.711°±0.2°, 15.6°±0.2°, 17.183°±0.2°, 18.019°±0.2°, and 18.999°±0.2°;

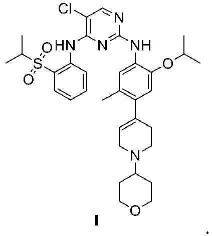

In one embodiment, the X-ray powder diffraction pattern of the crystal form A, measured using Cu-Kα radiation, has diffraction peaks at the following 2θ positions: 18.999°±0.2°, 5.494°±0.2°, 6.041°±0.2°, 6.451°±0.2°, 7.553°±0.2°, 8.711°±0.2°, 9.076°±0.2°, 10.588°±0.2°, 10.974°±0.2°, 11.195°±0.2°, 12.068°±0.2°, 12.252°±0.2°, 12.56°±0.2°, 13.139°±0.2°, 13.795°±0.2°, 13.975°±0.2°, 14.895°±0.2°, 15.6°±0.2°, 15.794°±0.2°, 16.269°±0.2°, 16.46°±0.2°, 16.635°±0.2°, 17.183°±0.2°, 17.445°±0.2°, 18.019°±0.2°, 18.228°±0.2°, 18.525°±0.2°, 19.240°±0.2°, 19.438°±0.2°, and 19.659°±0.2°.

In one embodiment, X-ray powder diffraction pattern analysis data of the crystal form A are substantially as shown in the following table:

| Position [°2θ]±0.2° | d-spacing [Å] | Relative intensity [%] | Position [°2θ]±0.2° | d-spacing [Å] | Relative intensity [%] |
|---|---|---|---|---|---|
| 18.999 | 4.6673 | 100.00% | 13.975 | 6.3318 | 6.60% |
| 5.494 | 16.0739 | 30.40% | 14.895 | 5.9428 | 12.80% |
| 6.041 | 14.6188 | 7.00% | 15.6 | 5.6757 | 32.30% |
| 6.451 | 13.691 | 14.20% | 15.794 | 5.6066 | 10.80% |
| 7.553 | 11.6955 | 72.90% | 16.269 | 5.4438 | 12.80% |
| 8.711 | 10.1426 | 40.00% | 16.46 | 5.3811 | 25.60% |
| 9.076 | 9.7354 | 4.80% | 16.635 | 5.325 | 9.90% |
| 10.588 | 8.3488 | 2.60% | 17.183 | 5.1563 | 76.50% |
| 10.974 | 8.056 | 28.40% | 17.445 | 5.0795 | 20.30% |
| 11.195 | 7.8976 | 7.10% | 18.019 | 4.9188 | 46.60% |
| 12.068 | 7.3278 | 25.80% | 18.228 | 4.8629 | 7.40% |
| 12.252 | 7.2183 | 4.40% | 18.525 | 4.7857 | 9.30% |
| 12.56 | 7.0421 | 14.80% | 19.24 | 4.6095 | 3.50% |
| 13.139 | 6.733 | 11.20% | 19.438 | 4.563 | 7.60% |
| 13.795 | 6.4143 | 12.70% | 19.659 | 4.5121 | 3.40% |

In one embodiment, a thermogravimetric analysis (TGA) curve of the crystal form A shows a weight loss of 0.08556% during heating from 37.52 °C to 150.30 °C.

In one embodiment, a differential scanning calorimetry (DSC) curve of the crystal form A has endothermic peaks with initial temperatures of 109.50 °C and 154.49 °C, respectively.

In one embodiment, the differential scanning calorimetry (DSC) curve of the crystal form A has endothermic peaks with peak temperatures of 117.55 °C and 157.12 °C, respectively.

The present disclosure provides a crystal form B of the compound represented by formula I, and an X-ray powder diffraction (XRPD) pattern of the crystal form has at least three, at least four, at least five, at least six, or at least seven characteristic peaks at the following 2θ angles: 10.879°+0.2°, 15.66°±0.2°, 16.036°±0.2°, 17.28°±0.2°, 18.279°±0.2°, 19.905°±0.2°, and 22.381°±0.2°;

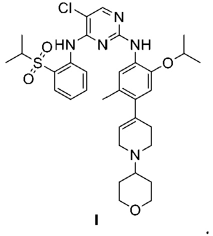

In one embodiment, the X-ray powder diffraction pattern of the crystal form B, measured using Cu-Kα radiation, has diffraction peaks at the following 2θ positions: 5.099+0.2°, 5.677+0.2°, 8.014+0.2°, 8.616+0.2°, 9.246±0.2°, 9.660±0.2°, 10.362±0.2°, 10.879±0.2°, 11.416±0.2°, 11.652±0.2°, 12.402±0.2°, 12.923±0.2°, 13.26±0.2°, 13.779±0.2°, 14.521±0.2°, 15.24±0.2°, 15.66±0.2°, 16.036±0.2°, 16.582±0.2°, 17.042±0.2°, 17.28±0.2°, 18.279±0.2°, 19.098+0.2°, 19.402+0.2°, 19.905+0.2°, 20.558+0.2°, 21.142+0.2°, 21.686+0.2°, 22.087±0.2°, and 22.381±0.2°.

In one embodiment, X-ray powder diffraction pattern analysis data of the crystal form B are substantially as shown in the following table:

| Position [°2θ]±0.2° | d-spacing [Å] | Relative intensity [%] | Position [°2θ]±0.2° | d-spacing [Å] | Relative intensity [%] |
|---|---|---|---|---|---|
| 5.099 | 17.3157 | 9.00% | 15.24 | 5.809 | 8.50% |
| 5.677 | 15.5563 | 32.30% | 15.66 | 5.6542 | 48.90% |
| 8.014 | 11.024 | 5.70% | 16.036 | 5.5224 | 40.90% |
| 8.616 | 10.2544 | 31.30% | 16.582 | 5.3418 | 9.60% |
| 9.246 | 9.5573 | 2.90% | 17.042 | 5.1985 | 29.00% |
| 9.66 | 9.1483 | 7.60% | 17.28 | 5.1277 | 43.20% |
| 10.362 | 8.53 | 16.30% | 18.279 | 4.8495 | 100.00% |
| 10.879 | 8.1261 | 67.20% | 19.098 | 4.6435 | 16.90% |
| 11.416 | 7.745 | 29.20% | 19.402 | 4.5712 | 8.50% |
| 11.652 | 7.5883 | 15.70% | 19.905 | 4.4569 | 71.20% |
| 12.402 | 7.1314 | 8.00% | 20.558 | 4.3168 | 15.60% |
| 12.923 | 6.8447 | 5.20% | 21.142 | 4.1988 | 13.60% |
| 13.26 | 6.6718 | 13.50% | 21.686 | 4.0947 | 2.30% |
| 13.779 | 6.4215 | 3.80% | 22.087 | 4.0213 | 12.90% |
| 14.521 | 6.095 | 4.30% | 22.381 | 3.9691 | 37.90% |

In one embodiment, a thermogravimetric analysis (TGA) curve of the crystal form B shows a weight loss of 0.6646% during heating from 19.94 °C to 150.30 °C.

In one embodiment, a differential scanning calorimetry (DSC) curve of the crystal form B has endothermic peaks with initial temperatures of 72.96 °C, 147.24 °C, and 157.75 °C, respectively.

In one embodiment, the differential scanning calorimetry (DSC) curve of the crystal form B has endothermic peaks with peak temperatures of 74.95 °C, 149.08 °C, and 159.49 °C, respectively.

The present disclosure provides a crystal form C of the compound represented by formula I, and an X-ray powder diffraction (XRPD) pattern of the crystal form has at least three, at least four, at least five, at least six, or at least seven characteristic peaks at the following 2θ angles: 5.499°±0.2°, 8.914°±0.2°, 13.282°±0.2°, 17.52°±0.2°, 17.84°±0.2°, 21.518°±0.2°, and 22.962°±0.2°;

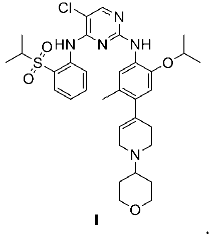

In one embodiment, the X-ray powder diffraction pattern of the crystal form C, measured using Cu-Kα radiation, has diffraction peaks at the following 2θ positions: 5.499°±0.2°, 8.914°±0.2°, 10.981°±0.2°, 11.997°±0.2°, 13.282°±0.2°, 13.526°±0.2°, 13.778°±0.2°, 14.294°±0.2°, 14.622°±0.2°, 15.38°±0.2°, 16.043°±0.2°, 16.236°±0.2°, 16.923°±0.2°, 17.52°±0.2°, 17.84°±0.2°, 18.299°±0.2°, 18.739°±0.2°, 19.601°±0.2°, 19.916°±0.2°, 20.038°±0.2°, 20.222°±0.2°, 20.777°±0.2°, 21.518°±0.2°, 21.743°±0.2°, 22.017°±0.2°, 22.362°±0.2°, 22.801°±0.2°, 22.962°±0.2°, 23.24°±0.2°, and 23.579°±0.2°.

In one embodiment, X-ray powder diffraction pattern analysis data of the crystal form C are substantially as shown in the following table:

| Position [°2θ]±0.2° | d-spacing [Å] | Relative intensity [%] | Position [°2θ]±0.2° | d-spacing [Å] | Relative intensity [%] |
|---|---|---|---|---|---|
| 5.499 | 16.0583 | 100.00% | 18.299 | 4.8443 | 12.20% |
| 8.914 | 9.9121 | 30.00% | 18.739 | 4.7314 | 22.40% |
| 10.981 | 8.0508 | 2.00% | 19.601 | 4.5252 | 2.00% |
| 11.997 | 7.3709 | 29.90% | 19.916 | 4.4544 | 6.60% |
| 13.282 | 6.6607 | 51.20% | 20.038 | 4.4275 | 16.50% |
| 13.526 | 6.5411 | 7.30% | 20.222 | 4.3877 | 20.60% |
| 13.778 | 6.4222 | 5.90% | 20.777 | 4.2718 | 20.60% |
| 14.294 | 6.1912 | 19.40% | 21.518 | 4.1262 | 43.20% |
| 14.622 | 6.0533 | 9.00% | 21.743 | 4.084 | 3.00% |
| 15.38 | 5.7564 | 27.80% | 22.017 | 4.0338 | 14.40% |
| 16.043 | 5.5201 | 6.00% | 22.362 | 3.9724 | 1.90% |
| 16.236 | 5.4548 | 15.40% | 22.801 | 3.8969 | 14.40% |
| 16.923 | 5.2349 | 1.20% | 22.962 | 3.8699 | 52.70% |
| 17.52 | 5.0578 | 49.10% | 23.24 | 3.8242 | 16.90% |
| 17.84 | 4.968 | 40.10% | 23.579 | 3.7701 | 15.20% |

In one embodiment, a thermogravimetric analysis (TGA) curve of the crystal form C shows a weight loss of 0.02699% during heating from 37.52 °C to 150.30 °C.

In one embodiment, a differential scanning calorimetry (DSC) curve of the crystal form C has an endothermic peak with an initial temperature of 157.61 °C.

In one embodiment, the differential scanning calorimetry (DSC) curve of the crystal form C has an endothermic peak with a peak temperature of 158.64 °C.

In one embodiment, a dynamic vapor sorption (DVS) curve of the crystal form C shows a hygroscopic weight gain of 0.31±0.005% at 25 °C and 80% RH.

The present disclosure provides a crystal form D of the compound represented by formula I, and an X-ray powder diffraction (XRPD) pattern of the crystal form has at least three, at least four, at least five, at least six, or at least seven characteristic peaks at the following 2θ angles: 11.539°±0.2°, 12.658°±0.2°, 13.558°±0.2°, 18.081°±0.2°, 18.859°±0.2°, 20.278°±0.2°, and 23.157°±0.2°;

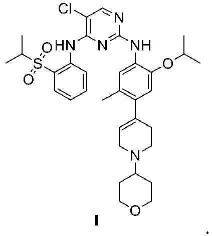

In one embodiment, the X-ray powder diffraction pattern of the crystal form D, measured using Cu-Kα radiation, has diffraction peaks at the following 2θ positions: 18.081°±0.2°, 20.278°±0.2°, and 23.157°±0.2°. In one embodiment, the X-ray powder diffraction pattern of the crystal form D, measured using Cu-Kα radiation, has diffraction peaks at the following 2θ positions: 13.558°±0.2°, 18.081°±0.2°, 20.278°+0.2°, and 23.157°±0.2°.

In one embodiment, the X-ray powder diffraction pattern of the crystal form D, measured using Cu-Kα radiation, has diffraction peaks at the following 2θ positions: 13.558°±0.2°, 18.081°±0.2°, 18.859°±0.2°, 20.278°±0.2°, and 23.157°±0.2°.

In one embodiment, the X-ray powder diffraction pattern of the crystal form D, measured using Cu-Kα radiation, has diffraction peaks at the following 2θ positions: 12.658°±0.2°, 13.558°±0.2°, 18.081°±0.2°, 18.859°±0.2°, 20.278°+0.2°, and 23.157°±0.2°.

In one embodiment, the X-ray powder diffraction pattern of the crystal form D, measured using Cu-Kα radiation, has diffraction peaks at the following 2θ positions: 11.539°±0.2°, 12.658°±0.2°, 13.558°±0.2°, 18.081°+0.2°, 18.859°+0.2°, 20.278°+0.2°, and 23.157°±0.2°.

In one embodiment, the X-ray powder diffraction pattern of the crystal form D, measured using Cu-Kα radiation, has diffraction peaks at the following 2θ positions: 6.297°±0.2°, 6.737°±0.2°, 11.097°±0.2°, 11.539°±0.2°, 12.381°±0.2°, 12.658°±0.2°, 12.837°±0.2°, 13.558°±0.2°, 14.102°±0.2°, 14.996°±0.2°, 15.66°±0.2°, 16.359°±0.2°, 16.522°±0.2°, 17.04°±0.2°, 17.2°±0.2°, 17.416°±0.2°, 18.081°±0.2°, 18.32°±0.2°, 18.515°±0.2°, 18.859°±0.2°, 19.501°±0.2°, 20.278°±0.2°, 20.541°±0.2°, 20.7°±0.2°, 20.937°±0.2°, 21.179°±0.2°, 21.801°±0.2°, 22.057°±0.2°, 22.543°±0.2°, and 23.157°±0.2°.

In one embodiment, X-ray powder diffraction pattern analysis data of the crystal form D are substantially as shown in the following table:

| Position [°2θ]±0.2° | d-spacing [Å] | Relative intensity [%] | Position [°2θ]±0.2° | d-spacing [Å] | Relative intensity [%] |
|---|---|---|---|---|---|
| 6.297 | 14.0244 | 2.10% | 17.416 | 5.0879 | 18.30% |
| 6.737 | 13.1091 | 14.50% | 18.081 | 4.9022 | 56.10% |
| 11.097 | 7.9668 | 5.70% | 18.32 | 4.8387 | 13.80% |
| 11.539 | 7.6629 | 23.70% | 18.515 | 4.7882 | 17.30% |
| 12.381 | 7.1434 | 3.70% | 18.859 | 4.7018 | 29.30% |
| 12.658 | 6.9878 | 25.90% | 19.501 | 4.5483 | 7.80% |
| 12.837 | 6.8905 | 16.40% | 20.278 | 4.3758 | 100.00% |
| 13.558 | 6.5255 | 40.90% | 20.541 | 4.3203 | 7.50% |
| 14.102 | 6.2751 | 8.90% | 20.7 | 4.2876 | 5.10% |
| 14.996 | 5.9031 | 1.20% | 20.937 | 4.2394 | 5.10% |
| 15.66 | 5.6541 | 17.90% | 21.179 | 4.1916 | 7.40% |
| 16.359 | 5.4142 | 19.70% | 21.801 | 4.0734 | 12.00% |
| 16.522 | 5.361 | 15.40% | 22.057 | 4.0267 | 5.70% |
| 17.04 | 5.1992 | 17.90% | 22.543 | 3.9409 | 2.20% |
| 17.2 | 5.1513 | 15.30% | 23.157 | 3.8379 | 41.80% |

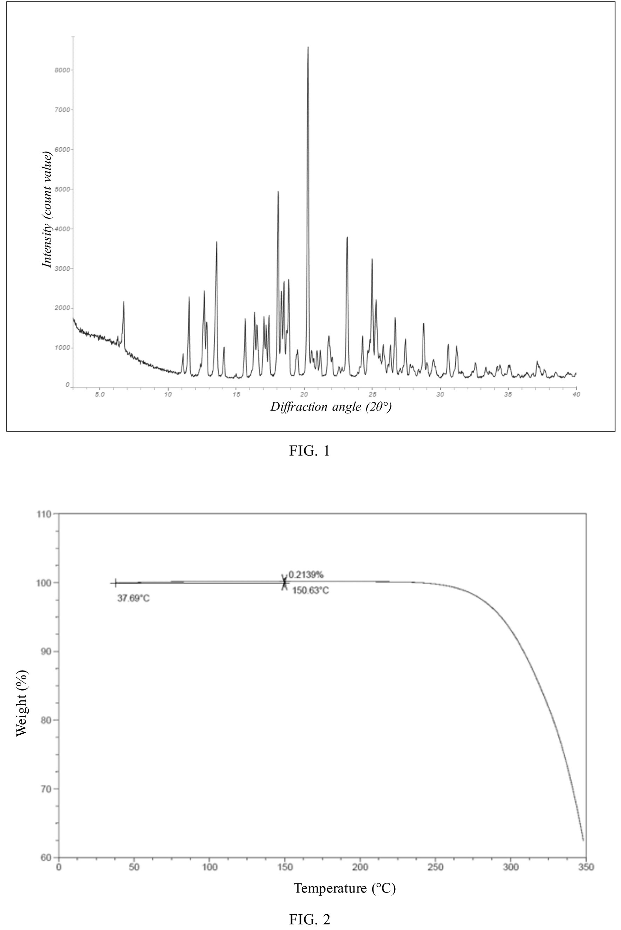

In one embodiment, the X-ray powder diffraction pattern of the crystal form D is substantially as shown in FIG. 1.

In one embodiment, a thermogravimetric analysis (TGA) curve of the crystal form D shows a weight loss of 0.2139±0.005% during heating from 37.69±3 °C to 150.63±3 °C.

In one embodiment, the thermogravimetric analysis (TGA) curve of the crystal form D shows a weight loss of 0.2139% during heating from 37.69 °C to 150.63 °C.

In one embodiment, the thermogravimetric analysis curve of the crystal form D is substantially as shown in FIG. 2.

In one embodiment, a differential scanning calorimetry (DSC) curve of the crystal form D has an endothermic peak with an initial temperature of 165.58±3 °C.

In one embodiment, the differential scanning calorimetry (DSC) curve of the crystal form D has an endothermic peak with an initial temperature of 165.58 °C.

In one embodiment, the differential scanning calorimetry (DSC) curve of the crystal form D has an endothermic peak with a peak temperature of 167.41±3 °C.

In one embodiment, the differential scanning calorimetry (DSC) curve of the crystal form D has an endothermic peak with a peak temperature of 167.41 °C.

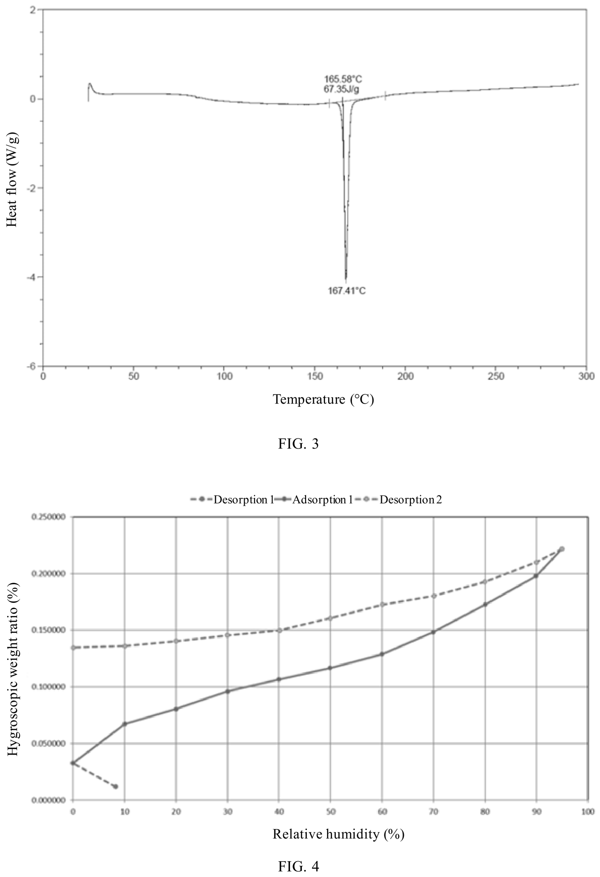

In one embodiment, the differential scanning calorimetry curve of the crystal form D is substantially as shown in FIG. 3.

In one embodiment, a dynamic vapor sorption (DVS) curve of the crystal form D shows a hygroscopic weight gain of 0. 14±0.005% at 25 °C and 80% RH.

In one embodiment, the dynamic vapor sorption (DVS) curve of the crystal form D shows a hygroscopic weight gain of 0.14% at 25 °C and 80% RH.

In one embodiment, the dynamic vapor sorption curve of the crystal form D is substantially as shown in FIG. 4. The present disclosure further provides a preparation method for the crystal form D of the compound represented by formula I, preferably any one of the following methods:
method I comprising the following steps: stirring an amorphous form of the compound represented by formula I in a solvent to precipitate a solid, so as to obtain the crystal form D of the compound represented by formula I, wherein the solvent is an organic solvent or a mixed solvent of an organic solvent and water; the organic solvent may be ethanol, isopropanol, acetonitrile, ethyl acetate, isopropyl acetate, methyl *tert*-butyl ether, toluene, n-heptane, dioxane, methyl acrylate, 2-methyltetrahydrofuran, cyclohexane, *n*-hexane, butanone, or methyl isobutyl ketone; when the organic solvent is ethanol, isopropanol, or dioxane, the stirring is performed at 50 °C; when the organic solvent is methyl *tert*-butyl ether or toluene, the stirring is performed at room temperature, e.g., 25 °C; when the solvent is a mixed solvent of an organic solvent and water, acetonitrile, ethyl acetate, isopropyl acetate, *n*-heptane, methyl acrylate, 2-methyltetrahydrofuran, cyclohexane, n-hexane, butanone, or methyl isobutyl ketone, the stirring is preferably performed at 25-50 °C; the stirring is preferably performed for a period of 2-4 days; the amorphous form of the compound represented by formula I and the solvent are preferably in a mass-to-volume ratio of (50 mg-500 mg):1 mL;
method II comprising the following steps: stirring an amorphous form of the compound represented by formula I in a good solvent to obtain a clear solution, adding a poor solvent, and stirring the mixture to precipitate a solid, so as to obtain the crystal form D of the compound represented by formula I, wherein the good solvent is tetrahydrofuran or dichloromethane; when the good solvent is tetrahydrofuran, the poor solvent is acetonitrile or methyl *tert*-butyl ether; when the good solvent is dichloromethane, the poor solvent is acetonitrile, methyl *tert*-butyl ether, ethyl acetate, or *n*-heptane; the stirring is preferably performed at room temperature; the stirring is preferably performed for a period of 3-5 days; the compound represented by formula I and the good solvent are preferably in a mass-to-volume ratio of (50 mg-500 mg):1 mL; the good solvent and the poor solvent are preferably in a volume ratio of 1:(4-5);
method III comprising the following steps: stirring an amorphous form of the compound represented by formula I in a solvent until a clear solution is obtained, and volatilizing the solution to obtain the crystal form D of the compound represented by formula I, wherein the solvent may be acetone, ethyl acetate, a mixed solvent of acetonitrile and dichloromethane, or a mixed solvent of isopropyl acetate and dichloromethane; the volatilizing is preferably performed at room temperature, e.g., 25 °C; the compound represented by formula I and the solvent are preferably in a mass-to-volume ratio of (10 mg-50 mg):1 mL, and more preferably 20 mg:1 mL; acetonitrile and dichloromethane are preferably in a volume ratio of 10:3; isopropyl acetate and dichloromethane are preferably in a volume ratio of 5:1; and
method IV comprising the following steps: stirring an amorphous form of the compound represented by formula I in a solvent at 50 °C until a clear solution is obtained, and cooling the solution to obtain the crystal form D of the compound represented by formula I, wherein the cooling is performed at 4 °C; the solvent may be tetrahydrofuran, ethyl acetate, a mixed solvent of acetonitrile and dichloromethane, or a mixed solvent of isopropyl acetate and dichloromethane; the compound represented by formula I and the solvent are preferably in a mass-to-volume ratio of (50 mg-500 mg):1 mL, and more preferably 50 mg:1 mL; acetonitrile and dichloromethane are preferably in a volume ratio of 1:1; isopropyl acetate and dichloromethane are preferably in a volume ratio of 1:1.

In one embodiment, in method I, when the solvent is a mixed solvent of an organic solvent and water, acetonitrile, ethyl acetate, isopropyl acetate, n-heptane, methyl acrylate, 2-methyltetrahydrofuran, cyclohexane, n-hexane, butanone, or methyl isobutyl ketone, the stirring is preferably performed at 25 °C or 50 °C; the stirring is preferably performed for a period of 3 days; the compound represented by formula I and the organic solvent are preferably in a mass-to-volume ratio of 83 mg:1 mL or 125 mg:1 mL.

In one embodiment, in method II, the stirring is preferably performed at 25 °C. The stirring is preferably performed for a period of 5 days; the compound represented by formula I and dichloromethane are preferably in a mass-to-volume ratio of 50 mg:1 mL; the good solvent and the poor solvent are preferably in a volume ratio of 1:4.

In one embodiment, the preparation method for the crystal form D may further comprise, after the solid is precipitated, the steps of separation and drying to obtain the crystal form D.

The present disclosure provides a crystal form E of the compound represented by formula I, and an X-ray powder diffraction (XRPD) pattern of the crystal form has at least three, at least four, at least five, at least six, or at least seven characteristic peaks at the following 2θ angles: 7.193°±0.2°, 14.94°±0.2°, 17.818°±0.2°, 19.801°±0.2°, 21.478°±0.2°, 22.297°±0.2°, and 26.062°±0.2°;

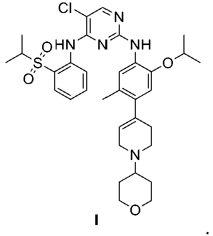

In one embodiment, the X-ray powder diffraction pattern of the crystal form E, measured using Cu-Kα radiation, has diffraction peaks at the following 2θ positions: 6.736°±0.2°, 7.193°±0.2°, 9.216°±0.2°, 9.799°±0.2°, 10.436°±0.2°, 12.396°±0.2°, 12.811°±0.2°, 13.459°±0.2°, 13.656°±0.2°, 14.438°±0.2°, 14.94°±0.2°, 15.098°±0.2°, 16.66°±0.2°, 17.061°±0.2°, 17.818°±0.2°, 18.442°±0.2°, 18.621°±0.2°, 19.801°±0.2°, 20.021°±0.2°, 20.217°±0.2°, 20.717°±0.2°, 21.097°±0.2°, 21.478°±0.2°, 21.84°±0.2°, 22.297°±0.2°, 23.377°±0.2°, 24.256°±0.2°, 26.062°±0.2°, 26.361°±0.2°, and 27.14°±0.2°.

In one embodiment, X-ray powder diffraction pattern analysis data of the crystal form E are substantially as shown in the following table:

| Position [°2θ]±0.2° | d-spacing [Å] | Relative intensity [%] | Position [°2θ]±0.2° | d-spacing [Å] | Relative intensity [%] |
|---|---|---|---|---|---|
| 6.736 | 13.1113 | 11.60% | 18.442 | 4.8071 | 32.90% |
| 7.193 | 12.2802 | 47.20% | 18.621 | 4.7612 | 27.00% |
| 9.216 | 9.5886 | 43.80% | 19.801 | 4.4802 | 46.80% |
| 9.799 | 9.0186 | 9.70% | 20.021 | 4.4314 | 21.90% |
| 10.436 | 8.4702 | 16.30% | 20.217 | 4.3887 | 24.60% |
| 12.396 | 7.1348 | 15.20% | 20.717 | 4.284 | 43.20% |
| 12.811 | 6.9047 | 8.00% | 21.097 | 4.2077 | 7.10% |
| 13.459 | 6.5736 | 14.80% | 21.478 | 4.134 | 70.60% |
| 13.656 | 6.4789 | 20.60% | 21.84 | 4.0662 | 6.00% |
| 14.438 | 6.1297 | 3.20% | 22.297 | 3.9838 | 100.00% |
| 14.94 | 5.9248 | 90.00% | 23.377 | 3.8023 | 12.40% |
| 15.098 | 5.8633 | 34.50% | 24.256 | 3.6664 | 18.50% |
| 16.66 | 5.317 | 29.40% | 26.062 | 3.4163 | 53.50% |
| 17.061 | 5.1929 | 16.40% | 26.361 | 3.3782 | 4.00% |
| 17.818 | 4.9741 | 64.90% | 27.14 | 3.283 | 40.00% |

In one embodiment, a thermogravimetric analysis (TGA) curve of the crystal form E shows a weight loss of 0.1142% during heating from 36.80 °C to 100.21 °C.

In one embodiment, a differential scanning calorimetry (DSC) curve of the crystal form E has endothermic peaks with initial temperatures of 105.44 °C, 121.48 °C, 155.87 °C, and 166.05 °C, respectively.

In one embodiment, the differential scanning calorimetry (DSC) curve of the crystal form E has endothermic peaks with peak temperatures of 112.13 °C, 126.80 °C, 158.60 °C, and 167.53 °C, respectively.

The present disclosure provides a crystal form F of the compound represented by formula I, and an X-ray powder diffraction (XRPD) pattern of the crystal form has at least three, at least four, at least five, at least six, or at least seven characteristic peaks at the following 2θ angles: 8.976°±0.2°, 13.278°±0.2°, 17.622°±0.2°, 18.001°±0.2°, 19.982°±0.2°, 20.858°±0.2°, and 21.12°±0.2°;

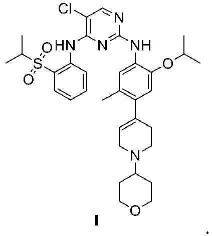

In one embodiment, the X-ray powder diffraction pattern of the crystal form F, measured using Cu-Kα radiation, has diffraction peaks at the following 2θ positions: 6.468°±0.2°, 8.976°±0.2°, 9.22°±0.2°, 9.917°±0.2°, 12.432°±0.2°, 13.006°±0.2°, 13.278°±0.2°, 13.794°±0.2°, 14.088°±0.2°, 14.639°±0.2°, 14.981°±0.2°, 15.257°±0.2°, 15.803°±0.2°, 16.373°±0.2°, 16.576°±0.2°, 17.622°±0.2°, 18.001°±0.2°, 18.505°±0.2°, 18.7°±0.2°, 19.661°±0.2°, 19.982°±0.2°, 20.581°±0.2°, 20.858°±0.2°, 21.12°±0.2°, 22.769°±0.2°, 23.077°±0.2°, 23.433°±0.2°, 24.399°±0.2°, 24.844°±0.2°, and 25.201°±0.2°.

In one embodiment, X-ray powder diffraction pattern analysis data of the crystal form F are substantially as shown in the following table:

| Position [°2θ]±0.2° | d-spacing [Å] | Relative intensity [%] | Position [°2θ]±0.2° | d-spacing [Å] | Relative intensity [%] |
|---|---|---|---|---|---|
| 6.468 | 13.6552 | 9.30% | 17.622 | 5.0289 | 26.10% |
| 8.976 | 9.8441 | 36.50% | 18.001 | 4.9239 | 83.60% |
| 9.22 | 9.5836 | 6.20% | 18.505 | 4.7908 | 6.20% |
| 9.917 | 8.9121 | 16.50% | 18.7 | 4.7412 | 10.00% |
| 12.432 | 7.1139 | 5.30% | 19.661 | 4.5116 | 19.80% |
| 13.006 | 6.8014 | 6.50% | 19.982 | 4.44 | 54.10% |
| 13.278 | 6.6627 | 35.80% | 20.581 | 4.3121 | 11.90% |
| 13.794 | 6.4144 | 20.00% | 20.858 | 4.2554 | 100.00% |
| 14.088 | 6.2814 | 3.70% | 21.12 | 4.2031 | 24.50% |
| 14.639 | 6.0461 | 19.60% | 22.769 | 3.9024 | 6.60% |
| 14.981 | 5.909 | 3.20% | 23.077 | 3.851 | 14.50% |
| 15.257 | 5.8027 | 4.80% | 23.433 | 3.7932 | 3.80% |
| 15.803 | 5.6035 | 18.30% | 24.399 | 3.6453 | 1.90% |
| 16.373 | 5.4097 | 8.10% | 24.844 | 3.581 | 9.60% |
| 16.576 | 5.3437 | 8.20% | 25.201 | 3.5311 | 5.60% |

In one embodiment, a thermogravimetric analysis (TGA) curve of the crystal form F shows a weight loss of 8.120% during heating from 36.87 °C to 200.38 °C.

In one embodiment, a differential scanning calorimetry (DSC) curve of the crystal form F has endothermic peaks with initial temperatures of 93.99 °C and 150.24 °C, respectively.

In one embodiment, the differential scanning calorimetry (DSC) curve of the crystal form F has endothermic peaks with peak temperatures of 100.94 °C and 155.50 °C, respectively.

The present disclosure provides a crystal form G of the compound represented by formula I, and an X-ray powder diffraction (XRPD) pattern of the crystal form has at least three, at least four, at least five, at least six, or at least seven characteristic peaks at the following 2θ angles: 14.776°±0.2°, 17.837°±0.2°, 19.219°±0.2°, 20.317°±0.2°, 21.343°±0.2°, 21.898°±0.2°, and 29.382°±0.2°;

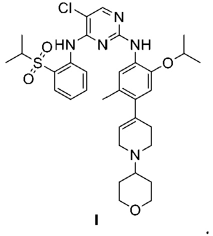

In one embodiment, the X-ray powder diffraction pattern of the crystal form G, measured using Cu-Kα radiation, has diffraction peaks at the following 2θ positions: 6.521°±0.2°, 7.294°±0.2°, 9.039°±0.2°, 9.237°±0.2°, 9.734°±0.2°, 10.459°±0.2°, 12.2°±0.2°, 12.681°±0.2°, 13.037°±0.2°, 13.778°±0.2°, 14.776°±0.2°, 15.095°±0.2°, 15.585°±0.2°, 16.741°±0.2°, 17.093°±0.2°, 17.837°±0.2°, 18.499°±0.2°, 18.799°±0.2°, 19.219°+0.2°, 19.422°+0.2°, 19.581°+0.2°, 19.888°+0.2°, 20.317°+0.2°, 21.093°±0.2°, 21.343°±0.2°, 21.898°±0.2°, 22.374°±0.2°, 22.702°±0.2°, 23.075°±0.2°, and 29.382°±0.2°.

In one embodiment, X-ray powder diffraction pattern analysis data of the crystal form G are substantially as shown in the following table:

| Position [°2θ]±0.2° | d-spacing [Å] | Relative intensity [%] | Position [°2θ]±0.2° | d-spacing [Å] | Relative intensity [%] |
|---|---|---|---|---|---|
| 6.521 | 13.5434 | 24.00% | 17.837 | 4.9686 | 52.40% |
| 7.294 | 12.1099 | 21.50% | 18.499 | 4.7923 | 16.60% |
| 9.039 | 9.7756 | 7.50% | 18.799 | 4.7164 | 23.80% |
| 9.237 | 9.5662 | 27.70% | 19.219 | 4.6145 | 32.30% |
| 9.734 | 9.079 | 3.80% | 19.422 | 4.5667 | 4.30% |
| 10.459 | 8.4516 | 7.00% | 19.581 | 4.53 | 12.70% |
| 12.2 | 7.249 | 19.80% | 19.888 | 4.4608 | 2.00% |
| 12.681 | 6.9749 | 2.30% | 20.317 | 4.3675 | 73.40% |
| 13.037 | 6.7854 | 6.60% | 21.093 | 4.2084 | 6.80% |
| 13.778 | 6.4221 | 14.70% | 21.343 | 4.1598 | 58.10% |
| 14.776 | 5.9905 | 74.00% | 21.898 | 4.0556 | 100.00% |
| 15.095 | 5.8645 | 17.00% | 22.374 | 3.9703 | 9.00% |
| 15.585 | 5.6813 | 1.50% | 22.702 | 3.9137 | 8.60% |
| 16.741 | 5.2915 | 18.40% | 23.075 | 3.8513 | 6.00% |
| 17.093 | 5.1833 | 12.00% | 29.382 | 3.0373 | 33.10% |

In one embodiment, a thermogravimetric analysis (TGA) curve of the crystal form G shows a weight loss of 0.1931% during heating from 37.20 °C to 100.54 °C.

In one embodiment, a differential scanning calorimetry (DSC) curve of the crystal form G has an endothermic peak with an initial temperature of 113.63 °C.

In one embodiment, the differential scanning calorimetry (DSC) curve of the crystal form G has an endothermic peak with a peak temperature of 121.09 °C.

The present disclosure provides a crystal form H of the compound represented by formula I, and an X-ray powder diffraction (XRPD) pattern of the crystal form has at least three, at least four, at least five, at least six, or at least seven characteristic peaks at the following 2θ angles: 5.444°±0.2°, 13.296°±0.2°, 10.885°±0.2°, 17.137°±0.2°, 17.512°±0.2°, 18.275°±0.2°, and 19.998°±0.2°;

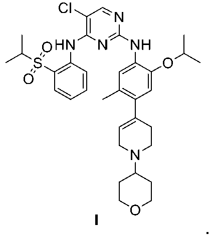

In one embodiment, the X-ray powder diffraction pattern of the crystal form H, measured using Cu-Kα radiation, has diffraction peaks at the following 2θ positions: 5.444°±0.2°, 8.528°±0.2°, 8.909°±0.2°, 9.669°+0.2°, 10.325°+0.2°, 10.885°+0.2°, 11.362°+0.2°, 11.994°+0.2°, 12.391°+0.2°, 13.296°+0.2°, 13.523°±0.2°, 14.24°±0.2°, 15.206°±0.2°, 15.381°±0.2°, 15.479°±0.2°, 15.972°±0.2°, 17.137°±0.2°, 17.512°±0.2°, 17.827°±0.2°, 18.275°±0.2°, 18.79°±0.2°, 19.003°±0.2°, 19.998°±0.2°, 20.774°±0.2°, 21.174°±0.2°, 21.483°±0.2°, 22.357°+0.2°, 22.901°±0.2°, 24.975°±0.2°, and 26.88°±0.2°.

In one embodiment, X-ray powder diffraction pattern analysis data of the crystal form H are substantially as shown in the following table:

| Position [°2θ]±0.2° | d-spacing [Å] | Relative intensity [%] | Position [°2θ]±0.2° | d-spacing [Å] | Relative intensity [%] |
|---|---|---|---|---|---|
| 5.444 | 16.2216 | 70.50% | 15.972 | 5.5445 | 38.30% |
| 8.528 | 10.3606 | 22.20% | 17.137 | 5.1701 | 43.00% |
| 8.909 | 9.918 | 16.00% | 17.512 | 5.0601 | 47.00% |
| 9.669 | 9.1395 | 19.50% | 17.827 | 4.9714 | 17.20% |
| 10.325 | 8.5606 | 34.20% | 18.275 | 4.8506 | 72.30% |
| 10.885 | 8.1215 | 39.60% | 18.79 | 4.7188 | 19.10% |
| 11.362 | 7.7814 | 28.30% | 19.003 | 4.6664 | 27.20% |
| 11.994 | 7.3728 | 31.80% | 19.998 | 4.4365 | 100.00% |
| 12.391 | 7.1376 | 14.80% | 20.774 | 4.2723 | 31.20% |
| 13.296 | 6.6535 | 50.90% | 21.174 | 4.1926 | 18.10% |
| 13.523 | 6.5427 | 24.70% | 21.483 | 4.1329 | 34.50% |
| 14.24 | 6.2147 | 15.90% | 22.357 | 3.9733 | 37.50% |
| 15.206 | 5.8221 | 11.40% | 22.901 | 3.8802 | 25.60% |
| 15.381 | 5.7561 | 38.20% | 24.975 | 3.5624 | 36.70% |
| 15.479 | 5.7198 | 32.90% | 26.88 | 3.3141 | 22.50% |

In one embodiment, a thermogravimetric analysis (TGA) curve of the crystal form H shows a weight loss of 1.110% during heating from 25.58 °C to 150.63 °C.

In one embodiment, a differential scanning calorimetry (DSC) curve of the crystal form H has an endothermic peak with an initial temperature of 138.69 °C.

In one embodiment, the differential scanning calorimetry (DSC) curve of the crystal form H has an endothermic peak with a peak temperature of 150.32 °C.

The present disclosure provides a crystal form I of the compound represented by formula I, and an X-ray powder diffraction (XRPD) pattern of the crystal form has at least three, at least four, at least five, at least six, or at least seven characteristic peaks at the following 2θ angles: 5.5058°±0.2°, 7.5176°±0.2°, 15.4455°±0.2°, 17.2025°±0.2°, 18.8815°±0.2°, 21.9424°±0.2°, and 23.5775°±0.2°;

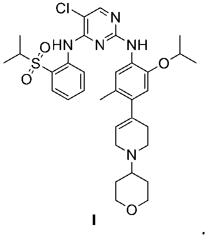

In one embodiment, the X-ray powder diffraction pattern of the crystal form I, measured using Cu-Kα radiation, has diffraction peaks at the following 2θ positions: 4.2926°±0.2°, 4.6616°±0.2°, 5.1103°±0.2°, 5.5058°±0.2°, 6.0446°±0.2°, 7.5176°±0.2°, 8.8154°±0.2°, 10.7679°±0.2°, 11.0145°±0.2°, 12.1007°±0.2°, 12.3584°±0.2°, 13.0245°±0.2°, 13.6613°±0.2°, 14.8321°±0.2°, 15.0219°±0.2°, 15.4455°±0.2°, 16.5217°±0.2°, 17.2025°±0.2°, 17.6832°±0.2°, 17.8573°±0.2°, 18.6876°±0.2°, 18.8815°±0.2°, 21.5792°±0.2°, 21.9424°±0.2°, 22.6197°±0.2°, 23.0634°±0.2°, 23.5775°±0.2°, 23.8621°±0.2°, 24.3147°±0.2°, and 24.7432°±0.2°.

In one embodiment, X-ray powder diffraction pattern analysis data of the crystal form I are substantially as shown in the following table:

| Position [°2θ]±0.2° | d-spacing [Å] | Relative intensity [%] | Position [°2θ]±0.2° | d-spacing [Å] | Relative intensity [%] |
|---|---|---|---|---|---|
| 4.2926 | 20.56787 | 2.20% | 15.4455 | 5.73222 | 32.70% |
| 4.6616 | 18.94076 | 1.70% | 16.5217 | 5.36118 | 5.90% |
| 5.1103 | 17.27852 | 2.60% | 17.2025 | 5.15053 | 29.70% |
| 5.5058 | 16.03831 | 32.10% | 17.6832 | 5.01158 | 8.60% |
| 6.0446 | 14.6098 | 2.40% | 17.8573 | 4.9631 | 5.90% |
| 7.5176 | 11.75013 | 100.00% | 18.6876 | 4.74441 | 12.70% |
| 8.8154 | 10.02298 | 12.60% | 18.8815 | 4.69614 | 32.50% |
| 10.7679 | 8.20956 | 1.10% | 21.5792 | 4.11476 | 12.60% |
| 11.0145 | 8.02625 | 18.20% | 21.9424 | 4.04746 | 28.80% |
| 12.1007 | 7.30817 | 11.10% | 22.6197 | 3.92778 | 4.00% |
| 12.3584 | 7.15633 | 0.80% | 23.0634 | 3.8532 | 3.80% |
| 13.0245 | 6.7918 | 1.40% | 23.5775 | 3.77035 | 50.40% |
| 13.6613 | 6.47661 | 5.10% | 23.8621 | 3.72601 | 1.50% |
| 14.8321 | 5.96788 | 1.00% | 24.3147 | 3.65767 | 4.10% |
| 15.0219 | 5.8929 | 1.60% | 24.7432 | 3.59529 | 0.40% |

In one embodiment, a thermogravimetric analysis (TGA) curve of the crystal form I shows a weight loss of 0.1408% during heating from 46.48 °C to 100.54 °C.

In one embodiment, a differential scanning calorimetry (DSC) curve of the crystal form I has endothermic peaks with initial temperatures of 115.91 °C and 155.71 °C, respectively.

In one embodiment, the differential scanning calorimetry (DSC) curve of the crystal form I has endothermic peaks with peak temperatures of 124.64 °C and 157.93 °C.

The present disclosure provides a crystal form J of the compound represented by formula I, and an X-ray powder diffraction (XRPD) pattern of the crystal form has at least three, at least four, at least five, at least six, or at least seven characteristic peaks at the following 2θ angles: 8.791°±0.2°, 12.203°±0.2°, 16.276°±0.2°, 17.338°±0.2°, 18.118°±0.2°, 18.942°±0.2°, and 20.201°+0.2°;

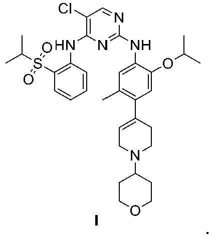

In one embodiment, the X-ray powder diffraction pattern of the crystal form J, measured using Cu-Kα radiation, has diffraction peaks at the following 2θ positions: 5.748°±0.2°, 8.791°±0.2°, 9.049°±0.2°, 9.42°±0.2°, 10.074°±0.2°, 10.882°±0.2°, 11.46°±0.2°, 11.826°±0.2°, 12.203°±0.2°, 13.259°±0.2°, 14.276°±0.2°, 14.694°±0.2°, 14.957°±0.2°, 15.505°±0.2°, 16.276°±0.2°, 17.338°±0.2°, 18.118°±0.2°, 18.942°±0.2°, 19.742°±0.2°, 19.925°±0.2°, 20.201°±0.2°, 20.376°±0.2°, 21.222°±0.2°, 21.389°±0.2°, 21.762°±0.2°, 22.501°±0.2°, 22.863°±0.2°, 23.016°±0.2°, 23.376°±0.2°, and 23.759°±0.2°.

In one embodiment, X-ray powder diffraction pattern analysis data of the crystal form J are substantially as shown in the following table:

| Position [°2θ]±0.2° | d-spacing [Å] | Relative intensity [%] | Position [°2θ]±0.2° | d-spacing [Å] | Relative intensity [%] |
|---|---|---|---|---|---|
| 5.748 | 15.3623 | 9.30% | 17.338 | 5.1105 | 73.50% |
| 8.791 | 10.0509 | 26.40% | 18.118 | 4.8923 | 100.00% |
| 9.049 | 9.7651 | 6.30% | 18.942 | 4.6814 | 43.60% |
| 9.42 | 9.3811 | 2.20% | 19.742 | 4.4934 | 5.10% |
| 10.074 | 8.7735 | 12.40% | 19.925 | 4.4526 | 11.80% |
| 10.882 | 8.1239 | 2.70% | 20.201 | 4.3922 | 18.70% |
| 11.46 | 7.7154 | 5.10% | 20.376 | 4.355 | 12.50% |
| 11.826 | 7.4773 | 6.80% | 21.222 | 4.1832 | 3.10% |
| 12.203 | 7.2473 | 17.70% | 21.389 | 4.1509 | 5.20% |
| 13.259 | 6.6723 | 1.00% | 21.762 | 4.0807 | 11.10% |
| 14.276 | 6.1991 | 8.20% | 22.501 | 3.9483 | 6.60% |
| 14.694 | 6.0236 | 7.00% | 22.863 | 3.8866 | 14.10% |
| 14.957 | 5.9183 | 13.40% | 23.016 | 3.8611 | 13.70% |
| 15.505 | 5.7104 | 9.40% | 23.376 | 3.8025 | 1.80% |
| 16.276 | 5.4416 | 15.40% | 23.759 | 3.742 | 11.30% |

In one embodiment, a thermogravimetric analysis (TGA) curve of the crystal form J shows a weight loss of 0.2556% during heating from 35.20 °C to 150.30 °C.

In one embodiment, a differential scanning calorimetry (DSC) curve of the crystal form J has endothermic peaks with initial temperatures of 132.08 °C and 152.97 °C, respectively.

In one embodiment, the differential scanning calorimetry (DSC) curve of the crystal form J has endothermic peaks with peak temperatures of 137.49 °C and 155.43 °C.

The present disclosure provides a crystal form K of the compound represented by formula I, and an X-ray powder diffraction (XRPD) pattern of the crystal form has at least three, at least four, at least five, at least six, or at least seven characteristic peaks at the following 2θ angles: 6.4564°±0.2°, 7.3943°±0.2°, 9.0755°±0.2°, 12.8967°±0.2°, 14.778°±0.2°, 19.2587°±0.2°, and 22.2392°±0.2°;

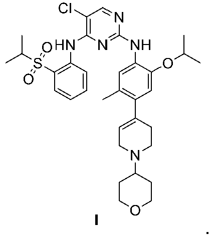

In one embodiment, the X-ray powder diffraction pattern of the crystal form K, measured using Cu-Kα radiation, has diffraction peaks at the following 2θ positions: 6.4564°±0.2°, 7.3943°+0.2°, 9.0755°+0.2°, 10.5562°+0.2°, 11.1454°+0.2°, 11.3993°+0.2°, 12.2643°±0.2°, 12.8967°+0.2°, 14.778°±0.2°, 15.3086°+0.2°, 15.682°±0.2°, 16.3008°±0.2°, 17.4854°±0.2°, 19.2587°±0.2°, 20.2788°±0.2°, 20.703°±0.2°, 21.2376°±0.2°, 21.5385°±0.2°, 21.688°±0.2°, 22.2392°±0.2°, 22.8603°±0.2°, 23.9619°±0.2°, 24.0585°±0.2°, 24.4456°±0.2°, 24.5831°±0.2°, 25.0787°+0.2°, 25.9017°±0.2°, 26.5371°±0.2°, 27.3914°+0.2°, and 28.2004°+0.2°.

In one embodiment, X-ray powder diffraction pattern analysis data of the crystal form K are substantially as shown in the following table:

| Position [°2θ]±0.2° | d-spacing [Å] | Relative intensity [%] | Position [°2θ]±0.2° | d-spacing [Å] | Relative intensity [%] |
|---|---|---|---|---|---|
| 6.4564 | 13.67892 | 100.00% | 20.703 | 4.28689 | 2.10% |
| 7.3943 | 11.94569 | 51.00% | 21.2376 | 4.18016 | 2.40% |
| 9.0755 | 9.73627 | 15.50% | 21.5385 | 4.12245 | 2.10% |
| 10.5562 | 8.37369 | 8.10% | 21.688 | 4.09436 | 0.90% |
| 11.1454 | 7.93229 | 10.20% | 22.2392 | 3.9941 | 29.30% |
| 11.3993 | 7.75621 | 1.40% | 22.8603 | 3.88698 | 8.40% |
| 12.2643 | 7.21102 | 5.90% | 23.9619 | 3.71072 | 5.20% |
| 12.8967 | 6.85882 | 11.00% | 24.0585 | 3.69604 | 4.70% |
| 14.778 | 5.98962 | 21.40% | 24.4456 | 3.63839 | 1.70% |
| 15.3086 | 5.7832 | 1.20% | 24.5831 | 3.61835 | 1.00% |
| 15.682 | 5.64631 | 10.80% | 25.0787 | 3.54795 | 0.80% |
| 16.3008 | 5.43335 | 5.60% | 25.9017 | 3.43705 | 5.80% |
| 17.4854 | 5.06781 | 0.40% | 26.5371 | 3.35618 | 3.10% |
| 19.2587 | 4.60499 | 26.50% | 27.3914 | 3.25342 | 0.40% |
| 20.2788 | 4.3756 | 1.10% | 28.2004 | 3.16189 | 0.90% |

In one embodiment, a thermogravimetric analysis (TGA) curve of the crystal form K shows a weight loss of 0.2766% during heating from 29.61 °C to 100.11 °C.

In one embodiment, a differential scanning calorimetry (DSC) curve of the crystal form K has an endothermic peak with an initial temperature of 112.76 °C.

In one embodiment, the differential scanning calorimetry (DSC) curve of the crystal form K has an endothermic peak with a peak temperature of 116.84 °C.

The present disclosure provides a crystal form L of the compound represented by formula I, and an X-ray powder diffraction (XRPD) pattern of the crystal form has at least three, at least four, at least five, at least six, or at least seven characteristic peaks at the following 2θ angles: 5.513°±0.2°, 13.504°±0.2°, 16.985°±0.2°, 18.045°±0.2°, 18.785°+0.2°, 20.219°±0.2°, and 21.844°±0.2°;

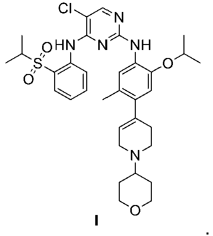

In one embodiment, the X-ray powder diffraction pattern of the crystal form L, measured using Cu-Kα radiation, has diffraction peaks at the following 2θ positions: 5.513°±0.2°, 6.207°±0.2°, 8.909°±0.2°, 11.061°±0.2°, 11.427°±0.2°, 11.96°±0.2°, 12.5°+0.2°, 12.798°±0.2°, 13.28°±0.2°, 13.504°±0.2°, 14.081°±0.2°, 14.547°+0.2°, 15.64°+0.2°, 16.404°+0.2°, 16.985°±0.2°, 17.159°+0.2°, 17.417°±0.2°, 18.045°±0.2°, 18.279°+0.2°, 18.489°+0.2°, 18.785°+0.2°, 19.323°+0.2°, 19.467°+0.2°, 20.219°+0.2°, 20.622°±0.2°, 21.46°±0.2°, 21.844°±0.2°, 22.006°±0.2°, 22.424°±0.2°, and 22.943°±0.2°.

In one embodiment, X-ray powder diffraction pattern analysis data of the crystal form L are substantially as shown in the following table:

| Position [°2θ]±0.2° | d-spacing [Å] | Relative intensity [%] | Position [°2θ]±0.2° | d-spacing [Å] | Relative intensity [%] |
|---|---|---|---|---|---|
| 5.513 | 16.0166 | 84.70% | 17.159 | 5.1635 | 5.80% |
| 6.207 | 14.2275 | 1.60% | 17.417 | 5.0877 | 2.70% |
| 8.909 | 9.918 | 1.30% | 18.045 | 4.912 | 55.10% |
| 11.061 | 7.9927 | 3.90% | 18.279 | 4.8497 | 1.30% |
| 11.427 | 7.7373 | 10.50% | 18.489 | 4.7949 | 1.10% |
| 11.96 | 7.394 | 1.30% | 18.785 | 4.7199 | 87.60% |
| 12.5 | 7.0758 | 16.50% | 19.323 | 4.5899 | 1.70% |
| 12.798 | 6.9113 | 8.40% | 19.467 | 4.5563 | 7.80% |
| 13.28 | 6.6615 | 9.70% | 20.219 | 4.3885 | 36.70% |
| 13.504 | 6.5516 | 100.00% | 20.622 | 4.3035 | 7.40% |
| 14.081 | 6.2844 | 1.50% | 21.46 | 4.1374 | 2.90% |
| 14.547 | 6.0843 | 1.00% | 21.844 | 4.0655 | 20.70% |
| 15.64 | 5.6614 | 7.30% | 22.006 | 4.036 | 8.30% |
| 16.404 | 5.3995 | 15.60% | 22.424 | 3.9616 | 4.00% |
| 16.985 | 5.2161 | 43.20% | 22.943 | 3.8731 | 5.00% |

In one embodiment, a thermogravimetric analysis (TGA) curve of the crystal form L shows a weight loss of 0.2308% during heating from 30.17 °C to 150.15 °C.

In one embodiment, a differential scanning calorimetry (DSC) curve of the crystal form L has an endothermic peak with an initial temperature of 165.90 °C.

In one embodiment, the differential scanning calorimetry (DSC) curve of the crystal form L has an endothermic peak with a peak temperature of 171.33 °C.

The present disclosure provides a crystal form M of the compound represented by formula I, and an X-ray powder diffraction (XRPD) pattern of the crystal form has at least three, at least four, at least five, at least six, or at least seven characteristic peaks at the following 2θ angles: 5.4969°±0.2°, 10.9723°±0.2°, 13.2767°±0.2°, 22°±0.2°, 23.5465°±0.2°, 27.5831°±0.2°, and 33.2405°±0.2°;

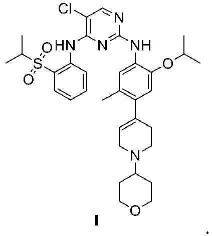

In one embodiment, the X-ray powder diffraction pattern of the crystal form M, measured using Cu-Kα radiation, has diffraction peaks at the following 2θ positions: 4.9624°±0.2°, 5.4969°±0.2°, 5.8171°±0.2°, 5.9372°±0.2°, 8.8974°±0.2°, 10.9723°±0.2°, 11.9937°±0.2°, 13.2767°±0.2°, 16.4617°±0.2°, 22°±0.2°, 22.3562°±0.2°, 22.9591°±0.2°, 23.2262°±0.2°, 23.5465°±0.2°, 27.5831°±0.2°, 28.7603°±0.2°, 28.9834°±0.2°, 33.2405°±0.2°, 34.6165°±0.2°, and 38.9789°±0.2°.

In one embodiment, X-ray powder diffraction pattern analysis data of the crystal form M are substantially as shown in the following table:

| Position [°2θ]±0.2° | d-spacing [Å] | Relative intensity [%] | Position [°2θ]±0.2° | d-spacing [Å] | Relative intensity [%] |
|---|---|---|---|---|---|
| 4.9624 | 17.79317 | 0.20% | 22.3562 | 3.97348 | 0.80% |
| 5.4969 | 16.06424 | 100.00% | 22.9591 | 3.87048 | 1.50% |
| 5.8171 | 15.18074 | 0.30% | 23.2262 | 3.82656 | 0.20% |
| 5.9372 | 14.87375 | 0.20% | 23.5465 | 3.77524 | 5.00% |
| 8.8974 | 9.93076 | 1.00% | 27.5831 | 3.23124 | 4.40% |
| 10.9723 | 8.05707 | 2.30% | 28.7603 | 3.1016 | 0.20% |
| 11.9937 | 7.37309 | 0.20% | 28.9834 | 3.07823 | 1.60% |
| 13.2767 | 6.66332 | 5.80% | 33.2405 | 2.69309 | 8.80% |
| 16.4617 | 5.38058 | 1.00% | 34.6165 | 2.58912 | 0.40% |
| 22 | 4.037 | 2.80% | 38.9789 | 2.30881 | 1.20% |

In one embodiment, a thermogravimetric analysis (TGA) curve of the crystal form M shows a weight loss of 0.02364% during heating from 29.95 °C to 150.15 °C.

In one embodiment, a differential scanning calorimetry (DSC) curve of the crystal form M has an endothermic peak with an initial temperature of 158.11 °C.

In one embodiment, the differential scanning calorimetry (DSC) curve of the crystal form M has an endothermic peak with a peak temperature of 163.24 °C.

The present disclosure provides a crystal form N of the compound represented by formula I, and an X-ray powder diffraction (XRPD) pattern of the crystal form has at least three, at least four, at least five, at least six, or at least seven characteristic peaks at the following 2θ angles: 10.402°±0.2°, 15.619°±0.2°, 17.4°±0.2°, 17.84°±0.2°, 18.404°±0.2°, 20.243°±0.2°, and 22.6°±0.2°;

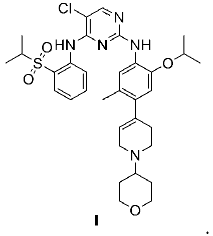

In one embodiment, the X-ray powder diffraction pattern of the crystal form N, measured using Cu-Kα radiation, has diffraction peaks at the following 2θ positions: 5.218°±0.2°, 6.643°±0.2°, 10.402°±0.2°, 10.874°±0.2°, 13.403°±0.2°, 13.583°±0.2°, 13.719°±0.2°, 14.031°±0.2°, 14.354°±0.2°, 14.742°±0.2°, 15.619°±0.2°, 15.96°±0.2°, 16.677°±0.2°, 16.824°±0.2°, 17.4°±0.2°, 17.84°±0.2°, 18.404°±0.2°, 18.945°±0.2°, 19.139°±0.2°, 19.477°±0.2°, 20.243°±0.2°, 21.798°±0.2°, 21.985°±0.2°, 22.179°±0.2°, 22.6°±0.2°, 22.844°±0.2°, 23.081°±0.2°, 23.272°±0.2°, 26.682°±0.2°, and 27.765°±0.2°.

In one embodiment, X-ray powder diffraction pattern analysis data of the crystal form N are substantially as shown in the following table:

| Position [°2θ]±0.2° | d-spacing [Å] | Relative intensity [%] | Position [°2θ]±0.2° | d-spacing [Å] | Relative intensity [%] |
|---|---|---|---|---|---|
| 5.218 | 16.9216 | 13.80% | 17.84 | 4.968 | 48.40% |
| 6.643 | 13.2947 | 5.70% | 18.404 | 4.8169 | 62.50% |
| 10.402 | 8.4973 | 50.20% | 18.945 | 4.6806 | 28.60% |
| 10.874 | 8.1297 | 30.40% | 19.139 | 4.6334 | 30.00% |
| 13.403 | 6.6009 | 5.30% | 19.477 | 4.5538 | 7.20% |
| 13.583 | 6.5137 | 7.20% | 20.243 | 4.3833 | 64.40% |
| 13.719 | 6.4493 | 4.50% | 21.798 | 4.0739 | 32.30% |
| 14.031 | 6.3066 | 3.50% | 21.985 | 4.0397 | 26.60% |
| 14.354 | 6.1655 | 24.60% | 22.179 | 4.0047 | 9.00% |
| 14.742 | 6.004 | 6.20% | 22.6 | 3.9312 | 70.00% |
| 15.619 | 5.6691 | 100.00% | 22.844 | 3.8897 | 7.50% |
| 15.96 | 5.5486 | 18.00% | 23.081 | 3.8503 | 7.70% |
| 16.677 | 5.3115 | 5.60% | 23.272 | 3.8192 | 1.50% |
| 16.824 | 5.2654 | 2.60% | 26.682 | 3.3383 | 42.60% |
| 17.4 | 5.0925 | 65.90% | 27.765 | 3.2105 | 4.30% |

In one embodiment, a thermogravimetric analysis (TGA) curve of the crystal form N shows a weight loss of 9.097% during heating from 29.97 °C to 125.41 °C.

In one embodiment, a differential scanning calorimetry (DSC) curve of the crystal form N has endothermic peaks with initial temperatures of 64.45 °C and 158.00 °C, respectively.

In one embodiment, the differential scanning calorimetry (DSC) curve of the crystal form N has endothermic peaks with peak temperatures of 72.35 °C and 164.10 °C, respectively.

The present disclosure provides a crystal form O of the compound represented by formula I, and an X-ray powder diffraction (XRPD) pattern of the crystal form has at least three, at least four, at least five, at least six, or at least seven characteristic peaks at the following 2θ angles: 6.2637°±0.2°, 12.5384°±0.2°, 15.377°±0.2°, 18.2659°+0.2°, 18.8011°+0.2°, 21.405°±0.2°, and 24.3656°±0.2°;

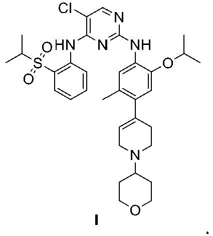

In one embodiment, the X-ray powder diffraction pattern of the crystal form O, measured using Cu-Kα radiation, has diffraction peaks at the following 2θ positions: 5.6701°±0.2°, 6.2637°±0.2°, 7.4001°±0.2°, 7.6972°±0.2°, 7.9176°±0.2°, 8.0144°±0.2°, 9.1172°±0.2°, 9.9174°±0.2°, 10.546°±0.2°, 11.1416°±0.2°, 12.5384°±0.2°, 13.8784°±0.2°, 14.0157°±0.2°, 14.9304°±0.2°, 15.377°±0.2°, 15.6815°±0.2°, 16.1223°±0.2°, 16.7054°±0.2°, 18.2659°±0.2°, 18.8011°±0.2°, 19.8482°±0.2°, 20.2401°±0.2°, 20.735°±0.2°, 21.405°±0.2°, 22.2174°±0.2°, 22.4377°±0.2°, 22.8608°±0.2°, 22.9801°±0.2°, 23.621°±0.2°, and 24.3656°±0.2°.

In one embodiment, X-ray powder diffraction pattern analysis data of the crystal form O are substantially as shown in the following table:

| Position [°2θ]±0.2° | d-spacing [Å] | Relative intensity [%] | Position [°2θ]±0.2° | d-spacing [Å] | Relative intensity [%] |
|---|---|---|---|---|---|
| 5.6701 | 15.57376 | 0.30% | 15.6815 | 5.64651 | 0.60% |
| 6.2637 | 14.09924 | 100.00% | 16.1223 | 5.49309 | 3.10% |
| 7.4001 | 11.93649 | 0.40% | 16.7054 | 5.30265 | 1.10% |
| 7.6972 | 11.47633 | 2.50% | 18.2659 | 4.853 | 28.50% |
| 7.9176 | 11.15736 | 0.30% | 18.8011 | 4.71603 | 39.40% |
| 8.0144 | 11.02287 | 0.30% | 19.8482 | 4.46953 | 0.30% |
| 9.1172 | 9.69187 | 0.90% | 20.2401 | 4.38387 | 0.60% |
| 9.9174 | 8.91159 | 3.30% | 20.735 | 4.28033 | 0.20% |
| 10.546 | 8.38173 | 0.30% | 21.405 | 4.14785 | 24.10% |
| 11.1416 | 7.93499 | 0.80% | 22.2174 | 3.99798 | 0.70% |
| 12.5384 | 7.05398 | 13.30% | 22.4377 | 3.95922 | 0.50% |
| 13.8784 | 6.37577 | 0.40% | 22.8608 | 3.88691 | 2.00% |
| 14.0157 | 6.31362 | 0.40% | 22.9801 | 3.86698 | 2.40% |
| 14.9304 | 5.9288 | 0.20% | 23.621 | 3.7635 | 2.30% |
| 15.377 | 5.7576 | 26.30% | 24.3656 | 3.65015 | 12.90% |

In one embodiment, a thermogravimetric analysis (TGA) curve of the crystal form O shows a weight loss of 8.444% during heating from 29.88 °C to 150.15 °C.

In one embodiment, a differential scanning calorimetry (DSC) curve of the crystal form O has endothermic peaks with initial temperatures of 117.33 °C, 147.75 °C, and 158.31 °C, respectively.

In one embodiment, the differential scanning calorimetry (DSC) curve of the crystal form O has endothermic peaks with peak temperatures of 122.88 °C, 149.62 °C, and 159.74 °C, respectively.

The present disclosure provides a crystal form P of the compound represented by formula I, and an X-ray powder diffraction (XRPD) pattern of the crystal form has at least three, at least four, at least five, at least six, or at least seven characteristic peaks at the following 2θ angles: 5.516°±0.2°, 7.54°±0.2°, 11.012°±0.2°, 18.08°±0.2°, 18.962°±0.2°, 20.278°±0.2°, and 23.558°±0.2°;

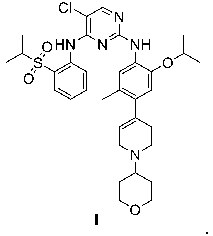

In one embodiment, the X-ray powder diffraction pattern of the crystal form P, measured using Cu-Kα radiation, has diffraction peaks at the following 2θ positions: 5.516°±0.2°, 6.723°±0.2°, 7.54°±0.2°, 8.761°±0.2°, 11.012°±0.2°, 11.534°±0.2°, 12.657°±0.2°, 12.839°±0.2°, 13.459°±0.2°, 13.558°±0.2°, 14.102°±0.2°, 15.658°+0.2°, 16.359°+0.2°, 16.519°+0.2°, 17.04°±0.2°, 17.195°+0.2°, 17.404°+0.2°, 18.08°+0.2°, 18.322°±0.2°, 18.515°±0.2°, 18.962°+0.2°, 20.278°+0.2°, 21.66°±0.2°, 21.901°±0.2°, 22.06°+0.2°, 23.14°+0.2°, 23.558°+0.2°, 23.777°+0.2°, 24.283°+0.2°, and 24.98°+0.2°.

In one embodiment, X-ray powder diffraction pattern analysis data of the crystal form P are substantially as shown in the following table:

| Position [°2θ]±0.2° | d-spacing [Å] | Relative intensity [%] | Position [°2θ]±0.2° | d-spacing [Å] | Relative intensity [%] |
|---|---|---|---|---|---|
| 5.516 | 16.0095 | 100.00% | 17.195 | 5.1527 | 12.30% |
| 6.723 | 13.1362 | 22.80% | 17.404 | 5.0914 | 18.70% |
| 7.54 | 11.7155 | 65.70% | 18.08 | 4.9024 | 36.70% |
| 8.761 | 10.085 | 20.40% | 18.322 | 4.8381 | 9.50% |
| 11.012 | 8.0279 | 70.00% | 18.515 | 4.7882 | 12.90% |
| 11.534 | 7.6656 | 11.80% | 18.962 | 4.6764 | 92.10% |
| 12.657 | 6.9884 | 11.90% | 20.278 | 4.3759 | 47.70% |
| 12.839 | 6.8897 | 10.70% | 21.66 | 4.0996 | 32.40% |
| 13.459 | 6.5734 | 14.30% | 21.901 | 4.0549 | 16.30% |
| 13.558 | 6.5259 | 26.90% | 22.06 | 4.0261 | 20.00% |
| 14.102 | 6.2751 | 6.50% | 23.14 | 3.8406 | 19.70% |
| 15.658 | 5.6549 | 12.70% | 23.558 | 3.7735 | 63.70% |
| 16.359 | 5.4143 | 11.00% | 23.777 | 3.7392 | 1.70% |
| 16.519 | 5.362 | 32.80% | 24.283 | 3.6624 | 8.60% |
| 17.04 | 5.1993 | 17.90% | 24.98 | 3.5617 | 17.50% |

In one embodiment, a thermogravimetric analysis (TGA) curve of the crystal form P shows a weight loss of 0.4118% during heating from 30.74 °C to 150.43 °C.

In one embodiment, a differential scanning calorimetry (DSC) curve of the crystal form P has endothermic peaks with initial temperatures of 106.68 °C and 165.23 °C, respectively.

In one embodiment, the differential scanning calorimetry (DSC) curve of the crystal form P has endothermic peaks with peak temperatures of 120.91 °C and 168.20 °C, respectively.

The present disclosure provides a crystal form Q of the compound represented by formula I, and an X-ray powder diffraction (XRPD) pattern of the crystal form has at least three, at least four, at least five, at least six, or at least seven characteristic peaks at the following 2θ angles: 17.957°±0.2°, 18.421°±0.2°, 18.578°±0.2°, 19.22°±0.2°, 19.563°±0.2°, 21.58°±0.2°, and 22.861°±0.2°;

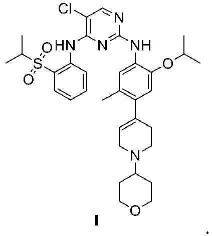

In one embodiment, the X-ray powder diffraction pattern of the crystal form Q, measured using Cu-Kα radiation, has diffraction peaks at the following 2θ positions: 7.481°±0.2°, 8.711°±0.2°, 9.357°±0.2°, 9.878°±0.2°, 10.03°±0.2°, 10.323°±0.2°, 10.685°±0.2°, 11.682°±0.2°, 12.365°±0.2°, 12.879°±0.2°, 13.262°±0.2°, 13.701°±0.2°, 14.221°±0.2°, 15.008°±0.2°, 15.307°±0.2°, 15.644°±0.2°, 15.981°±0.2°, 16.418°±0.2°, 17.483°±0.2°, 17.957°±0.2°, 18.421°±0.2°, 18.578°±0.2°, 19.102°±0.2°, 19.22°±0.2°, 19.563°±0.2°, 20.162°±0.2°, 20.453°±0.2°, 20.779°±0.2°, 21.58°±0.2°, and 22.861°±0.2°.

In one embodiment, X-ray powder diffraction pattern analysis data of the crystal form Q are substantially as shown in the following table:

| Position [°2θ]±0.2° | d-spacing [Å] | Relative intensity [%] | Position [°2θ]±0.2° | d-spacing [Å] | Relative intensity [%] |
|---|---|---|---|---|---|
| 7.481 | 11.8079 | 15.00% | 15.644 | 5.6599 | 19.10% |
| 8.711 | 10.1434 | 15.70% | 15.981 | 5.5413 | 22.60% |
| 9.357 | 9.4442 | 18.50% | 16.418 | 5.3949 | 19.50% |
| 9.878 | 8.9474 | 45.70% | 17.483 | 5.0685 | 39.40% |
| 10.03 | 8.8117 | 41.50% | 17.957 | 4.9358 | 94.20% |
| 10.323 | 8.5624 | 12.50% | 18.421 | 4.8125 | 66.30% |
| 10.685 | 8.273 | 13.70% | 18.578 | 4.7722 | 64.60% |
| 11.682 | 7.5691 | 8.40% | 19.102 | 4.6425 | 46.00% |
| 12.365 | 7.1525 | 24.20% | 19.22 | 4.6142 | 59.10% |
| 12.879 | 6.8683 | 7.40% | 19.563 | 4.5339 | 100.00% |
| 13.262 | 6.6705 | 39.50% | 20.162 | 4.4006 | 26.30% |
| 13.701 | 6.4578 | 44.00% | 20.453 | 4.3387 | 41.80% |
| 14.221 | 6.2228 | 39.50% | 20.779 | 4.2713 | 51.00% |
| 15.008 | 5.8984 | 16.80% | 21.58 | 4.1147 | 84.50% |
| 15.307 | 5.7837 | 19.70% | 22.861 | 3.8869 | 54.50% |

In one embodiment, a thermogravimetric analysis (TGA) curve of the crystal form Q shows a weight loss of 1.609% during heating from 30.07 °C to 150.15 °C.

In one embodiment, a differential scanning calorimetry (DSC) curve of the crystal form Q has endothermic peaks with initial temperatures of 73.53 °C and 139.04 °C, respectively.

In one embodiment, the differential scanning calorimetry (DSC) curve of the crystal form Q has endothermic peaks with peak temperatures of 97.37 °C and 142.10 °C, respectively.

The present disclosure provides a crystal form R of the compound represented by formula I, and an X-ray powder diffraction (XRPD) pattern of the crystal form has at least three, at least four, at least five, at least six, or at least seven characteristic peaks at the following 2θ angles: 14.756°±0.2°, 17.758°±0.2°, 18.698°±0.2°, 20.377°±0.2°, 21.338°±0.2°, 21.979°±0.2°, and 25.521°±0.2°;

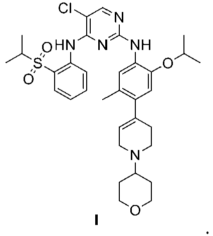

In one embodiment, the X-ray powder diffraction pattern of the crystal form R, measured using Cu-Kα radiation, has diffraction peaks at the following 2θ positions: 6.612°±0.2°, 7.2°±0.2°, 9.141°±0.2°, 10.376°±0.2°, 12.355°±0.2°, 13.183°±0.2°, 13.638°±0.2°, 14.756°±0.2°, 15.059°±0.2°, 16.62°±0.2°, 17.1°±0.2°, 17.758°±0.2°, 18.44°±0.2°, 18.698°±0.2°, 19.397°±0.2°, 19.836°±0.2°, 20.121°±0.2°, 20.377°±0.2°, 21.142°±0.2°, 21.338°±0.2°, 21.979°±0.2°, 22.201°±0.2°, 23.019°±0.2°, 24.201°±0.2°, 24.898°±0.2°, 25.521°±0.2°, 26.581°±0.2°, 26.92°±0.2°, 27.243°±0.2°, and 29.38°±0.2°.

In one embodiment, X-ray powder diffraction pattern analysis data of the crystal form R are substantially as shown in the following table:

| Position [°2θ]±0.2° | d-spacing [Å] | Relative intensity [%] | Position [°2θ]±0.2° | d-spacing [Å] | Relative intensity [%] |
|---|---|---|---|---|---|
| 6.612 | 13.3565 | 20.40% | 19.836 | 4.4724 | 31.00% |
| 7.2 | 12.2685 | 43.90% | 20.121 | 4.4095 | 16.60% |
| 9.141 | 9.6668 | 42.50% | 20.377 | 4.3547 | 64.50% |
| 10.376 | 8.5184 | 15.70% | 21.142 | 4.1988 | 16.80% |
| 12.355 | 7.1583 | 27.00% | 21.338 | 4.1608 | 71.70% |
| 13.183 | 6.7103 | 11.40% | 21.979 | 4.0409 | 100.00% |
| 13.638 | 6.4876 | 21.50% | 22.201 | 4.0009 | 18.10% |
| 14.756 | 5.9983 | 86.60% | 23.019 | 3.8605 | 18.90% |
| 15.059 | 5.8784 | 48.60% | 24.201 | 3.6746 | 23.70% |
| 16.62 | 5.3296 | 28.50% | 24.898 | 3.5733 | 16.60% |
| 17.1 | 5.181 | 23.20% | 25.521 | 3.4874 | 52.30% |
| 17.758 | 4.9906 | 99.40% | 26.581 | 3.3508 | 31.90% |
| 18.44 | 4.8074 | 21.40% | 26.92 | 3.3093 | 16.80% |
| 18.698 | 4.7417 | 51.90% | 27.243 | 3.2708 | 12.60% |
| 19.397 | 4.5726 | 49.40% | 29.38 | 3.0375 | 34.90% |

In one embodiment, a thermogravimetric analysis (TGA) curve of the crystal form R shows a weight loss of 4.375% during heating from 30.17 °C to 150.72 °C.

In one embodiment, a differential scanning calorimetry (DSC) curve of the crystal form R has endothermic peaks with initial temperatures of 25.50 °C, 103.97 °C, 115.73 °C, and 145.79 °C, respectively.

In one embodiment, the differential scanning calorimetry (DSC) curve of the crystal form R has endothermic peaks with peak temperatures of 33.23 °C, 108.31 °C, 120.82 °C, and 156.29 °C, respectively.

The present disclosure provides one equivalent of mesylate crystal form A of the compound represented by formula I, and an X-ray powder diffraction (XRPD) pattern of the crystal form has at least three, at least four, at least five, at least six, or at least seven characteristic peaks at the following 2θ angles: 10.2973°±0.2°, 14.7185°±0.2°, 15.1407°±0.2°, 17.8003°±0.2°, 18.3418°±0.2°, 20.6191°±0.2°, and 20.7584°±0.2°;

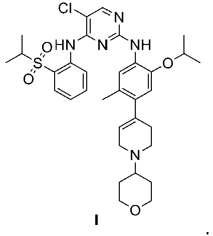

In one embodiment, the X-ray powder diffraction pattern of the one equivalent of mesylate crystal form A, measured using Cu-Kα radiation, has diffraction peaks at the following 2θ positions: 10.2973°±0.2°, 15.1407°±0.2°, and 17.8003°±0.2°.

In one embodiment, the X-ray powder diffraction pattern of the one equivalent of mesylate crystal form A, measured using Cu-Kα radiation, has diffraction peaks at the following 2θ positions: 10.2973°±0.2°, 15.1407°±0.2°, 17.8003°±0.2°, and 20.6191°±0.2°.

In one embodiment, the X-ray powder diffraction pattern of the one equivalent of mesylate crystal form A, measured using Cu-Kα radiation, has diffraction peaks at the following 2θ positions: 10.2973°±0.2°, 15.1407°+0.2°, 17.8003°±0.2°, 18.3418°+0.2°, and 20.6191°±0.2°.

In one embodiment, the X-ray powder diffraction pattern of the one equivalent of mesylate crystal form A, measured using Cu-Kα radiation, has diffraction peaks at the following 2θ positions: 10.2973°±0.2°, 15.1407°+0.2°, 17.8003°±0.2°, 18.3418°+0.2°, 20.6191°+0.2°, and 20.7584°+0.2°.

In one embodiment, the X-ray powder diffraction pattern of the one equivalent of mesylate crystal form A, measured using Cu-Kα radiation, has diffraction peaks at the following 2θ positions: 10.2973°±0.2°, 14.7185°±0.2°, 15.1407°±0.2°, 17.8003°±0.2°, 18.3418°±0.2°, 20.6191°±0.2°, and 20.7584°±0.2°.

In one embodiment, the X-ray powder diffraction pattern of the one equivalent of mesylate crystal form A, measured using Cu-Kα radiation, has diffraction peaks at the following 2θ positions: 10.2973°±0.2°, 10.7003°±0.2°, 11.5381°±0.2°, 12.618°±0.2°, 12.918°±0.2°, 13.995°±0.2°, 14.7185°±0.2°, 15.1407°±0.2°, 15.6419°+0.2°, 17.8003°+0.2°, 18.1003°+0.2°, 18.3418°+0.2°, 18.7198°+0.2°, 19.281°±0.2°, 19.8025°±0.2°, 20.3791°±0.2°, 20.6191°±0.2°, 20.7584°±0.2°, 21.2626°±0.2°, 21.4387°±0.2°, 21.7211°±0.2°, 22.0791°±0.2°, 22.442°±0.2°, 22.8785°±0.2°, 23.1402°±0.2°, 23.4006°±0.2°,23.739°±0.2°,24.2791°±0.2°, 24.6595°+0.2°, and 25.2227°+0.2°.

In one embodiment, X-ray powder diffraction pattern analysis data of the one equivalent of mesylate crystal form A are substantially as shown in the following table:

| Position [°2θ]±0.2° | d-spacing [Å] | Relative intensity [%] | Position [°2θ]±0.2° | d-spacing [Å] | Relative intensity [%] |
|---|---|---|---|---|---|
| 10.2973 | 8.58364 | 100.00% | 20.3791 | 4.35427 | 9.60% |
| 10.7003 | 8.2612 | 3.40% | 20.6191 | 4.30414 | 65.00% |
| 11.5381 | 7.66317 | 11.30% | 20.7584 | 4.27556 | 44.80% |
| 12.618 | 7.00966 | 35.70% | 21.2626 | 4.17531 | 32.20% |
| 12.918 | 6.84756 | 24.00% | 21.4387 | 4.14141 | 13.80% |
| 13.995 | 6.32292 | 4.70% | 21.7211 | 4.0882 | 33.20% |
| 14.7185 | 6.01369 | 44.60% | 22.0791 | 4.02272 | 5.20% |
| 15.1407 | 5.84694 | 69.20% | 22.442 | 3.95847 | 27.40% |
| 15.6419 | 5.6607 | 20.60% | 22.8785 | 3.88393 | 20.60% |
| 17.8003 | 4.97888 | 87.70% | 23.1402 | 3.84059 | 26.00% |
| 18.1003 | 4.89702 | 31.00% | 23.4006 | 3.79845 | 11.10% |
| 18.3418 | 4.83307 | 52.80% | 23.739 | 3.74505 | 10.70% |
| 18.7198 | 4.73634 | 35.50% | 24.2791 | 3.66295 | 5.30% |
| 19.281 | 4.59973 | 18.00% | 24.6595 | 3.60731 | 30.50% |
| 19.8025 | 4.47975 | 6.90% | 25.2227 | 3.52802 | 10.10% |

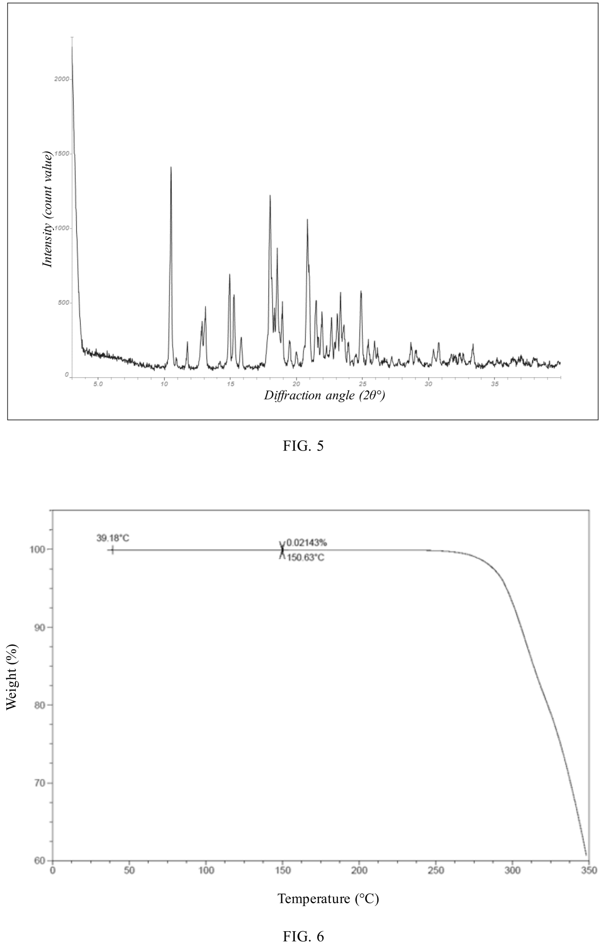

In one embodiment, the X-ray powder diffraction pattern of the one equivalent of mesylate crystal form A is substantially as shown in FIG. 5.

In one embodiment, a thermogravimetric analysis (TGA) curve of the one equivalent of mesylate crystal form A shows a weight loss of 0.02143±0.005% during heating from 39.18±3 °C to 150.63±3 °C.

In one embodiment, the thermogravimetric analysis (TGA) curve of the one equivalent of mesylate crystal form A shows a weight loss of 0.02143% during heating from 39.18 °C to 150.63 °C.

In one embodiment, the thermogravimetric analysis curve of the one equivalent of mesylate crystal form A is substantially as shown in FIG. 6.

In one embodiment, a differential scanning calorimetry (DSC) curve of the one equivalent of mesylate crystal form A has endothermic peaks with initial temperatures of 85.22±3 °C and 220.46±3 °C, respectively.

In one embodiment, the differential scanning calorimetry (DSC) curve of the one equivalent of mesylate crystal form A has endothermic peaks with initial temperatures of 85.22 °C and 220.46 °C, respectively.

In one embodiment, the differential scanning calorimetry (DSC) curve of the one equivalent of mesylate crystal form A has endothermic peaks with peak temperatures of 86.90±3 °C and 222.22±3 °C, respectively.

In one embodiment, the differential scanning calorimetry (DSC) curve of the one equivalent of mesylate crystal form A has endothermic peaks with peak temperatures of 86.90 °C and 222.22 °C, respectively.

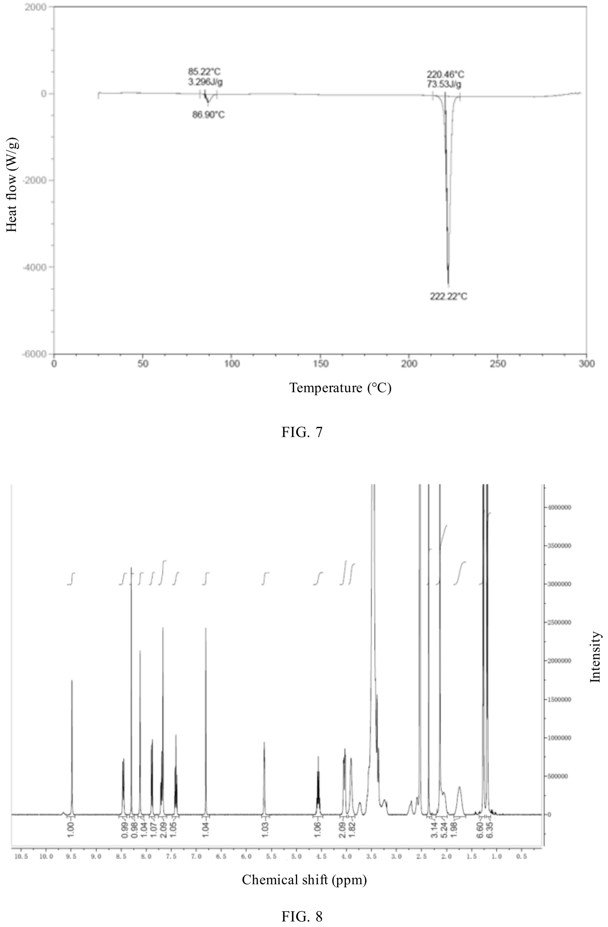

In one embodiment, the differential scanning calorimetry curve of the one equivalent of mesylate crystal form A is substantially as shown in FIG. 7.

In one embodiment, a dynamic vapor sorption (DVS) curve of the one equivalent of mesylate crystal form A shows a hygroscopic weight gain of 0.31±0.005% at 25 °C and 80% RH.

In one embodiment, the dynamic vapor sorption (DVS) curve of the one equivalent of mesylate crystal form A shows a hygroscopic weight gain of 0.31% at 25 °C and 80% RH.

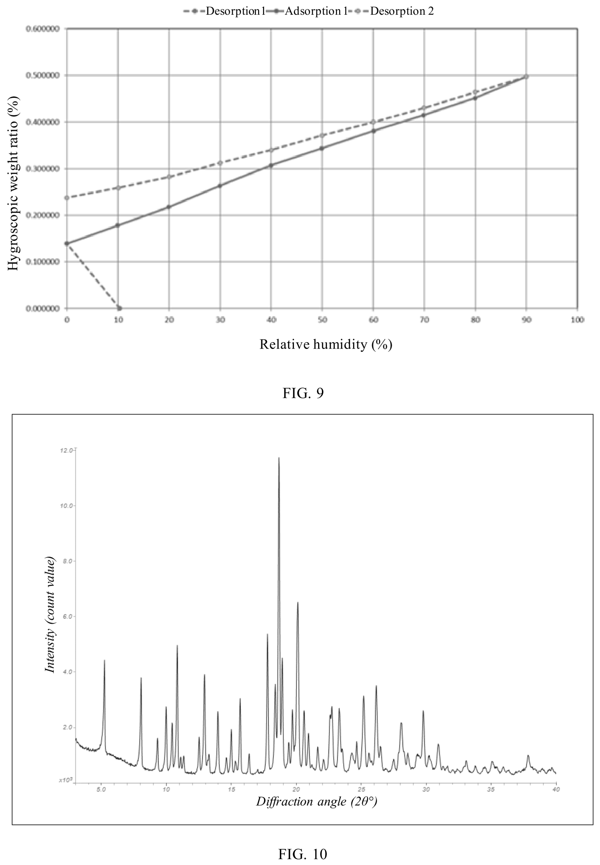

In one embodiment, the dynamic vapor sorption curve of the one equivalent of mesylate crystal form A is as shown in FIG. 9.

In one embodiment, ¹H-NMR results of the one equivalent of mesylate crystal form A show that methanesulfonic acid and a free base in a sample are in a molar ratio of 1:1.

In one embodiment, a proton nuclear magnetic resonance spectrum of the one equivalent of mesylate crystal form A is shown in FIG. 8.

The present disclosure further provides a preparation method for the one equivalent of mesylate crystal form A of the compound represented by formula I, which comprises the following steps: stirring an amorphous form of the compound represented by formula I and methanesulfonic acid in tetrahydrofuran to precipitate a solid, so as to obtain the one equivalent of mesylate crystal form A of the compound represented by formula I, wherein the stirring is performed at room temperature, e.g., 25 °C; the stirring is preferably for a period of 2-7 days, and further preferably 3 days; the amorphous form of the compound represented by formula I and methanesulfonic acid are preferably in a molar ratio of 1:1; the compound represented by formula I and the solvent are preferably in a mass-to-volume ratio of (10 mg-100 mg):1 mL, and further preferably 25 mg:1 mL. In one embodiment, the preparation method for the one equivalent of mesylate crystal form A may further comprise, after the solid is precipitated, the steps of separation and drying to obtain the one equivalent of mesylate crystal form A.

The present disclosure provides one equivalent of mesylate crystal form B of the compound represented by formula I, and an X-ray powder diffraction (XRPD) pattern of the crystal form has at least three, at least four, at least five, at least six, or at least seven characteristic peaks at the following 2θ angles: 5.087°±0.2°, 9.975°±0.2°, 15.895°±0.2°, 19.013°±0.2°, 19.521°±0.2°, 24.658°±0.2°, and 25.066°±0.2°;

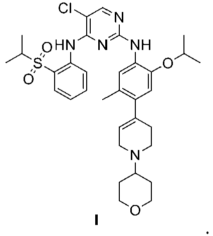

In one embodiment, the X-ray powder diffraction pattern of the one equivalent of mesylate crystal form B, measured using Cu-Kα radiation, has diffraction peaks at the following 2θ positions: 5.087°±0.2°, 6.896°±0.2°, 9.975°±0.2°, 11.998°±0.2°, 13.18°±0.2°, 14.156°±0.2°, 14.733°±0.2°, 15.895°±0.2°, 16.663°±0.2°, 17.025°±0.2°, 17.456°±0.2°, 17.945°±0.2°, 19.013°±0.2°, 19.521°±0.2°, 20.317°±0.2°, 21.079°±0.2°, 21.294°±0.2°, 21.911°±0.2°, 22.851°±0.2°, 22.964°±0.2°, 23.783°±0.2°, 24.254°±0.2°, 24.658°±0.2°, 25.066°±0.2°, 25.792°±0.2°, 26.82°±0.2°, 27.34°±0.2°, 27.821°±0.2°, 27.985°±0.2°, and 30.133°±0.2°.

In one embodiment, X-ray powder diffraction pattern analysis data of the one equivalent of mesylate crystal form B are substantially as shown in the following table:

| Position [°2θ]±0.2° | d-spacing [Å] | Relative intensity [%] | Position [°2θ]±0.2° | d-spacing [Å] | Relative intensity [%] |
|---|---|---|---|---|---|
| 5.087 | 17.3579 | 44.00% | 21.079 | 4.2113 | 17.40% |
| 6.896 | 12.8086 | 41.80% | 21.294 | 4.1692 | 37.80% |
| 9.975 | 8.8605 | 84.10% | 21.911 | 4.0533 | 10.30% |
| 11.998 | 7.3707 | 19.70% | 22.851 | 3.8886 | 24.30% |
| 13.18 | 6.7118 | 23.70% | 22.964 | 3.8696 | 11.60% |
| 14.156 | 6.2513 | 31.30% | 23.783 | 3.7382 | 36.60% |
| 14.733 | 6.0078 | 30.10% | 24.254 | 3.6667 | 20.50% |
| 15.895 | 5.571 | 100.00% | 24.658 | 3.6075 | 86.60% |
| 16.663 | 5.3159 | 11.20% | 25.066 | 3.5498 | 54.90% |
| 17.025 | 5.2037 | 24.30% | 25.792 | 3.4514 | 34.30% |
| 17.456 | 5.0763 | 14.50% | 26.82 | 3.3214 | 27.60% |
| 17.945 | 4.939 | 25.80% | 27.34 | 3.2594 | 27.10% |
| 19.013 | 4.664 | 79.40% | 27.821 | 3.2041 | 22.60% |
| 19.521 | 4.5437 | 68.70% | 27.985 | 3.1857 | 20.80% |
| 20.317 | 4.3674 | 22.40% | 30.133 | 2.9633 | 18.50% |

In one embodiment, a thermogravimetric analysis (TGA) curve of the one equivalent of mesylate crystal form B shows a weight loss of 4.876% during heating from 28.09 °C to 90.26 °C.

In one embodiment, a differential scanning calorimetry (DSC) curve of the one equivalent of mesylate crystal form B has endothermic peaks with initial temperatures of 36.95 °C, 93.93 °C, 130.40 °C, and 218.58 °C, respectively.

In one embodiment, the differential scanning calorimetry (DSC) curve of the one equivalent of mesylate crystal form B has endothermic peaks with peak temperatures of 57.12 °C, 99.03 °C, 136.18 °C, and 219.99 °C, respectively.

In one embodiment, ¹H-NMR results of the one equivalent of mesylate crystal form B show that methanesulfonic acid and a free base in a sample are in a molar ratio of 1:1.

The present disclosure provides one equivalent of mesylate crystal form C of the compound represented by formula I, and an X-ray powder diffraction (XRPD) pattern of the crystal form has at least three, at least four, at least five, at least six, or at least seven characteristic peaks at the following 2θ angles: 15.133°±0.2°, 15.369°±0.2°, 18.155°±0.2°, 19.055°±0.2°, 22.54°±0.2°, 28.617°±0.2°, and 33.447°±0.2°;

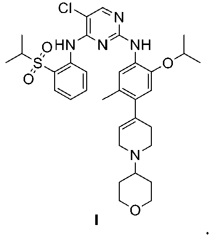

In one embodiment, the X-ray powder diffraction pattern of the one equivalent of mesylate crystal form C, measured using Cu-Kα radiation, has diffraction peaks at the following 2θ positions: 10.424°±0.2°, 10.555°±0.2°, 12.919°±0.2°, 14.078°±0.2°, 14.292°±0.2°, 15.133°±0.2°, 15.369°±0.2°, 15.844°±0.2°, 17.882°±0.2°, 18.155°±0.2°, 18.323°±0.2°, 18.733°±0.2°, 19.055°±0.2°, 19.674°±0.2°, 20.996°±0.2°, 21.605°±0.2°, 22.009°±0.2°, 22.54°±0.2°, 22.968°±0.2°, 23.277°±0.2°, 23.709°±0.2°, 24.975°±0.2°, 25.203°±0.2°, 25.406°±0.2°, 27.333°±0.2°, 28.078°±0.2°, 28.617°±0.2°, 28.764°±0.2°, 29.378°±0.2°, and 33.447°±0.2°.

In one embodiment, X-ray powder diffraction pattern analysis data of the one equivalent of mesylate crystal form C are substantially as shown in the following table:

| Position [°2θ]±0.2° | d-spacing [Å] | Relative intensity [%] | Position [°2θ]±0.2° | d-spacing [Å] | Relative intensity [%] |
|---|---|---|---|---|---|
| 10.424 | 8.4793 | 1.30% | 21.605 | 4.1099 | 1.20% |
| 10.555 | 8.3743 | 1.60% | 22.009 | 4.0353 | 5.30% |
| 12.919 | 6.8472 | 5.40% | 22.54 | 3.9415 | 6.90% |
| 14.078 | 6.2857 | 1.20% | 22.968 | 3.8689 | 4.00% |
| 14.292 | 6.1922 | 2.30% | 23.277 | 3.8183 | 0.80% |
| 15.133 | 5.8498 | 100.00% | 23.709 | 3.7497 | 2.10% |
| 15.369 | 5.7604 | 25.80% | 24.975 | 3.5624 | 0.70% |
| 15.844 | 5.5888 | 1.40% | 25.203 | 3.5307 | 1.40% |
| 17.882 | 4.9564 | 2.80% | 25.406 | 3.503 | 0.50% |
| 18.155 | 4.8824 | 14.00% | 27.333 | 3.2602 | 5.80% |
| 18.323 | 4.8381 | 4.00% | 28.078 | 3.1754 | 0.50% |
| 18.733 | 4.7329 | 1.50% | 28.617 | 3.1168 | 8.60% |
| 19.055 | 4.6537 | 9.00% | 28.764 | 3.1012 | 3.60% |
| 19.674 | 4.5086 | 4.20% | 29.378 | 3.0377 | 1.30% |
| 20.996 | 4.2277 | 1.80% | 33.447 | 2.6769 | 11.60% |

In one embodiment, a thermogravimetric analysis (TGA) curve of the one equivalent of mesylate crystal form C shows a weight loss of 0.04218% during heating from 34.91 °C to 250.03 °C.

In one embodiment, a differential scanning calorimetry (DSC) curve of the one equivalent of mesylate crystal form C has endothermic peaks with initial temperatures of 85.66 °C and 220.56 °C, respectively.

In one embodiment, the differential scanning calorimetry (DSC) curve of the one equivalent of mesylate crystal form C has endothermic peaks with peak temperatures of 87.04 °C and 222.69 °C, respectively.

In one embodiment, ¹H-NMR results of the one equivalent of mesylate crystal form C show that methanesulfonic acid and a free base in a sample are in a molar ratio of 1:1.

The present disclosure provides one equivalent of mesylate crystal form D of the compound represented by formula I, and an X-ray powder diffraction (XRPD) pattern of the crystal form has at least three, at least four, at least five, at least six, or at least seven characteristic peaks at the following 2θ angles: 13.335°±0.2°, 15.369°±0.2°, 18.019°±0.2°, 18.234°±0.2°, 18.62°±0.2°, 21.173°±0.2°, and 25.632°±0.2°;

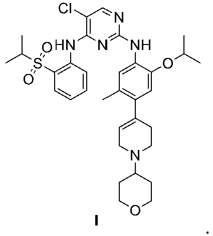

In one embodiment, the X-ray powder diffraction pattern of the one equivalent of mesylate crystal form D, measured using Cu-Kα radiation, has diffraction peaks at the following 2θ positions: 10.328°±0.2°, 10.659°±0.2°, 11.775°±0.2°, 13.112°±0.2°, 13.335°±0.2°, 14.352°±0.2°, 14.898°±0.2°, 15.369°±0.2°, 15.873°±0.2°, 16.152°±0.2°, 17.848°±0.2°, 18.019°±0.2°, 18.234°±0.2°, 18.62°±0.2°, 18.925°±0.2°, 21.173°±0.2°, 21.566°±0.2°, 21.757°±0.2°, 22.643°±0.2°, 22.892°±0.2°, 23.478°±0.2°, 23.771°±0.2°, 23.895°±0.2°, 24.14°±0.2°, 24.53°±0.2°, 25.069°±0.2°, 25.632°±0.2°, 26.022°±0.2°, 26.692°±0.2°, and 26.825°±0.2°.

In one embodiment, X-ray powder diffraction pattern analysis data of the one equivalent of mesylate crystal form D are substantially as shown in the following table:

| Position [°2θ]±0.2° | d-spacing [Å] | Relative intensity [%] | Position [°2θ]±0.2° | d-spacing [Å] | Relative intensity [%] |
|---|---|---|---|---|---|
| 10.328 | 8.5581 | 2.50% | 21.173 | 4.1928 | 85.50% |
| 10.659 | 8.2929 | 5.20% | 21.566 | 4.1172 | 11.40% |
| 11.775 | 7.5094 | 4.10% | 21.757 | 4.0815 | 13.50% |
| 13.112 | 6.7466 | 5.50% | 22.643 | 3.9238 | 3.70% |
| 13.335 | 6.6343 | 19.80% | 22.892 | 3.8816 | 17.50% |
| 14.352 | 6.1665 | 12.20% | 23.478 | 3.7861 | 2.50% |
| 14.898 | 5.9417 | 14.50% | 23.771 | 3.74 | 5.10% |
| 15.369 | 5.7605 | 26.40% | 23.895 | 3.721 | 1.60% |
| 15.873 | 5.5789 | 7.00% | 24.14 | 3.6838 | 1.90% |
| 16.152 | 5.4831 | 2.50% | 24.53 | 3.6261 | 2.70% |
| 17.848 | 4.9656 | 15.50% | 25.069 | 3.5493 | 5.60% |
| 18.019 | 4.9188 | 27.90% | 25.632 | 3.4726 | 26.10% |
| 18.234 | 4.8614 | 100.00% | 26.022 | 3.4214 | 9.00% |
| 18.62 | 4.7615 | 26.00% | 26.692 | 3.337 | 1.90% |
| 18.925 | 4.6855 | 18.30% | 26.825 | 3.3208 | 3.70% |

In one embodiment, a thermogravimetric analysis (TGA) curve of the one equivalent of mesylate crystal form D shows a weight loss of 0.006279% during heating from 22.75 °C to 250.00 °C.

In one embodiment, a differential scanning calorimetry (DSC) curve of the one equivalent of mesylate crystal form D has endothermic peaks with initial temperatures of 84.66 °C and 220.11 °C, respectively.

In one embodiment, the differential scanning calorimetry (DSC) curve of the one equivalent of mesylate crystal form D has endothermic peaks with peak temperatures of 86.50 °C and 222.17 °C, respectively.

In one embodiment, ¹H-NMR results of the one equivalent of mesylate crystal form D show that methanesulfonic acid and a free base in a sample are in a molar ratio of 1:1.

The present disclosure provides one equivalent of mesylate crystal form E of the compound represented by formula I, and an X-ray powder diffraction (XRPD) pattern of the crystal form has at least three, at least four, at least five, at least six, or at least seven characteristic peaks at the following 2θ angles: 10.561°±0.2°, 18.089°±0.2°, 18.523°±0.2°, 19.026°±0.2°, 19.526°±0.2°, 22.768°±0.2°, and 22.967°±0.2°;

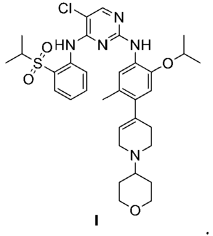

In one embodiment, the X-ray powder diffraction pattern of the one equivalent of mesylate crystal form E, measured using Cu-Kα radiation, has diffraction peaks at the following 2θ positions: 5.047°±0.2°, 10.561°±0.2°, 12.766°±0.2°, 12.98°±0.2°, 14.213°±0.2°, 14.641°±0.2°, 15.055°±0.2°, 15.786°±0.2°, 16.053°±0.2°, 16.323°±0.2°, 17.037°±0.2°, 17.845°±0.2°, 18.089°±0.2°, 18.523°±0.2°, 19.026°±0.2°, 19.526°±0.2°, 20.172°±0.2°, 20.653°±0.2°, 20.848°±0.2°, 21.072°±0.2°, 21.722°±0.2°, 22.144°±0.2°, 22.768°±0.2°, 22.967°±0.2°, 23.202°±0.2°, 23.417°±0.2°, 23.881°±0.2°, 24.427°±0.2°, 24.662°±0.2°, and 24.969°±0.2°.

In one embodiment, X-ray powder diffraction pattern analysis data of the one equivalent of mesylate crystal form E are substantially as shown in the following table:

| Position [°2θ]±0.2° | d-spacing [Å] | Relative intensity [%] | Position [°2θ]±0.2° | d-spacing [Å] | Relative intensity [%] |
|---|---|---|---|---|---|
| 5.047 | 17.4935 | 6.80% | 19.526 | 4.5426 | 44.30% |
| 10.561 | 8.3697 | 58.60% | 20.172 | 4.3985 | 26.20% |
| 12.766 | 6.9285 | 9.20% | 20.653 | 4.2972 | 11.10% |
| 12.98 | 6.8148 | 19.30% | 20.848 | 4.2575 | 23.20% |
| 14.213 | 6.2263 | 8.30% | 21.072 | 4.2126 | 15.00% |
| 14.641 | 6.0454 | 25.40% | 21.722 | 4.0881 | 21.80% |
| 15.055 | 5.88 | 31.80% | 22.144 | 4.0111 | 4.90% |
| 15.786 | 5.6095 | 43.40% | 22.768 | 3.9026 | 61.80% |
| 16.053 | 5.5167 | 15.80% | 22.967 | 3.8692 | 66.20% |
| 16.323 | 5.4259 | 35.50% | 23.202 | 3.8305 | 34.80% |
| 17.037 | 5.2002 | 13.10% | 23.417 | 3.7959 | 19.00% |
| 17.845 | 4.9665 | 5.80% | 23.881 | 3.7232 | 6.40% |
| 18.089 | 4.9001 | 55.90% | 24.427 | 3.6411 | 25.10% |
| 18.523 | 4.7862 | 100.00% | 24.662 | 3.6069 | 12.40% |
| 19.026 | 4.6607 | 50.00% | 24.969 | 3.5633 | 10.70% |

In one embodiment, a thermogravimetric analysis (TGA) curve of the one equivalent of mesylate crystal form E shows a weight loss of 1.181% during heating from 38.02 °C to 150.14 °C.

In one embodiment, a differential scanning calorimetry (DSC) curve of the one equivalent of mesylate crystal form E has endothermic peaks with initial temperatures of 89.99 °C, 101.61 °C, and 219.49 °C, respectively. In one embodiment, the differential scanning calorimetry (DSC) curve of the one equivalent of mesylate crystal form E has endothermic peaks with peak temperatures of 90.21 °C, 109.10 °C, and 221.65 °C, respectively. In one embodiment, ¹H-NMR results of the one equivalent of mesylate crystal form E show that methanesulfonic acid and a free base in a sample are in a molar ratio of 1:1, and residual 1,4-dioxane solvent and the free base are in a molar ratio of 0.12:1 and a weight ratio of 1.4%.

The present disclosure provides one equivalent of mesylate crystal form F of the compound represented by formula I, and an X-ray powder diffraction (XRPD) pattern of the crystal form has at least three, at least four, at least five, at least six, or at least seven characteristic peaks at the following 2θ angles: 10.219°±0.2°, 13.318°±0.2°, 16.777°±0.2°, 18.6°±0.2°, 18.802°±0.2°, 20.981°±0.2°, and 22.621°±0.2°;

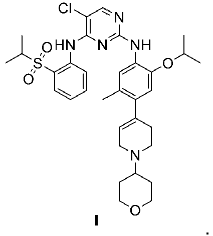

In one embodiment, the X-ray powder diffraction pattern of the one equivalent of mesylate crystal form F, measured using Cu-Kα radiation, has diffraction peaks at the following 2θ positions: 10.219°±0.2°, 10.535°±0.2°, 12.441°±0.2°, 13.318°±0.2°, 14.441°±0.2°, 14.978°±0.2°, 15.639°±0.2°, 16.14°±0.2°, 16.777°±0.2°, 17.697°±0.2°, 18.501°±0.2°, 18.6°±0.2°, 18.802°±0.2°, 19.859°±0.2°, 19.995°±0.2°, 20.5°±0.2°, 20.66°±0.2°, 20.981°±0.2°, 21.162°±0.2°, 21.337°±0.2°, 21.78°±0.2°, 22.079°±0.2°, 22.198°±0.2°, 22.621°±0.2°, 22.941°±0.2°, 23.723°±0.2°, 23.984°±0.2°, 24.321°±0.2°, 24.76°±0.2°, and 28.78°±0.2°.

In one embodiment, X-ray powder diffraction pattern analysis data of the one equivalent of mesylate crystal form F are substantially as shown in Table 24:

| Position [°2θ]±0.2° | d-spacing [Å] | Relative intensity [%] | Position [°2θ]+0.2° | d-spacing [Å] | Relative intensity [%] |
|---|---|---|---|---|---|
| 10.219 | 8.6491 | 56.00% | 20.5 | 4.3288 | 14.50% |
| 10.535 | 8.3905 | 14.70% | 20.66 | 4.2957 | 26.30% |
| 12.441 | 7.109 | 18.90% | 20.981 | 4.2308 | 40.70% |
| 13.318 | 6.6426 | 46.90% | 21.162 | 4.195 | 9.20% |
| 14.441 | 6.1286 | 7.80% | 21.337 | 4.161 | 15.70% |
| 14.978 | 5.9102 | 19.30% | 21.78 | 4.0773 | 25.20% |
| 15.639 | 5.6617 | 22.10% | 22.079 | 4.0228 | 4.80% |
| 16.14 | 5.4872 | 21.50% | 22.198 | 4.0015 | 8.70% |
| 16.777 | 5.2803 | 100.00% | 22.621 | 3.9275 | 58.90% |
| 17.697 | 5.0076 | 15.30% | 22.941 | 3.8734 | 24.00% |
| 18.501 | 4.7919 | 25.80% | 23.723 | 3.7476 | 8.40% |
| 18.6 | 4.7666 | 32.00% | 23.984 | 3.7074 | 2.80% |
| 18.802 | 4.7157 | 54.60% | 24.321 | 3.6567 | 8.60% |
| 19.859 | 4.467 | 17.10% | 24.76 | 3.5929 | 20.80% |
| 19.995 | 4.437 | 16.30% | 28.78 | 3.0995 | 10.10% |

In one embodiment, a thermogravimetric analysis (TGA) curve of the one equivalent of mesylate crystal form F shows a weight loss of 4.840% during heating from 39.51 °C to 150.96 °C.

In one embodiment, a differential scanning calorimetry (DSC) curve of the one equivalent of mesylate crystal form F has endothermic peaks with initial temperatures of 115.92 °C and 213.19 °C, respectively.

In one embodiment, the differential scanning calorimetry (DSC) curve of the one equivalent of mesylate crystal form F has endothermic peaks with peak temperatures of 127.70 °C and 217.04 °C, respectively.

In one embodiment, ¹H-NMR results of the one equivalent of mesylate crystal form F show that methanesulfonic acid and a free base in a sample are in a molar ratio of 1:1; residual 1,4-dioxane solvent and the free base are in a molar ratio of 0.43:1 and a weight ratio of 4.9%.

The present disclosure provides one equivalent of maleate crystal form A of the compound represented by formula I, and an X-ray powder diffraction (XRPD) pattern of the crystal form has at least three, at least four, at least five, at least six, or at least seven characteristic peaks at the following 2θ angles: 5.2344°±0.2°, 10.8375°±0.2°, 12.9364°±0.2°, 17.781°±0.2°, 18.6613°±0.2°, 18.9188°±0.2°, and 20.1186°±0.2°;

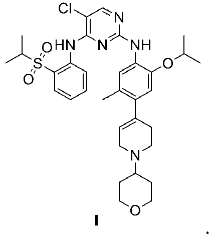

In one embodiment, the X-ray powder diffraction pattern of the one equivalent of maleate crystal form A, measured using Cu-Kα radiation, has diffraction peaks at the following 2θ positions: 17.781°±0.2°, 18.6613°±0.2°, and 20.1186°±0.2°.

In one embodiment, the X-ray powder diffraction pattern of the one equivalent of maleate crystal form A, measured using Cu-Kα radiation, has diffraction peaks at the following 2θ positions: 10.8375°±0.2°, 17.781°±0.2°, 18.6613°±0.2°, and 20.1186°±0.2°.

In one embodiment, the X-ray powder diffraction pattern of the one equivalent of maleate crystal form A, measured using Cu-Kα radiation, has diffraction peaks at the following 2θ positions: 10.8375°±0.2°, 17.781°±0.2°, 18.6613°±0.2°, 18.9188°±0.2°, and 20.1186°±0.2°.

In one embodiment, the X-ray powder diffraction pattern of the one equivalent of maleate crystal form A, measured using Cu-Kα radiation, has diffraction peaks at the following 2θ positions: 5.2344°+0.2°, 10.8375°±0.2°, 17.781°±0.2°, 18.6613°±0.2°, 18.9188°±0.2°, and 20.1186°±0.2°.

In one embodiment, the X-ray powder diffraction pattern of the one equivalent of maleate crystal form A, measured using Cu-Kα radiation, has diffraction peaks at the following 2θ positions: 5.2344°+0.2°, 10.8375°±0.2°, 12.9364°±0.2°, 17.781°±0.2°, 18.6613°±0.2°, 18.9188°±0.2°, and 20.1186°±0.2°.

In one embodiment, the X-ray powder diffraction pattern of the one equivalent of maleate crystal form A, measured using Cu-Kα radiation, has diffraction peaks at the following 2θ positions: 5.2344°±0.2°, 8.0562°±0.2°, 9.3187°±0.2°, 9.981°±0.2°, 10.4404°±0.2°, 10.8375°±0.2°, 12.5202°±0.2°, 12.9364°±0.2°, 13.9614°±0.2°, 14.9983°±0.2°, 15.6783°±0.2°, 17.781°±0.2°, 18.3794°±0.2°, 18.6613°±0.2°, 18.9188°±0.2°, 19.4195°±0.2°, 19.701°±0.2°, 20.1186°±0.2°, 20.5991°±0.2°, 20.9388°±0.2°, 22.6192°±0.2°, 22.739°±0.2°, 23.3011°±0.2°, 24.6412°±0.2°, 25.1802°±0.2°, 26.1581 °±0.2°, 28.061°±0.2°,29.7621°+0.2°,30.2013°±0.2°, and 30.9209°+0.2°.

In one embodiment, X-ray powder diffraction pattern analysis data of the one equivalent of maleate crystal form A are substantially as shown in the following table:

| Position [°2θ]±0.2° | d-spacing [Å] | Relative intensity [%] | Position [°20]±0.2° | d-spacing [Å] | Relative intensity [%] |
|---|---|---|---|---|---|
| 5.2344 | 16.86931 | 34.50% | 19.4195 | 4.56722 | 7.60% |
| 8.0562 | 10.96576 | 33.60% | 19.701 | 4.50259 | 19.40% |
| 9.3187 | 9.48276 | 12.10% | 20.1186 | 4.41008 | 60.80% |
| 9.981 | 8.85495 | 23.50% | 20.5991 | 4.30827 | 20.20% |
| 10.4404 | 8.4663 | 15.80% | 20.9388 | 4.23914 | 12.20% |
| 10.8375 | 8.15697 | 44.70% | 22.6192 | 3.92786 | 19.90% |
| 12.5202 | 7.06419 | 12.20% | 22.739 | 3.90744 | 22.70% |
| 12.9364 | 6.83786 | 34.10% | 23.3011 | 3.81444 | 23.60% |
| 13.9614 | 6.33807 | 23.10% | 24.6412 | 3.60994 | 11.20% |
| 14.9983 | 5.90214 | 15.70% | 25.1802 | 3.53388 | 26.50% |
| 15.6783 | 5.64762 | 27.00% | 26.1581 | 3.40394 | 27.50% |
| 17.781 | 4.98423 | 50.50% | 28.061 | 3.17728 | 16.40% |
| 18.3794 | 4.82328 | 31.40% | 29.7621 | 2.99944 | 20.70% |
| 18.6613 | 4.75104 | 100.00% | 30.2013 | 2.95681 | 4.20% |
| 18.9188 | 4.68697 | 40.80% | 30.9209 | 2.88962 | 9.30% |

In one embodiment, the X-ray powder diffraction pattern of the one equivalent of maleate crystal form A is substantially as shown in FIG. 10.

In one embodiment, a thermogravimetric analysis (TGA) curve of the one equivalent of maleate crystal form A shows a weight loss of 0.1441±0.005% during heating from 30.24±3 °C to 150.15±3 °C.

In one embodiment, the thermogravimetric analysis (TGA) curve of the one equivalent of maleate crystal form A shows a weight loss of 0.1441% during heating from 30.24 °C to 150.15 °C.

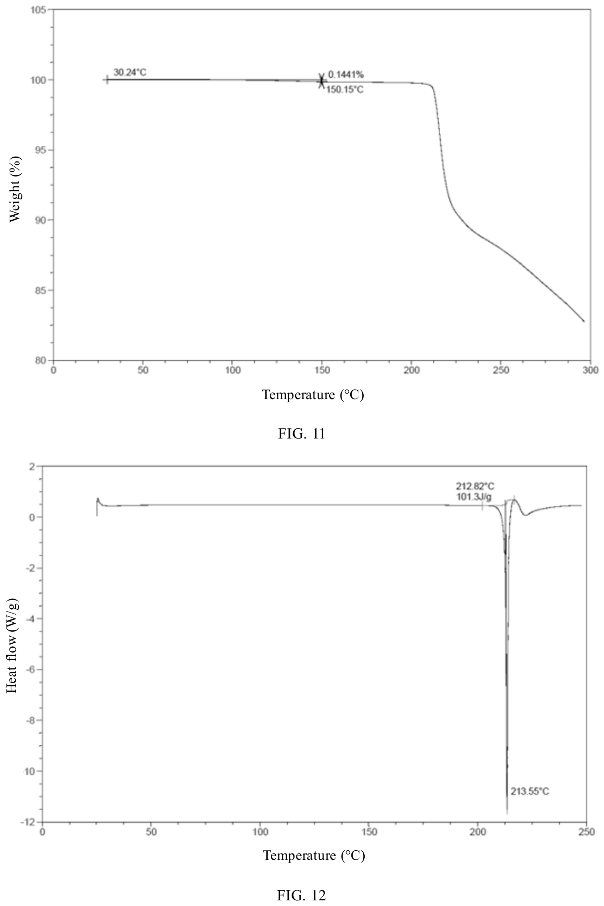

In one embodiment, the thermogravimetric analysis curve of the one equivalent of maleate crystal form A is substantially as shown in FIG. 11.

In one embodiment, a differential scanning calorimetry (DSC) curve of the one equivalent of maleate crystal form A has an endothermic peak with an initial temperature of 212.82±3 °C.

In one embodiment, the differential scanning calorimetry (DSC) curve of the one equivalent of maleate crystal form A has an endothermic peak with an initial temperature of 212.82 °C.

In one embodiment, the differential scanning calorimetry (DSC) curve of the one equivalent of maleate crystal form A has an endothermic peak with a peak temperature of 213.55±3 °C.

In one embodiment, the differential scanning calorimetry (DSC) curve of the one equivalent of maleate crystal form A has an endothermic peak with a peak temperature of 213.55 °C.

In one embodiment, the differential scanning calorimetry curve of the one equivalent of maleate crystal form A is substantially as shown in FIG. 12.

In one embodiment, a dynamic vapor sorption (DVS) curve of the one equivalent of maleate crystal form A shows a hygroscopic weight gain of 0.11±0.005% at 25 °C and 80% RH.

In one embodiment, the dynamic vapor sorption (DVS) curve of the one equivalent of maleate crystal form A shows a hygroscopic weight gain of 0.11% at 25 °C and 80% RH.

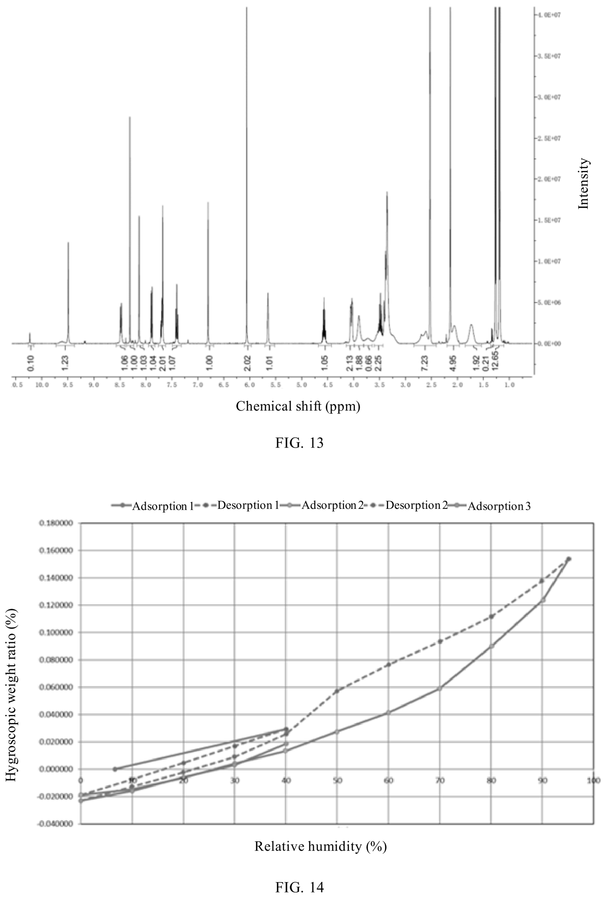

In one embodiment, the dynamic vapor sorption curve of the one equivalent of maleate crystal form A is as shown in FIG. 14.

In one embodiment, ¹H-NMR results of the one equivalent of maleate crystal form A show that maleic acid and a free base in a sample are in a molar ratio of 1:1.

In one embodiment, a proton nuclear magnetic resonance spectrum of the one equivalent of maleate crystal form A is shown in FIG. 13.

The present disclosure further provides a preparation method for the one equivalent of maleate crystal form A of the compound represented by formula I, which comprises the following steps: stirring an amorphous form of the compound represented by formula I and maleic acid in ethanol at 50 °C to obtain a suspension, and further stirring the obtained suspension at room temperature to precipitate a solid, so as to obtain the one equivalent of maleate crystal form A of the compound represented by formula I, wherein further stirring the suspension at room temperature is preferably performed at 25 °C; further stirring the suspension at room temperature is preferably performed for a period of 6-8 days, and further preferably 7 days; the amorphous form of the compound represented by formula I and maleic acid are preferably in a mass ratio of 5:1; the amorphous form of the compound represented by formula I and ethanol are preferably in a mass-to-volume ratio of (50-500 mg):1 mL, and further preferably 250 mg:1 mL.

In one embodiment, the preparation method for the one equivalent of maleate crystal form A may further comprise, after the solid is precipitated, the steps of separation and drying to obtain the one equivalent of maleate crystal form A.

The present disclosure provides one equivalent of maleate crystal form B of the compound represented by formula I, and an X-ray powder diffraction (XRPD) pattern of the crystal form has at least three, at least four, at least five, at least six, or at least seven characteristic peaks at the following 2θ angles: 15.941°±0.2°, 16.504°±0.2°, 17.24°±0.2°, 18.257°±0.2°, 20.861°±0.2°, 22.819°±0.2°, and 23.024°±0.2°;

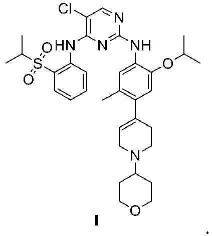

In one embodiment, the X-ray powder diffraction pattern of the one equivalent of maleate crystal form B, measured using Cu-Kα radiation, has diffraction peaks at the following 2θ positions: 7.67°±0.2°, 7.965°±0.2°, 10.358°±0.2°, 11.077°±0.2°, 13.06°±0.2°, 14.716°±0.2°, 15.149°±0.2°, 15.572°±0.2°, 15.941°±0.2°, 16.301°±0.2°, 16.504°±0.2°, 17.24°±0.2°, 18.257°±0.2°, 18.881°±0.2°, 20.012°±0.2°, 20.273°±0.2°, 20.861°±0.2°, 21.616°±0.2°, 22.044°±0.2°, 22.280°±0.2°, 22.558°±0.2°, 22.819°±0.2°, 23.024°±0.2°, 23.175°±0.2°, 23.599°±0.2°, 23.981°±0.2°, 25.755°±0.2°, 26.059°±0.2°, 26.532°±0.2°, and 26.983°±0.2°. In one embodiment, X-ray powder diffraction pattern analysis data of the one equivalent of maleate crystal form B are substantially as shown in the following table:

| Position [°2θ]+0.2° | d-spacing [Å] | Relative intensity [%] | Position [°2θ]±0.2° | d-spacing [Å] | Relative intensity [%] |
|---|---|---|---|---|---|
| 7.67 | 11.5166 | 2.60% | 20.273 | 4.3768 | 7.10% |
| 7.965 | 11.0912 | 4.80% | 20.861 | 4.2549 | 39.50% |
| 10.358 | 8.5335 | 21.90% | 21.616 | 4.1079 | 21.70% |
| 11.077 | 7.9813 | 2.90% | 22.044 | 4.0291 | 2.60% |
| 13.06 | 6.7732 | 2.40% | 22.280 | 3.9869 | 10.20% |
| 14.716 | 6.0146 | 8.90% | 22.558 | 3.9384 | 14.50% |
| 15.149 | 5.8438 | 3.50% | 22.819 | 3.8939 | 36.70% |
| 15.572 | 5.6858 | 17.80% | 23.024 | 3.8596 | 25.00% |
| 15.941 | 5.5553 | 45.00% | 23.175 | 3.835 | 15.30% |
| 16.301 | 5.4332 | 5.10% | 23.599 | 3.7669 | 12.20% |
| 16.504 | 5.367 | 24.40% | 23.981 | 3.7078 | 15.00% |
| 17.24 | 5.1394 | 58.80% | 25.755 | 3.4563 | 11.40% |
| 18.257 | 4.8553 | 100.00% | 26.059 | 3.4166 | 15.90% |
| 18.881 | 4.6961 | 9.00% | 26.532 | 3.3568 | 1.10% |
| 20.012 | 4.4361 | 19.20% | 26.983 | 3.3017 | 5.80% |

In one embodiment, a thermogravimetric analysis (TGA) curve of the one equivalent of maleate crystal form B shows a weight loss of 0. 1454% during heating from 30.45 °C to 150.43 °C.

In one embodiment, a differential scanning calorimetry (DSC) curve of the one equivalent of maleate crystal form B has an endothermic peak with an initial temperature of 214.44 °C.

In one embodiment, the differential scanning calorimetry (DSC) curve of the one equivalent of maleate crystal form B has an endothermic peak with a peak temperature of 215.00 °C.

In one embodiment, ¹H- NMR results of the one equivalent of maleate crystal form B show that maleic acid and a free base in a sample are in a molar ratio of 1:1.

The present disclosure provides one equivalent of tartrate crystal form A of the compound represented by formula I, and an X-ray powder diffraction (XRPD) pattern of the crystal form has at least three, at least four, at least five, at least six, or at least seven characteristic peaks at the following 2θ angles: 10.296°±0.2°, 11.744°±0.2°, 15.078°±0.2°, 18.321°±0.2°, 19.562°±0.2°, 20.32°±0.2°, and 21.6°±0.2°;

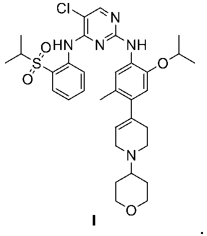

In one embodiment, the X-ray powder diffraction pattern of the one equivalent of tartrate crystal form A, measured using Cu-Kα radiation, has diffraction peaks at the following 2θ positions: 3.938°±0.2°, 9.562°±0.2°, 10.296°±0.2°, 10.836°±0.2°, 11.126°±0.2°, 11.744°±0.2°, 12.78°±0.2°, 13.908°±0.2°, 14.074°±0.2°, 15.078°±0.2°, 15.318°±0.2°, 15.714°±0.2°, 16.865°±0.2°, 17.358°±0.2°, 18.321°±0.2°, 18.856°±0.2°, 19.562°±0.2°, 20.32°±0.2°, 21.6°±0.2°, 22.619°±0.2°, 23.802°±0.2°, 23.98°±0.2°, 25.046°±0.2°, 25.311°+0.2°, 25.998°±0.2°, 26.5°+0.2°, 27.1°±0.2°, 27.827°±0.2°, 28.286°±0.2°, and 28.917°+0.2°.

In one embodiment, X-ray powder diffraction pattern analysis data of the one equivalent of tartrate crystal form A are substantially as shown in the following table:

| Position [°2θ]+0.2° | d-spacing [Å] | Relative intensity [%] | Position [°20]±0.2° | d-spacing [Å] | Relative intensity [%] |
|---|---|---|---|---|---|
| 3.938 | 22.4218 | 26.00% | 18.856 | 4.7024 | 6.30% |
| 9.562 | 9.2418 | 4.80% | 19.562 | 4.5342 | 100.00% |
| 10.296 | 8.5844 | 41.00% | 20.32 | 4.3668 | 56.20% |
| 10.836 | 8.1578 | 21.40% | 21.6 | 4.1108 | 35.50% |
| 11.126 | 7.9461 | 7.00% | 22.619 | 3.928 | 20.30% |
| 11.744 | 7.5294 | 47.90% | 23.802 | 3.7353 | 28.00% |
| 12.78 | 6.9214 | 15.90% | 23.98 | 3.7079 | 30.30% |
| 13.908 | 6.3621 | 3.30% | 25.046 | 3.5525 | 3.40% |
| 14.074 | 6.2875 | 5.20% | 25.311 | 3.5159 | 3.40% |
| 15.078 | 5.8712 | 45.20% | 25.998 | 3.4245 | 13.80% |
| 15.318 | 5.7797 | 19.70% | 26.5 | 3.3608 | 7.50% |
| 15.714 | 5.635 | 16.40% | 27.1 | 3.2878 | 4.20% |
| 16.865 | 5.2528 | 5.60% | 27.827 | 3.2035 | 5.40% |
| 17.358 | 5.1047 | 10.20% | 28.286 | 3.1525 | 2.70% |
| 18.321 | 4.8385 | 63.80% | 28.917 | 3.0851 | 23.50% |

In one embodiment, a thermogravimetric analysis (TGA) curve of the one equivalent of tartrate crystal form A shows a weight loss of 2.467% during heating from 28.23 °C to 150.30 °C.

In one embodiment, a differential scanning calorimetry (DSC) curve of the one equivalent of tartrate crystal form A has endothermic peaks with initial temperatures of 28.77 °C, 118.07 °C, and 214.88 °C, respectively. In one embodiment, the differential scanning calorimetry (DSC) curve of the one equivalent of tartrate crystal form A has endothermic peaks with peak temperatures of 51.87 °C, 130.04 °C, and 223.29 °C, respectively.

The present disclosure provides one equivalent of tartrate crystal form B of the compound represented by formula I, and an X-ray powder diffraction (XRPD) pattern of the crystal form has at least three, at least four, at least five, at least six, or at least seven characteristic peaks at the following 2θ angles: 8.817°±0.2°, 10.879°±0.2°, 16.161°+0.2°, 17.421°+0.2°, 17.657°+0.2°, 18.018°±0.2°, and 19.182°±0.2°;

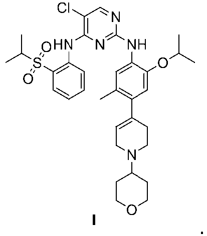

In one embodiment, the X-ray powder diffraction pattern of the one equivalent of tartrate crystal form B, measured using Cu-Kα radiation, has diffraction peaks at the following 2θ positions: 8.423°±0.2°, 8.817°±0.2°, 9.406°+0.2°, 9.859°+0.2°, 10.331°±0.2°, 10.879°+0.2°, 11.359°±0.2°, 12.193°±0.2°, 12.52°+0.2°, 12.898°+0.2°, 13.036°+0.2°, 13.218°±0.2°, 13.36°±0.2°, 14.283°+0.2°, 15.039°+0.2°, 15.579°±0.2°, 16.161°±0.2°, 16.321°±0.2°, 16.879°±0.2°, 17.002°±0.2°, 17.421°±0.2°, 17.657°±0.2°, 18.018°±0.2°, 19.062°±0.2°, 19.182°±0.2°, 19.422°±0.2°, 19.76°±0.2°, 20.076°±0.2°, 20.34°±0.2°, and 21.04°±0.2°.

In one embodiment, X-ray powder diffraction pattern analysis data of the one equivalent of tartrate crystal form B are substantially as shown in the following table:

| Position [°2θ]+0.2° | d-spacing [Å] | Relative intensity [%] | Position [°2θ]±0.2° | d-spacing [Å] | Relative intensity [%] |
|---|---|---|---|---|---|
| 8.423 | 10.4893 | 6.00% | 15.579 | 5.6833 | 1.60% |
| 8.817 | 10.0208 | 100.00% | 16.161 | 5.4802 | 30.90% |
| 9.406 | 9.3945 | 2.20% | 16.321 | 5.4266 | 6.90% |
| 9.859 | 8.9643 | 23.80% | 16.879 | 5.2485 | 8.80% |
| 10.331 | 8.5553 | 1.20% | 17.002 | 5.2108 | 14.80% |
| 10.879 | 8.126 | 50.70% | 17.421 | 5.0865 | 62.40% |
| 11.359 | 7.7837 | 5.10% | 17.657 | 5.0188 | 53.90% |
| 12.193 | 7.2529 | 2.70% | 18.018 | 4.9192 | 60.90% |
| 12.52 | 7.0641 | 2.10% | 19.062 | 4.6521 | 27.70% |
| 12.898 | 6.858 | 9.10% | 19.182 | 4.6233 | 44.70% |
| 13.036 | 6.7859 | 3.00% | 19.422 | 4.5666 | 9.30% |
| 13.218 | 6.6929 | 4.60% | 19.76 | 4.4892 | 30.00% |
| 13.36 | 6.6221 | 3.30% | 20.076 | 4.4193 | 28.90% |
| 14.283 | 6.1962 | 7.10% | 20.34 | 4.3626 | 26.30% |
| 15.039 | 5.8862 | 25.90% | 21.04 | 4.219 | 17.80% |

In one embodiment, a thermogravimetric analysis (TGA) curve of the one equivalent of tartrate crystal form B shows a weight loss of 7.235% during heating from 40.16 °C to 150.16 °C.

In one embodiment, a differential scanning calorimetry (DSC) curve of the one equivalent of tartrate crystal form B has endothermic peaks with initial temperatures of 118.67 °C and 205.16 °C, respectively.

In one embodiment, the differential scanning calorimetry (DSC) curve of the one equivalent of tartrate crystal form B has endothermic peaks with peak temperatures of 128.63 °C and 222.35 °C, respectively.

In one embodiment, ¹H-NMR results of the one equivalent of tartrate crystal form B show that tartaric acid and a free base in a sample are in a molar ratio of 1:1, and residual tetrahydrofuran solvent and the free base are in a molar ratio of 0.99:1 and a weight ratio of 8.3%.

The present disclosure provides one equivalent of tartrate crystal form C of the compound represented by formula I, and an X-ray powder diffraction (XRPD) pattern of the crystal form has at least three, at least four, at least five, at least six, or at least seven characteristic peaks at the following 2θ angles: 10.196°±0.2°, 15.543°±0.2°, 15.757°+0.2°, 17.004°+0.2°, 18.196°+0.2°, 20.699°+0.2°, and 21.665°+0.2°;

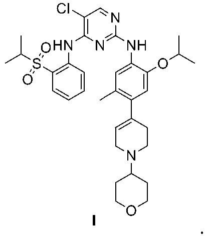

In one embodiment, the X-ray powder diffraction pattern of the one equivalent of tartrate crystal form C, measured using Cu-Kα radiation, has diffraction peaks at the following 2θ positions: 10.196°±0.2°, 15.543°±0.2°, and 18.196°±0.2°.

In one embodiment, the X-ray powder diffraction pattern of the one equivalent of tartrate crystal form C, measured using Cu-Kα radiation, has diffraction peaks at the following 2θ positions: 10.196°±0.2°, 15.543°±0.2°, 17.004°±0.2°, and 18.196°±0.2°.

In one embodiment, the X-ray powder diffraction pattern of the one equivalent of tartrate crystal form C, measured using Cu-Kα radiation, has diffraction peaks at the following 2θ positions: 10.196°±0.2°, 15.543°±0.2°, 17.004°±0.2°, 18.196°±0.2°, and 20.699°±0.2°.

In one embodiment, the X-ray powder diffraction pattern of the one equivalent of tartrate crystal form C, measured using Cu-Kα radiation, has diffraction peaks at the following 2θ positions: 10.196°+0.2°, 15.543°±0.2°, 15.757°+0.2°, 17.004°±0.2°, 18.196°±0.2°, and 20.699°±0.2°.

In one embodiment, the X-ray powder diffraction pattern of the one equivalent of tartrate crystal form C, measured using Cu-Kα radiation, has diffraction peaks at the following 2θ positions: 10.196°±0.2°, 15.543°±0.2°, 15.757°+0.2°, 17.004°±0.2°, 18.196°±0.2°, 20.699°±0.2°, and 21.665°±0.2°.

In one embodiment, the X-ray powder diffraction pattern of the one equivalent of tartrate crystal form C, measured using Cu-Kα radiation, has diffraction peaks at the following 2θ positions: 8.48°±0.2°, 10.196°±0.2°, 14.537°±0.2°, 15.052°+0.2°, 15.277°+0.2°, 15.543°+0.2°, 15.757°+0.2°, 16.003°+0.2°, 16.377°±0.2°, 17.004°±0.2°, 18.196°±0.2°, 18.782°±0.2°, 19.226°±0.2°, 19.608°±0.2°, 20.001°±0.2°, 20.699°±0.2°, 21.665°±0.2°, 22.058°±0.2°, 22.545°±0.2°, 22.875°±0.2°, 23.097°±0.2°, 23.505°±0.2°, 23.819°±0.2°, 24.313°±0.2°, 24.642°±0.2°, 25.027°±0.2°, 25.699°±0.2°, 26.666°±0.2°, 26.867°±0.2°, and 27.857°±0.2°. In one embodiment, X-ray powder diffraction pattern analysis data of the one equivalent of tartrate crystal form C are substantially as shown in the following table:

| Position [°2θ]±0.2° | d-spacing [Å] | Relative intensity [%] | Position [°20]±0.2° | d-spacing [Å] | Relative intensity [%] |
|---|---|---|---|---|---|
| 8.48 | 10.419 | 1.60% | 20.699 | 4.2877 | 37.10% |
| 10.196 | 8.669 | 44.50% | 21.665 | 4.0987 | 30.00% |
| 14.537 | 6.0882 | 14.50% | 22.058 | 4.0264 | 25.80% |
| 15.052 | 5.8813 | 1.30% | 22.545 | 3.9407 | 25.90% |
| 15.277 | 5.7951 | 6.50% | 22.875 | 3.8845 | 24.30% |
| 15.543 | 5.6965 | 48.50% | 23.097 | 3.8477 | 2.90% |
| 15.757 | 5.6195 | 32.10% | 23.505 | 3.7818 | 15.00% |
| 16.003 | 5.5339 | 16.80% | 23.819 | 3.7327 | 18.50% |
| 16.377 | 5.4083 | 24.60% | 24.313 | 3.6578 | 2.40% |
| 17.004 | 5.2103 | 39.50% | 24.642 | 3.6098 | 3.50% |
| 18.196 | 4.8714 | 100.00% | 25.027 | 3.5552 | 2.70% |
| 18.782 | 4.7208 | 16.10% | 25.699 | 3.4637 | 21.30% |
| 19.226 | 4.6128 | 2.70% | 26.666 | 3.3402 | 4.80% |
| 19.608 | 4.5238 | 3.20% | 26.867 | 3.3157 | 4.00% |
| 20.001 | 4.4357 | 29.00% | 27.857 | 3.2001 | 1.30% |

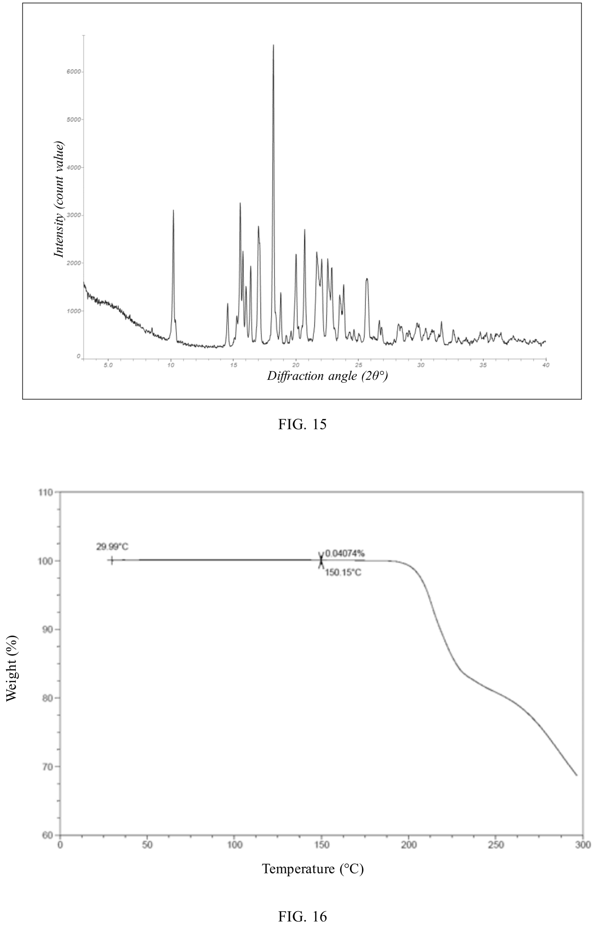

In one embodiment, the X-ray powder diffraction pattern of the one equivalent of tartrate crystal form C is substantially as shown in FIG. 15.

In one embodiment, a thermogravimetric analysis (TGA) curve of the one equivalent of tartrate crystal form C shows a weight loss of 0.04074±0.005% during heating from 29.99±3 °C to 150.15±3 °C.

In one embodiment, the thermogravimetric analysis (TGA) curve of the one equivalent of tartrate crystal form C shows a weight loss of 0.04074% during heating from 29.99 °C to 150.15 °C.

In one embodiment, the thermogravimetric analysis curve of the one equivalent of tartrate crystal form C is substantially as shown in FIG. 16.

In one embodiment, a differential scanning calorimetry (DSC) curve of the one equivalent of tartrate crystal form C has an endothermic peak with an initial temperature of 188.73±3 °C.

In one embodiment, the differential scanning calorimetry (DSC) curve of the one equivalent of tartrate crystal form C has an endothermic peak with an initial temperature of 188.73 °C.

In one embodiment, the differential scanning calorimetry (DSC) curve of the one equivalent of tartrate crystal form C has an endothermic peak with a peak temperature of 190.42±3 °C.

In one embodiment, the differential scanning calorimetry (DSC) curve of the one equivalent of tartrate crystal form C has an endothermic peak with a peak temperature of 190.42 °C.

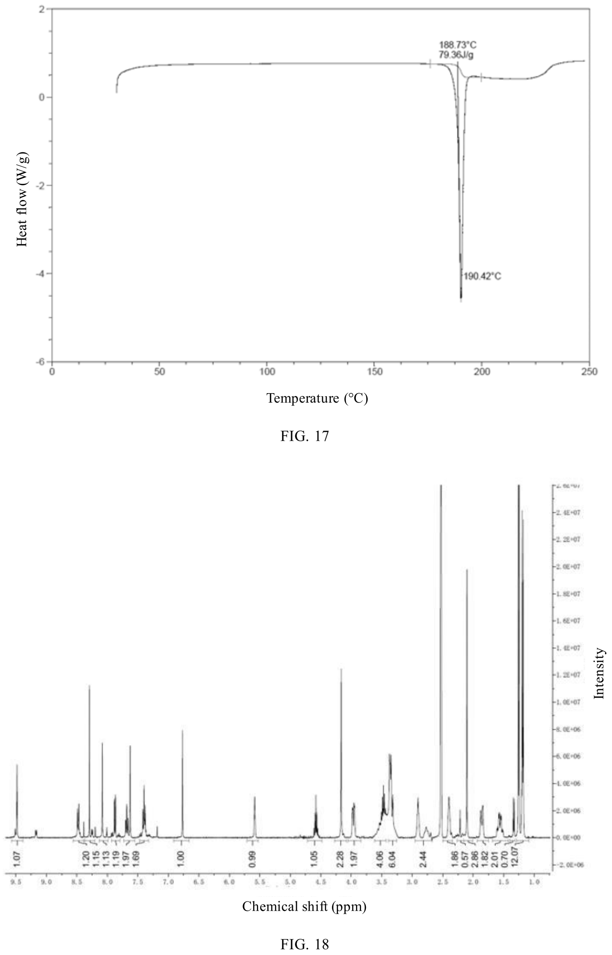

In one embodiment, the differential scanning calorimetry curve of the one equivalent of tartrate crystal form C is substantially as shown in FIG. 17.

In one embodiment, ¹H-NMR results of the one equivalent of tartrate crystal form C show that tartaric acid and a free base in a sample are in a molar ratio of 1:1, with no solvent remaining.

In one embodiment, a proton nuclear magnetic resonance spectrum of the one equivalent of tartrate crystal form C is shown in FIG. 18.

The present disclosure further provides a preparation method for the one equivalent of tartrate crystal form C of the compound represented by formula I, preferably any one of the following methods:
method I comprising the following steps: stirring the one equivalent of tartrate crystal form B in a solvent to precipitate a solid, so as to obtain the one equivalent of tartrate crystal form C of the compound represented by formula I, wherein the solvent is acetonitrile; the stirring is preferably performed at 35-45 °C; the stirring is preferably performed for a period of 2-7 days; the compound represented by formula I and the solvent are preferably in a mass-to-volume ratio of (50 mg-500 mg):1 mL; and
method II comprising the following steps: stirring the one equivalent of tartrate crystal form B in dichloromethane to obtain a clear solution, adding n-heptane, and stirring the mixture to precipitate a solid, so as to obtain the one equivalent of tartrate crystal form C of the compound represented by formula I, wherein the stirring is preferably performed at room temperature; the stirring is preferably performed for a period of 3-5 days; the one equivalent of tartrate crystal form B and the good solvent are preferably in a mass-to-volume ratio of (50 mg-500 mg):1 mL; the good solvent and the anti-solvent are preferably in a volume ratio of 1:(4-5).

In one embodiment, in method I, the stirring is preferably performed for a period of 3 days; the stirring is preferably performed at 40 °C; the one equivalent of tartrate crystal form B and the solvent are preferably in a mass-to-volume ratio of 250 mg:1 mL.

In one embodiment, in method II, the stirring is preferably performed at 25 °C; the stirring is preferably performed for a period of 5 days; the one equivalent of tartrate crystal form B and dichloromethane are preferably in a mass-to-volume ratio of 50 mg:1 mL; dichloromethane and n-heptane are preferably in a volume ratio of 1:4.

In one embodiment, the preparation method for the one equivalent of tartrate crystal form C may further comprise, after the solid is precipitated, the steps of separation and drying to obtain the one equivalent of tartrate crystal form C.

The present disclosure provides one equivalent of tartrate crystal form D of the compound represented by formula I, and an X-ray powder diffraction (XRPD) pattern of the crystal form has at least three, at least four, at least five, at least six, or at least seven characteristic peaks at the following 2θ angles: 8.762°±0.2°, 15.257°+0.2°, 16.537°+0.2°, 17.539°+0.2°, 19.237°+0.2°, 20.401°±0.2°, and 23.817°±0.2°;

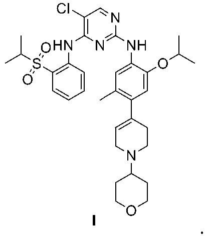

In one embodiment, the X-ray powder diffraction pattern of the one equivalent of tartrate crystal form D, measured using Cu-Kα radiation, has diffraction peaks at the following 2θ positions: 8.762°±0.2°,9.243°±0.2°, 11.323°±0.2°, 11.682°±0.2°, 13.705°±0.2°, 14.852°±0.2°, 15.257°±0.2°, 15.519°±0.2°, 16.065°±0.2°, 16.537°±0.2°, 17.539°±0.2°, 19.237°+0.2°, 19.698°±0.2°, 20.066°±0.2°, 20.401°±0.2°, 21.144°±0.2°, 21.339°±0.2°, 21.856°±0.2°, 21.992°±0.2°, 22.977°±0.2°, 23.817°±0.2°, 24.081°±0.2°, 25.043°±0.2°, 26.068°±0.2°, 26.676°±0.2°, 27.702°±0.2°, 30.799°±0.2°, 31.764°±0.2°, 32.035°±0.2°, and 32.63°±0.2°. In one embodiment, X-ray powder diffraction pattern analysis data of the one equivalent of tartrate crystal form D are substantially as shown in the following table:

| Position [°2θ]+0.2° | d-spacing [Å] | Relative intensity [%] | Position [°2θ]±0.2° | d-spacing [Å] | Relative intensity [%] |
|---|---|---|---|---|---|
| 8.762 | 10.0835 | 57.20% | 21.144 | 4.1985 | 22.10% |
| 9.243 | 9.5603 | 33.80% | 21.339 | 4.1605 | 29.70% |
| 11.323 | 7.8086 | 34.00% | 21.856 | 4.0633 | 26.90% |
| 11.682 | 7.5691 | 10.30% | 21.992 | 4.0384 | 16.80% |
| 13.705 | 6.4558 | 13.90% | 22.977 | 3.8676 | 18.80% |
| 14.852 | 5.9598 | 40.30% | 23.817 | 3.733 | 100.00% |
| 15.257 | 5.8027 | 63.90% | 24.081 | 3.6926 | 53.40% |
| 15.519 | 5.7054 | 11.90% | 25.043 | 3.5529 | 30.70% |
| 16.065 | 5.5126 | 29.10% | 26.068 | 3.4154 | 10.70% |
| 16.537 | 5.3563 | 59.40% | 26.676 | 3.3391 | 32.90% |
| 17.539 | 5.0525 | 59.80% | 27.702 | 3.2176 | 10.40% |
| 19.237 | 4.6102 | 66.70% | 30.799 | 2.9007 | 21.90% |
| 19.698 | 4.5033 | 20.10% | 31.764 | 2.8148 | 8.40% |
| 20.066 | 4.4216 | 10.10% | 32.035 | 2.7916 | 2.50% |
| 20.401 | 4.3497 | 61.00% | 32.63 | 2.7421 | 4.00% |

In one embodiment, a thermogravimetric analysis (TGA) curve of the one equivalent of tartrate crystal form D shows a weight loss of 5.376% during heating from 30.04 °C to 175.17 °C.

In one embodiment, a differential scanning calorimetry (DSC) curve of the one equivalent of tartrate crystal form D has endothermic peaks with initial temperatures of 123.48 °C and 179.70 °C, respectively.

In one embodiment, the differential scanning calorimetry (DSC) curve of the one equivalent of tartrate crystal form D has endothermic peaks with peak temperatures of 130.10 °C and 221.00 °C.

In one embodiment, ¹H-NMR results of the one equivalent of tartrate crystal form D show that tartaric acid and a free base in a sample are in a molar ratio of 1:1, and residual ethyl acetate solvent and the free base are in a molar ratio of 0.34:1 and a weight ratio of 3.69%.

The present disclosure provides one equivalent of tartrate crystal form E of the compound represented by formula I, and an X-ray powder diffraction (XRPD) pattern of the crystal form has at least three, at least four, at least five, at least six, or at least seven characteristic peaks at the following 2θ angles: 11.677°±0.2°, 15.562°±0.2°, 18.562°±0.2°, 19.2°±0.2°, 19.461°±0.2°, 20.463°±0.2°, and 23.421°±0.2°;

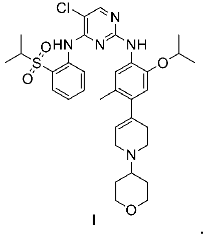

In one embodiment, the X-ray powder diffraction pattern of the one equivalent of tartrate crystal form E, measured using Cu-Kα radiation, has diffraction peaks at the following 2θ positions: 3.899°±0.2°, 10.198°±0.2°, 10.717°±0.2°, 11.12°±0.2°, 11.399°±0.2°, 11.677°±0.2°, 12.781°±0.2°, 13.06°±0.2°, 13.699°±0.2°, 14.901°±0.2°, 15.562°±0.2°, 16.401°±0.2°, 17.095°±0.2°, 17.321°±0.2°, 17.76°±0.2°, 18.562°±0.2°, 18.972°±0.2°, 19.2°±0.2°, 19.461°±0.2°, 20.119°+0.2°, 20.463°±0.2°, 20.839°±0.2°, 21.24°±0.2°, 21.58°±0.2°, 22.518°±0.2°, 22.875°±0.2°, 23.421°±0.2°, 23.637°±0.2°, 24.143°±0.2°, and 24.318°±0.2°.

In one embodiment, X-ray powder diffraction pattern analysis data of the one equivalent of tartrate crystal form E are substantially as shown in the following table:

| Position [°20]±0.2° | d-spacing [Å] | Relative intensity [%] | Position [°2θ]±0.2° | d-spacing [Å] | Relative intensity [%] |
|---|---|---|---|---|---|
| 3.899 | 22.6447 | 19.50% | 18.562 | 4.7762 | 97.80% |
| 10.198 | 8.6668 | 31.40% | 18.972 | 4.6738 | 4.40% |
| 10.717 | 8.2488 | 24.70% | 19.2 | 4.6189 | 33.60% |
| 11.12 | 7.9504 | 6.10% | 19.461 | 4.5577 | 100.00% |
| 11.399 | 7.7565 | 7.30% | 20.119 | 4.4099 | 20.40% |
| 11.677 | 7.5726 | 50.90% | 20.463 | 4.3366 | 48.00% |
| 12.781 | 6.9205 | 12.20% | 20.839 | 4.2592 | 5.40% |
| 13.06 | 6.7736 | 18.00% | 21.24 | 4.1797 | 15.50% |
| 13.699 | 6.4587 | 4.20% | 21.58 | 4.1146 | 30.90% |
| 14.901 | 5.9405 | 30.70% | 22.518 | 3.9453 | 13.00% |
| 15.562 | 5.6897 | 32.70% | 22.875 | 3.8846 | 5.50% |
| 16.401 | 5.4005 | 12.70% | 23.421 | 3.7952 | 36.50% |
| 17.095 | 5.1825 | 14.10% | 23.637 | 3.761 | 15.00% |
| 17.321 | 5.1154 | 5.80% | 24.143 | 3.6834 | 23.80% |
| 17.76 | 4.9901 | 10.20% | 24.318 | 3.6572 | 16.90% |

In one embodiment, a thermogravimetric analysis (TGA) curve of the one equivalent of tartrate crystal form E shows a weight loss of 7.196% during heating from 29.88 °C to 175.45 °C.

In one embodiment, a differential scanning calorimetry (DSC) curve of the one equivalent of tartrate crystal form E has an endothermic peak with an initial temperature of 112.11 °C.

In one embodiment, the differential scanning calorimetry (DSC) curve of the one equivalent of tartrate crystal form E has an endothermic peak with a peak temperature of 122.45 °C.

In one embodiment, ¹H-NMR results of the one equivalent of tartrate crystal form E show that tartaric acid and a free base in a sample are in a molar ratio of 1:1, and residual ethanol solvent and the free base are in a molar ratio of 0.88:1 and a weight ratio of 4.85%.

The present disclosure provides one equivalent of tartrate crystal form F of the compound represented by formula I, and an X-ray powder diffraction (XRPD) pattern of the crystal form has at least three, at least four, at least five, at least six, or at least seven characteristic peaks at the following 2θ angles: 10.098°±0.2°, 10.579°±0.2°, 18.341°±0.2°, 18.618°±0.2°, 19.36°±0.2°, 20.204°±0.2°, and 20.563°±0.2°;

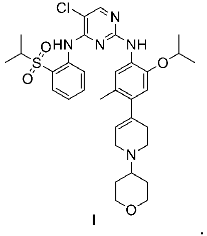

In one embodiment, the X-ray powder diffraction pattern of the one equivalent of tartrate crystal form F, measured using Cu-Kα radiation, has diffraction peaks at the following 20 positions: 5.052°+0.2°, 8.502°+0.2°, 9.085°+0.2°, 10.098°±0.2°, 10.579°+0.2°, 11.483°+0.2°, 12.663°+0.2°, 13.13°+0.2°, 13.998°+0.2°, 14.102°±0.2°, 15.172°±0.2°, 15.6°±0.2°, 16.693°±0.2°, 17.076°±0.2°, 17.762°±0.2°, 18.341°±0.2°, 18.618°±0.2°, 19.36°±0.2°, 20.204°±0.2°, 20.563°±0.2°, 20.977°±0.2°, 21.997°±0.2°, 22.508°±0.2°, 23.68°+0.2°, 25.14°+0.2°, 25.46°+0.2°, 25.675°+0.2°, 26.401°±0.2°, 28.005°±0.2°, and 28.201°±0.2°.

In one embodiment, X-ray powder diffraction pattern analysis data of the one equivalent of tartrate crystal form F are substantially as shown in the following table:

| Position [°2θ]±0.2° | d-spacing [Å] | Relative intensity [%] | Position [°2θ]+0.2° | d-spacing [Å] | Relative intensity [%] |
|---|---|---|---|---|---|
| 5.052 | 17.4762 | 40.80% | 18.341 | 4.8333 | 44.40% |
| 8.502 | 10.3919 | 30.60% | 18.618 | 4.7619 | 44.40% |
| 9.085 | 9.7266 | 17.40% | 19.36 | 4.5812 | 68.60% |
| 10.098 | 8.7525 | 100.00% | 20.204 | 4.3915 | 89.40% |
| 10.579 | 8.3557 | 85.40% | 20.563 | 4.3157 | 55.00% |
| 11.483 | 7.6995 | 43.90% | 20.977 | 4.2315 | 20.50% |
| 12.663 | 6.9851 | 6.70% | 21.997 | 4.0375 | 13.40% |
| 13.13 | 6.7376 | 12.30% | 22.508 | 3.9471 | 8.70% |
| 13.998 | 6.3213 | 6.90% | 23.68 | 3.7543 | 13.90% |
| 14.102 | 6.2754 | 5.70% | 25.14 | 3.5395 | 10.40% |
| 15.172 | 5.8348 | 10.00% | 25.46 | 3.4957 | 21.90% |
| 15.6 | 5.6757 | 27.50% | 25.675 | 3.4669 | 10.10% |
| 16.693 | 5.3066 | 6.50% | 26.401 | 3.3732 | 37.50% |
| 17.076 | 5.1883 | 9.10% | 28.005 | 3.1835 | 12.60% |
| 17.762 | 4.9897 | 37.50% | 28.201 | 3.1618 | 18.80% |

In one embodiment, a thermogravimetric analysis (TGA) curve of the one equivalent of tartrate crystal form F shows a weight loss of 3.174% during heating from 30.17 °C to 175.17 °C.

In one embodiment, a differential scanning calorimetry (DSC) curve of the one equivalent of tartrate crystal form F has endothermic peaks with initial temperatures of 30.67 °C, 89.32 °C, and 142.85 °C, respectively. In one embodiment, the differential scanning calorimetry (DSC) curve of the one equivalent of tartrate crystal form F has endothermic peaks with peak temperatures of 36.44 °C, 118.40 °C, and 150.31 °C, respectively. In one embodiment, ¹H-NMR results of the one equivalent of tartrate crystal form F show that tartaric acid and a free base in a sample are in a molar ratio of 1:1, and residual methanol solvent and the free base are in a molar ratio of 0.74:1 and a weight ratio of 2.90%.

The present disclosure provides one equivalent of citrate crystal form A of the compound represented by formula I, and an X-ray powder diffraction (XRPD) pattern of the crystal form has at least three, at least four, at least five, at least six, or at least seven characteristic peaks at the following 2θ angles: 8.656°±0.2°, 13.455°±0.2°, 17.158°+0.2°, 18.093°±0.2°, 18.64°+0.2°, 21.819°+0.2°, and 22.916°+0.2°;

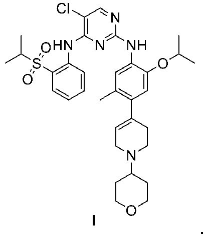

In one embodiment, the X-ray powder diffraction pattern of the one equivalent of citrate crystal form A, measured using Cu-Kα radiation, has diffraction peaks at the following 2θ positions: 4.351 °±0.2°, 7.501°±0.2°, 8.656°±0.2°, 9.991°±0.2°, 11.372°±0.2°, 11.663°±0.2°, 12.631°±0.2°, 13.065°±0.2°, 13.455°±0.2°, 13.703°±0.2°, 14.171°±0.2°, 15.113°±0.2°, 15.718°±0.2°, 17.158°±0.2°, 17.317°±0.2°, 17.715°±0.2°, 18.093°±0.2°, 18.64°±0.2°, 19.577°±0.2°, 19.77°±0.2°, 20.118°±0.2°, 20.403°±0.2°, 20.805°±0.2°, 21.122°±0.2°, 21.819°±0.2°, 22.916°±0.2°, 23.367°±0.2°, 23.637°±0.2°, 24.236°±0.2°, and 25.379°±0.2°. In one embodiment, X-ray powder diffraction pattern analysis data of the one equivalent of citrate crystal form A are substantially as shown in the following table:

| Position [°2θ]+0.2° | d-spacing [Å] | Relative intensity [%] | Position [°2θ]±0.2° | d-spacing [Å] | Relative intensity [%] |
|---|---|---|---|---|---|
| 4.351 | 20.291 | 29.50% | 17.715 | 5.0026 | 34.00% |
| 7.501 | 11.7767 | 25.80% | 18.093 | 4.899 | 42.80% |
| 8.656 | 10.2069 | 99.80% | 18.64 | 4.7564 | 48.10% |
| 9.991 | 8.8465 | 2.40% | 19.577 | 4.5308 | 7.00% |
| 11.372 | 7.7747 | 20.40% | 19.77 | 4.4869 | 4.40% |
| 11.663 | 7.5817 | 7.30% | 20.118 | 4.4102 | 4.30% |
| 12.631 | 7.0026 | 5.90% | 20.403 | 4.3492 | 16.60% |
| 13.065 | 6.7706 | 3.40% | 20.805 | 4.2661 | 3.90% |
| 13.455 | 6.5754 | 100.00% | 21.122 | 4.2028 | 33.20% |
| 13.703 | 6.4571 | 24.70% | 21.819 | 4.07 | 88.00% |
| 14.171 | 6.2448 | 5.50% | 22.916 | 3.8776 | 49.70% |
| 15.113 | 5.8577 | 25.90% | 23.367 | 3.8038 | 18.20% |
| 15.718 | 5.6333 | 23.20% | 23.637 | 3.761 | 11.90% |
| 17.158 | 5.1639 | 59.70% | 24.236 | 3.6694 | 18.90% |
| 17.317 | 5.1167 | 30.90% | 25.379 | 3.5066 | 19.10% |

In one embodiment, a thermogravimetric analysis (TGA) curve of the one equivalent of citrate crystal form A shows a weight loss of 0.7543% during heating from 29.61 °C to 150.16 °C.

In one embodiment, a differential scanning calorimetry (DSC) curve of the one equivalent of citrate crystal form A has endothermic peaks with initial temperatures of 60.16 °C, 151.22 °C, 156.20 °C, and 167.07 °C, respectively.

In one embodiment, the differential scanning calorimetry (DSC) curve of the one equivalent of citrate crystal form A has endothermic peaks with peak temperatures of 95.36 °C, 152.85 °C, 160.14 °C, and 185.35 °C, respectively.

In one embodiment, ¹H-NMR results of the one equivalent of citrate crystal form A show that citric acid and a free base in a sample are in a molar ratio of 1:1, with no solvent remaining.

The present disclosure provides one equivalent of citrate crystal form B of the compound represented by formula I, and an X-ray powder diffraction (XRPD) pattern of the crystal form has at least three, at least four, at least five, at least six, or at least seven characteristic peaks at the following 2θ angles: 15.0928°±0.2°, 16.8372°±0.2°, 18.5382°±0.2°, 19.194°±0.2°, 21.4798°±0.2°, 23.6411°±0.2°, and 26.9181°±0.2°;

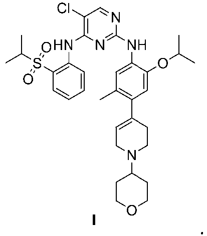

In one embodiment, the X-ray powder diffraction pattern of the one equivalent of citrate crystal form B, measured using Cu-Kα radiation, has diffraction peaks at the following 2θ positions: 16.8372°±0.2°, 21.4798°±0.2°, and 23.6411°±0.2°.

In one embodiment, the X-ray powder diffraction pattern of the one equivalent of citrate crystal form B, measured using Cu-Kα radiation, has diffraction peaks at the following 2θ positions: 15.0928°±0.2°, 16.8372°±0.2°, 21.4798°±0.2°, and 23.6411°±0.2°.

In one embodiment, the X-ray powder diffraction pattern of the one equivalent of citrate crystal form B, measured using Cu-Kα radiation, has diffraction peaks at the following 2θ positions: 15.0928°±0.2°, 16.8372°±0.2°, 19.194°±0.2°, 21.4798°±0.2°, and 23.6411°±0.2°.

In one embodiment, the X-ray powder diffraction pattern of the one equivalent of citrate crystal form B, measured using Cu-Kα radiation, has diffraction peaks at the following 2θ positions: 15.0928°±0.2°, 16.8372°±0.2°, 18.5382°±0.2°, 19.194°±0.2°, 21.4798°±0.2°, and 23.6411°±0.2°.

In one embodiment, the X-ray powder diffraction pattern of the one equivalent of citrate crystal form B, measured using Cu-Kα radiation, has diffraction peaks at the following 2θ positions: 15.0928°±0.2°, 16.8372°±0.2°, 18.5382°±0.2°, 19.194°±0.2°, 21.4798°±0.2°, 23.6411°±0.2°, and 26.9181°±0.2°.

In one embodiment, the X-ray powder diffraction pattern of the one equivalent of citrate crystal form B, measured using Cu-Kα radiation, has diffraction peaks at the following 2θ positions: 7.5584°±0.2°, 9.2726°±0.2°, 10.6593°±0.2°, 11.3352°±0.2°, 12.54°±0.2°, 13.5438°±0.2°, 13.7735°±0.2°, 14.0152°±0.2°, 15.0928°±0.2°, 15.4425°±0.2°, 15.628°±0.2°, 16.8372°±0.2°, 18.5382°±0.2°, 19.194°±0.2°, 21.1607°±0.2°, 21.4798°±0.2°, 22.0822°±0.2°, 22.7152°±0.2°, 23.6411°±0.2°, 24.0169°±0.2°, 24.6133°±0.2°, 26.9181°±0.2°, 27.5774°±0.2°,27.9026°±0.2°, 30.3368°±0.2°, 32.439°±0.2°, 33.9973°±0.2°, 34.2994°±0.2°, 34.7631°±0.2°, and 35.1435°±0.2°.

In one embodiment, X-ray powder diffraction pattern analysis data of the one equivalent of citrate crystal form B are substantially as shown in the following table:

| Position [°20]±0.2° | d-spacing [Å] | Relative intensity [%] | Position [°2θ]±0.2° | d-spacing [Å] | Relative intensity [%] |
|---|---|---|---|---|---|
| 7.5584 | 11.68671 | 5.80% | 21.4798 | 4.13358 | 55.30% |
| 9.2726 | 9.52976 | 13.70% | 22.0822 | 4.02216 | 10.30% |
| 10.6593 | 8.29289 | 30.00% | 22.7152 | 3.91149 | 11.80% |
| 11.3352 | 7.79989 | 13.90% | 23.6411 | 3.76034 | 100.00% |
| 12.54 | 7.05311 | 12.70% | 24.0169 | 3.70234 | 23.00% |
| 13.5438 | 6.53253 | 4.80% | 24.6133 | 3.61397 | 14.70% |
| 13.7735 | 6.42407 | 20.20% | 26.9181 | 3.30954 | 33.50% |
| 14.0152 | 6.31383 | 11.10% | 27.5774 | 3.23189 | 15.00% |
| 15.0928 | 5.8654 | 45.40% | 27.9026 | 3.19496 | 23.90% |
| 15.4425 | 5.73333 | 14.50% | 30.3368 | 2.94392 | 10.80% |
| 15.628 | 5.66571 | 15.80% | 32.439 | 2.75778 | 13.40% |
| 16.8372 | 5.26142 | 80.80% | 33.9973 | 2.63484 | 10.20% |
| 18.5382 | 4.78231 | 38.10% | 34.2994 | 2.61233 | 1.90% |
| 19.194 | 4.62037 | 41.20% | 34.7631 | 2.57854 | 0.90% |
| 21.1607 | 4.19519 | 15.10% | 35.1435 | 2.55149 | 2.80% |

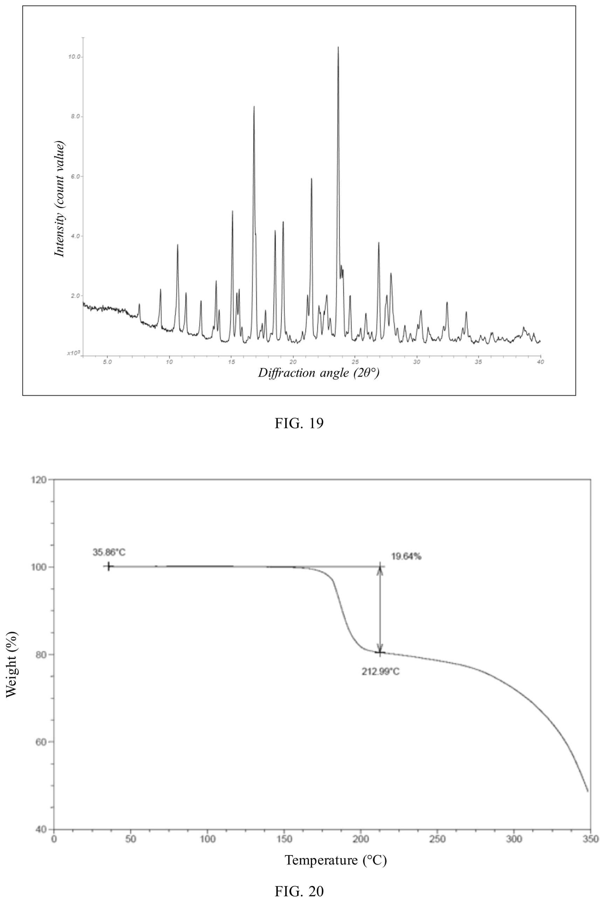

In one embodiment, the X-ray powder diffraction pattern of the one equivalent of citrate crystal form B is substantially as shown in FIG. 19.

In one embodiment, a thermogravimetric analysis (TGA) curve of the one equivalent of citrate crystal form B shows a weight loss of 19.64±0.005% during heating from 35.86±3 °C to 212.99±3 °C.

In one embodiment, the thermogravimetric analysis (TGA) curve of the one equivalent of citrate crystal form B shows a weight loss of 19.64% during heating from 35.86 °C to 212.99 °C.

In one embodiment, the thermogravimetric analysis curve of the one equivalent of citrate crystal form B is substantially as shown in FIG. 20.

In one embodiment, a differential scanning calorimetry (DSC) curve of the one equivalent of citrate crystal form B has an endothermic peak with an initial temperature of 178.23±3 °C.

In one embodiment, the differential scanning calorimetry (DSC) curve of the one equivalent of citrate crystal form B has an endothermic peak with an initial temperature of 178.23 °C.

In one embodiment, the differential scanning calorimetry (DSC) curve of the one equivalent of citrate crystal form B has an endothermic peak with a peak temperature of 180.38±3 °C.

In one embodiment, the differential scanning calorimetry (DSC) curve of the one equivalent of citrate crystal form B has an endothermic peak with a peak temperature of 180.38 °C.

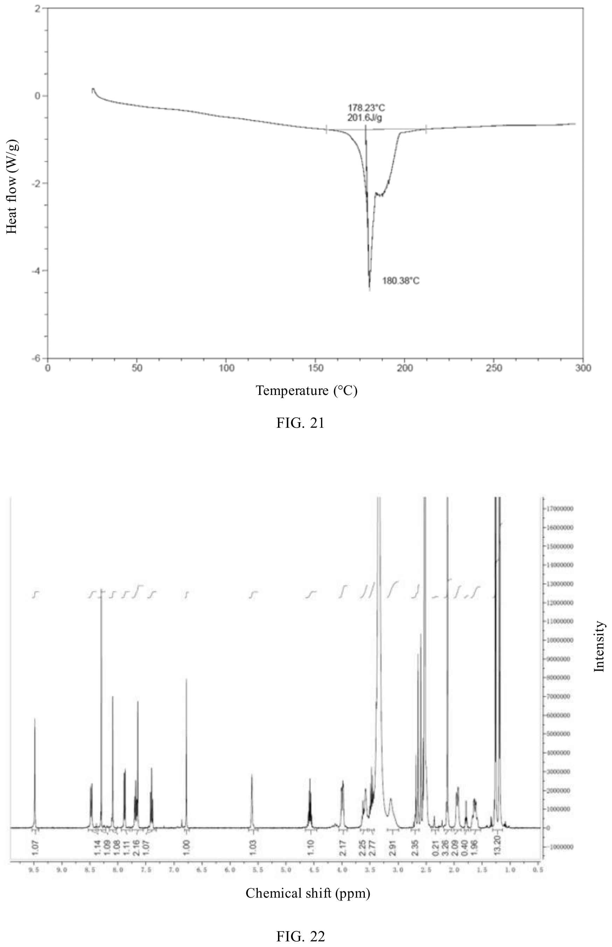

In one embodiment, the differential scanning calorimetry curve of the one equivalent of citrate crystal form B is substantially as shown in FIG. 21.

In one embodiment, ¹H-NMR results of the one equivalent of citrate crystal form B show that citric acid and a free base in a sample are in a molar ratio of 1:1, with no solvent remaining.

In one embodiment, a proton nuclear magnetic resonance spectrum of the one equivalent of citrate crystal form B is shown in FIG. 22.

In one embodiment, a dynamic vapor sorption (DVS) curve of the one equivalent of citrate crystal form B shows a hygroscopic weight gain of 0.19%±0.005% at 25 °C and 80% RH.

In one embodiment, the dynamic vapor sorption (DVS) curve of the one equivalent of citrate crystal form B shows a hygroscopic weight gain of 0.19% at 25 °C and 80% RH.

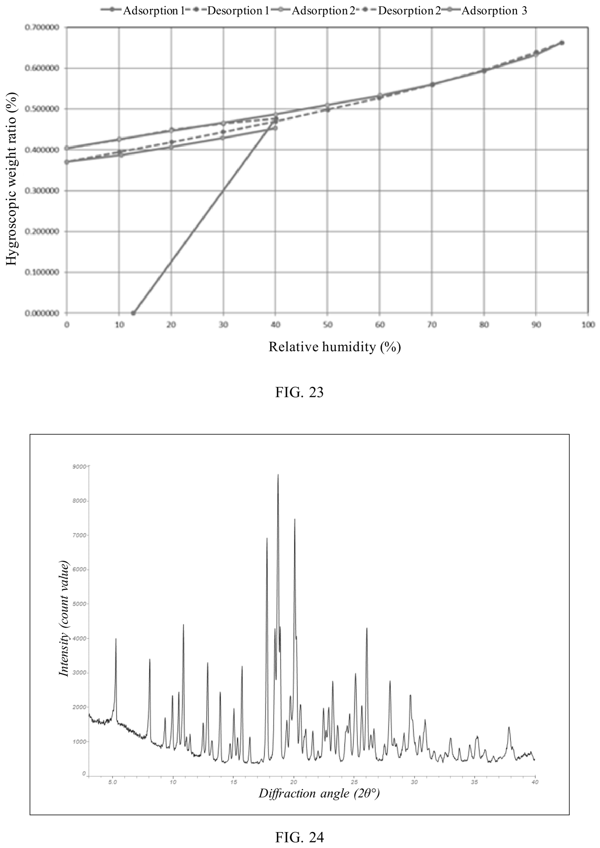

In one embodiment, the dynamic vapor sorption curve of the one equivalent of citrate crystal form B is substantially as shown in FIG. 23.

The present disclosure further provides a preparation method for the one equivalent of citrate crystal form B of the compound represented by formula I, which comprises the following steps: stirring an amorphous form of the compound represented by formula I and citric acid in a solvent at 50 °C to obtain a suspension, and further stirring the obtained suspension at room temperature to precipitate a solid, so as to obtain the one equivalent of citrate crystal form B of the compound represented by formula I, wherein the solvent is tetrahydrofuran; the solvent and the compound represented by formula I are preferably in a mass-to-volume ratio of (10-100 mg):1 mL, and further preferably 25 mg:1 mL; citric acid and the compound represented by formula I are preferably in a molar ratio of 1:1; further stirring the obtained suspension at room temperature is preferably performed at 25 °C; further stirring the obtained suspension at room temperature is preferably performed for a period of 3-5 days, and further preferably 4 days.

In one embodiment, the preparation method for the one equivalent of citrate crystal form B may further comprise, after the solid is precipitated, the steps of separation and drying to obtain the one equivalent of citrate crystal form B.

The present disclosure provides one equivalent of succinate crystal form A of the compound represented by formula I, and an X-ray powder diffraction (XRPD) pattern of the crystal form has at least three, at least four, at least five, at least six, or at least seven characteristic peaks at the following 20 angles: 4.499°±0.2°, 11.997°±0.2°, 15.099°±0.2°, 18.02°±0.2°, 20.297°±0.2°, 20.696°±0.2°, and 23.059°±0.2°;

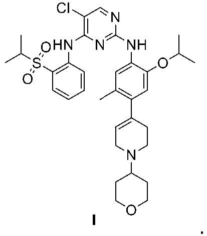

In one embodiment, the X-ray powder diffraction pattern of the one equivalent of succinate crystal form A, measured using Cu-Kα radiation, has diffraction peaks at the following 2θ positions: 4.499°+0.2°, 6.876°+0.2°, 8.773°±0.2°, 8.959°±0.2°, 10.401°±0.2°, 11.997°±0.2°, 13.296°±0.2°, 13.616°±0.2°, 14.006°±0.2°, 14.337°±0.2°, 15.099°±0.2°, 15.944°±0.2°, 16.218°±0.2°, 16.64°±0.2°, 17.316°±0.2°, 17.556°±0.2°, 18.02°+0.2°, 18.538°+0.2°, 19.117°+0.2°, 19.584°+0.2°, 20.297°+0.2°, 20.696°+0.2°, 20.859°+0.2°, 21.478°±0.2°, 22.08°±0.2°, 22.458°±0.2°, 23.059°±0.2°, 23.801°±0.2°, 24.066°±0.2°, and 24.3°±0.2°.

In one embodiment, X-ray powder diffraction pattern analysis data of the one equivalent of succinate crystal form A are substantially as shown in the following table:

| Position [°2θ]+0.2° | d-spacing [Å] | Relative intensity [%] | Position [°2θ]±0.2° | d-spacing [Å] | Relative intensity [%] |
|---|---|---|---|---|---|
| 4.499 | 19.6262 | 65.90% | 17.556 | 5.0477 | 57.50% |
| 6.876 | 12.845 | 6.20% | 18.02 | 4.9186 | 89.40% |
| 8.773 | 10.0713 | 53.50% | 18.538 | 4.7825 | 59.40% |
| 8.959 | 9.8621 | 28.20% | 19.117 | 4.6387 | 47.60% |
| 10.401 | 8.4981 | 8.40% | 19.584 | 4.5292 | 54.00% |
| 11.997 | 7.3709 | 100.00% | 20.297 | 4.3717 | 61.30% |
| 13.296 | 6.6535 | 28.60% | 20.696 | 4.2882 | 99.50% |
| 13.616 | 6.4981 | 19.70% | 20.859 | 4.2551 | 21.40% |
| 14.006 | 6.3179 | 8.00% | 21.478 | 4.134 | 11.80% |
| 14.337 | 6.1731 | 17.20% | 22.08 | 4.0225 | 20.20% |
| 15.099 | 5.8628 | 89.00% | 22.458 | 3.9556 | 18.80% |
| 15.944 | 5.5541 | 17.50% | 23.059 | 3.854 | 79.20% |
| 16.218 | 5.4609 | 24.70% | 23.801 | 3.7355 | 25.90% |
| 16.64 | 5.3235 | 18.40% | 24.066 | 3.6949 | 13.40% |
| 17.316 | 5.117 | 45.70% | 24.3 | 3.6599 | 40.90% |

In one embodiment, a thermogravimetric analysis (TGA) curve of the one equivalent of succinate crystal form A shows a weight loss of 0.2518% during heating from 38.71 °C to 150.16 °C.

In one embodiment, a differential scanning calorimetry (DSC) curve of the one equivalent of succinate crystal form A has an endothermic peak with an initial temperature of 167.37 °C.

In one embodiment, the differential scanning calorimetry (DSC) curve of the one equivalent of succinate crystal form A has an endothermic peak with a peak temperature of 168.66 °C.

In one embodiment, ¹H-NMR results of the one equivalent of succinate crystal form A show that succinic acid and a free base in a sample are in a molar ratio of 1:1, with no solvent remaining.

The present disclosure provides one equivalent of succinate crystal form B of the compound represented by formula I, and an X-ray powder diffraction (XRPD) pattern of the crystal form has at least three, at least four, at least five, at least six, or at least seven characteristic peaks at the following 2θ angles: 8.7986°±0.2°, 9.6353°±0.2°, 15.0967°+0.2°, 17.6804°+0.2°, 17.8403°+0.2°, 19.4381°±0.2°, and 20.9405°±0.2°;

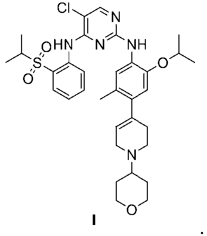

In one embodiment, the X-ray powder diffraction pattern of the one equivalent of succinate crystal form B, measured using Cu-Kα radiation, has diffraction peaks at the following 2θ positions: 4.413°±0.2°, 8.5426°±0.2°, 8.7986°±0.2°, 9.6353°±0.2°, 9.8769°±0.2°, 10.5788°±0.2°, 11.6749°±0.2°, 12.1356°±0.2°, 12.419°±0.2°, 12.731°±0.2°, 12.9957°±0.2°, 13.1995°±0.2°, 13.6583°±0.2°, 13.897°±0.2°, 14.7617°±0.2°, 15.0967°±0.2°, 16.0212°±0.2°, 16.4598°±0.2°, 17.0656°±0.2°, 17.4649°±0.2°, 17.6804°+0.2°, 17.8403°±0.2°, 19.4381°±0.2°, 20.9405°±0.2°, 22.097°±0.2°, 24.5793°±0.2°, 25.176°±0.2°, 25.6151°±0.2°, 25.8172°±0.2°, and 26.6656°+0.2°.

In one embodiment, X-ray powder diffraction pattern analysis data of the one equivalent of succinate crystal form B are substantially as shown in the following table:

| Position [°20]±0.2° | d-spacing [Å] | Relative intensity [%] | Position [°20]±0.2° | d-spacing [Å] | Relative intensity [%] |
|---|---|---|---|---|---|
| 4.413 | 20.00693 | 7.70% | 15.0967 | 5.86386 | 24.50% |
| 8.5426 | 10.34236 | 8.30% | 16.0212 | 5.52753 | 3.10% |
| 8.7986 | 10.04208 | 52.40% | 16.4598 | 5.38122 | 1.20% |
| 9.6353 | 9.17188 | 33.20% | 17.0656 | 5.19154 | 2.40% |
| 9.8769 | 8.94801 | 14.20% | 17.4649 | 5.07373 | 19.70% |
| 10.5788 | 8.35585 | 4.40% | 17.6804 | 5.01235 | 65.70% |
| 11.6749 | 7.57368 | 7.00% | 17.8403 | 4.96781 | 100.00% |
| 12.1356 | 7.28719 | 2.40% | 19.4381 | 4.5629 | 33.40% |
| 12.419 | 7.12155 | 2.40% | 20.9405 | 4.23879 | 20.80% |
| 12.731 | 6.9477 | 2.20% | 22.097 | 4.01949 | 13.70% |
| 12.9957 | 6.80677 | 3.40% | 24.5793 | 3.61889 | 20.20% |
| 13.1995 | 6.70213 | 6.60% | 25.176 | 3.53445 | 12.10% |
| 13.6583 | 6.47802 | 2.90% | 25.6151 | 3.47486 | 9.10% |
| 13.897 | 6.36727 | 6.60% | 25.8172 | 3.44811 | 3.80% |
| 14.7617 | 5.9962 | 7.80% | 26.6656 | 3.3403 | 4.00% |

In one embodiment, a thermogravimetric analysis (TGA) curve of the one equivalent of succinate crystal form B shows a weight loss of 4.942% during heating from 38.51 °C to 137.03 °C.

In one embodiment, a differential scanning calorimetry (DSC) curve of the one equivalent of succinate crystal form B has endothermic peaks with initial temperatures of 93.69 °C, 141.56 °C, 176.96 °C, and 188.31 °C, respectively.

In one embodiment, the differential scanning calorimetry (DSC) curve of the one equivalent of succinate crystal form B has endothermic peaks with peak temperatures of 114.14 °C, 151.48 °C, 179.76 °C, and 219.49 °C, respectively.

In one embodiment, ¹H-NMR results of the one equivalent of succinate crystal form B show that succinic acid and a free base in a sample are in a molar ratio of 1:1, and residual tetrahydrofuran solvent and the free base are in a molar ratio of 0.75:1 and a weight ratio of 6.7%.

The present disclosure provides one equivalent of succinate crystal form C of the compound represented by formula I, and an X-ray powder diffraction (XRPD) pattern of the crystal form has at least three, at least four, at least five, at least six, or at least seven characteristic peaks at the following 2θ angles: 8.8106°±0.2°, 9.8327°±0.2°, 10.5744°±0.2°, 17.6389°±0.2°, 17.8746°±0.2°, 19.5814°±0.2°, and 21.1774°±0.2°;

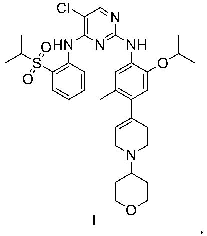

In one embodiment, the X-ray powder diffraction pattern of the one equivalent of succinate crystal form C, measured using Cu-Kα radiation, has diffraction peaks at the following 2θ positions: 4.4254°±0.2°, 8.1567°±0.2°, 8.8106°±0.2°, 9.8327°±0.2°, 10.5744°±0.2°, 10.9741°±0.2°, 12.0169°±0.2°, 12.7766°±0.2°, 13.2101°±0.2°, 14.5967°±0.2°, 14.9064°±0.2°, 15.475°±0.2°, 16.3197°±0.2°, 17.6389°±0.2°, 17.8746°±0.2°, 18.541°±0.2°, 19.0788°±0.2°, 19.5814°±0.2°, 19.9794°±0.2°, 20.2206°±0.2°, 20.6426°±0.2°, 20.7938°±0.2°, 21.1774°±0.2°, 21.3595°±0.2°, 22.0773°±0.2°, 22.8039°±0.2°, 22.9977°±0.2°, 23.7612°±0.2°, 23.9994°±0.2°, and 24.1298°±0.2°.

In one embodiment, X-ray powder diffraction pattern analysis data of the one equivalent of succinate crystal form C are substantially as shown in the following table:

| Position [°2θ]+0.2° | d-spacing [Å] | Relative intensity [%] | Position [°2θ]+0.2° | d-spacing [Å] | Relative intensity [%] |
|---|---|---|---|---|---|
| 4.4254 | 19.95095 | 7.70% | 18.541 | 4.78159 | 2.60% |
| 8.1567 | 10.83085 | 3.20% | 19.0788 | 4.64801 | 4.60% |
| 8.8106 | 10.02841 | 33.20% | 19.5814 | 4.52983 | 16.20% |
| 9.8327 | 8.98815 | 17.70% | 19.9794 | 4.44047 | 4.70% |
| 10.5744 | 8.35934 | 16.20% | 20.2206 | 4.38806 | 0.70% |
| 10.9741 | 8.05572 | 3.00% | 20.6426 | 4.2993 | 4.90% |
| 12.0169 | 7.35894 | 10.00% | 20.7938 | 4.26837 | 2.90% |
| 12.7766 | 6.92301 | 10.10% | 21.1774 | 4.19192 | 17.90% |
| 13.2101 | 6.69677 | 1.60% | 21.3595 | 4.15658 | 3.90% |
| 14.5967 | 6.06359 | 13.30% | 22.0773 | 4.02304 | 7.40% |
| 14.9064 | 5.93832 | 1.90% | 22.8039 | 3.89647 | 1.60% |
| 15.475 | 5.72137 | 4.30% | 22.9977 | 3.86407 | 2.50% |
| 16.3197 | 5.42709 | 4.90% | 23.7612 | 3.74161 | 1.80% |
| 17.6389 | 5.02407 | 100.00% | 23.9994 | 3.70501 | 4.30% |
| 17.8746 | 4.95834 | 23.00% | 24.1298 | 3.68528 | 4.60% |

In one embodiment, a thermogravimetric analysis (TGA) curve of the one equivalent of succinate crystal form C shows a weight loss of 5.472% during heating from 38.13 °C to 125.09 °C.

In one embodiment, a differential scanning calorimetry (DSC) curve of the one equivalent of succinate crystal form C has endothermic peaks with initial temperatures of 98.60 °C, 147.67 °C, 162.11 °C, and 181.14 °C, respectively.

In one embodiment, the differential scanning calorimetry (DSC) curve of the one equivalent of succinate crystal form C has endothermic peaks with peak temperatures of 120.14 °C, 150.58 °C, 164.99 °C, and 182.19 °C, respectively.

In one embodiment, ¹H-NMR results of the one equivalent of succinate crystal form C show that succinic acid and a free base in a sample are in a molar ratio of 1:1, and residual 1,4-dioxane solvent and the free base are in a molar ratio of 0.90:1 and a weight ratio of 9.5%.

The present disclosure provides one equivalent of succinate crystal form D of the compound represented by formula I, and an X-ray powder diffraction (XRPD) pattern of the crystal form has at least three, at least four, at least five, at least six, or at least seven characteristic peaks at the following 2θ angles: 15.715°±0.2°, 17.778°±0.2°, 18.44°±0.2°, 18.698°±0.2°, 18.86°±0.2°, 20.079°±0.2°, and 20.218°±0.2°;

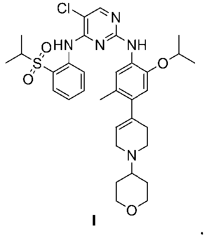

In one embodiment, the X-ray powder diffraction pattern of the one equivalent of succinate crystal form D, measured using Cu-Kα radiation, has diffraction peaks at the following 2θ positions: 17.778°±0.2°, 18.698°±0.2°, and 20.079°±0.2°.

In one embodiment, the X-ray powder diffraction pattern of the one equivalent of succinate crystal form D, measured using Cu-Kα radiation, has diffraction peaks at the following 2θ positions: 17.778°±0.2°, 18.698°±0.2°, 18.86°±0.2°, and 20.079°±0.2°.

In one embodiment, the X-ray powder diffraction pattern of the one equivalent of succinate crystal form D, measured using Cu-Kα radiation, has diffraction peaks at the following 2θ positions: 17.778°±0.2°, 18.44°±0.2°, 18.698°±0.2°, 18.86°±0.2°, and 20.079°±0.2°.

In one embodiment, the X-ray powder diffraction pattern of the one equivalent of succinate crystal form D, measured using Cu-Kα radiation, has diffraction peaks at the following 2θ positions: 17.778°±0.2°, 18.44°±0.2°, 18.698°±0.2°, 18.86°±0.2°, 20.079°±0.2°, and 20.218°±0.2°.

In one embodiment, the X-ray powder diffraction pattern of the one equivalent of succinate crystal form D, measured using Cu-Kα radiation, has diffraction peaks at the following 2θ positions: 15.715°±0.2°, 17.778°±0.2°, 18.44°±0.2°, 18.698°±0.2°, 18.86°±0.2°, 20.079°±0.2°, and 20.218°±0.2°.

In one embodiment, the X-ray powder diffraction pattern of the one equivalent of succinate crystal form D, measured using Cu-Kα radiation, has diffraction peaks at the following 2θ positions: 5.255°±0.2°, 8.059°±0.2°, 9.338°±0.2°, 9.941°±0.2°, 10.472°±0.2°, 11.401°±0.2°, 12.483°±0.2°, 12.86°±0.2°, 13.203°±0.2°, 13.901°±0.2°, 14.72°±0.2°, 15.038°±0.2°, 15.715°±0.2°, 16.36°±0.2°, 17.778°±0.2°, 18.44°±0.2°, 18.698°±0.2°, 18.86°±0.2°, 19.421°±0.2°, 19.719°±0.2°, 20.079°+0.2°, 20.218°±0.2°, 20.542°±0.2°, 20.862°±0.2°, 20.997°±0.2°, 21.579°±0.2°, 22.461°±0.2°, 22.679°±0.2°, 22.899°±0.2°, and 23.222°±0.2°. In one embodiment, X-ray powder diffraction pattern analysis data of the one equivalent of succinate crystal form D are substantially as shown in the following table:

| Position [°20]±0.2° | d-spacing [Å] | Relative intensity [%] | Position [°2θ]±0.2° | d-spacing [Å] | Relative intensity [%] |
|---|---|---|---|---|---|
| 5.255 | 16.8041 | 38.10% | 18.44 | 4.8076 | 58.90% |
| 8.059 | 10.962 | 37.20% | 18.698 | 4.7418 | 79.40% |
| 9.338 | 9.4636 | 13.00% | 18.86 | 4.7013 | 59.20% |
| 9.941 | 8.8906 | 23.60% | 19.421 | 4.5669 | 17.20% |
| 10.472 | 8.4405 | 23.50% | 19.719 | 4.4985 | 21.40% |
| 11.401 | 7.7552 | 7.80% | 20.079 | 4.4186 | 99.10% |
| 12.483 | 7.0851 | 13.20% | 20.218 | 4.3886 | 46.50% |
| 12.86 | 6.8785 | 40.80% | 20.542 | 4.32 | 18.10% |
| 13.203 | 6.7002 | 7.30% | 20.862 | 4.2546 | 10.60% |
| 13.901 | 6.3655 | 31.20% | 20.997 | 4.2276 | 13.80% |
| 14.72 | 6.0132 | 5.90% | 21.579 | 4.1148 | 12.80% |
| 15.038 | 5.8865 | 20.80% | 22.461 | 3.9552 | 23.00% |
| 15.715 | 5.6345 | 41.90% | 22.679 | 3.9176 | 13.20% |
| 16.36 | 5.414 | 11.40% | 22.899 | 3.8806 | 23.40% |
| 17.778 | 4.985 | 100.00% | 23.222 | 3.8272 | 30.80% |

In one embodiment, the X-ray powder diffraction pattern of the one equivalent of succinate crystal form D is substantially as shown in FIG. 24.

In one embodiment, a thermogravimetric analysis (TGA) curve of the one equivalent of succinate crystal form D shows a weight loss of 0. 1218±0.005% during heating from 30.10±3 °C to 150.15±3 °C.

In one embodiment, the thermogravimetric analysis (TGA) curve of the one equivalent of succinate crystal form D shows a weight loss of 0.1218% during heating from 30.10 °C to 150.15 °C.

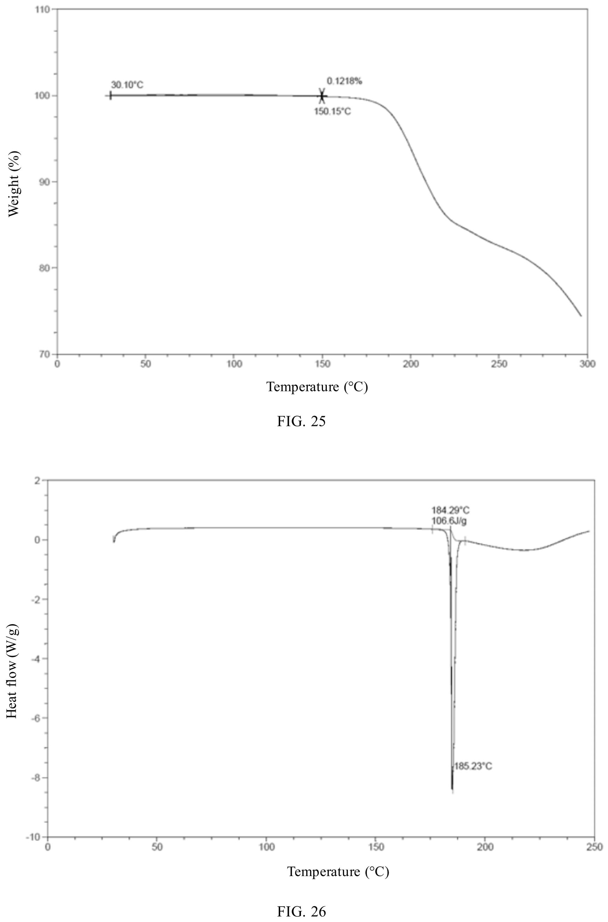

In one embodiment, the thermogravimetric analysis curve of the one equivalent of succinate crystal form D is substantially as shown in FIG. 25.

In one embodiment, a differential scanning calorimetry (DSC) curve of the one equivalent of succinate crystal form D has an endothermic peak with an initial temperature of 184.29±3 °C.

In one embodiment, the differential scanning calorimetry (DSC) curve of the one equivalent of succinate crystal form D has an endothermic peak with an initial temperature of 184.29 °C.

In one embodiment, the differential scanning calorimetry (DSC) curve of the one equivalent of succinate crystal form D has an endothermic peak with a peak temperature of 185.23±3 °C.

In one embodiment, the differential scanning calorimetry (DSC) curve of the one equivalent of succinate crystal form D has an endothermic peak with a peak temperature of 185.23 °C.

In one embodiment, the differential scanning calorimetry curve of the one equivalent of succinate crystal form D is substantially as shown in FIG. 26.

In one embodiment, ¹H-NMR results of the one equivalent of succinate crystal form D show that succinic acid and a free base in a sample are in a molar ratio of 1:1, with no solvent remaining.

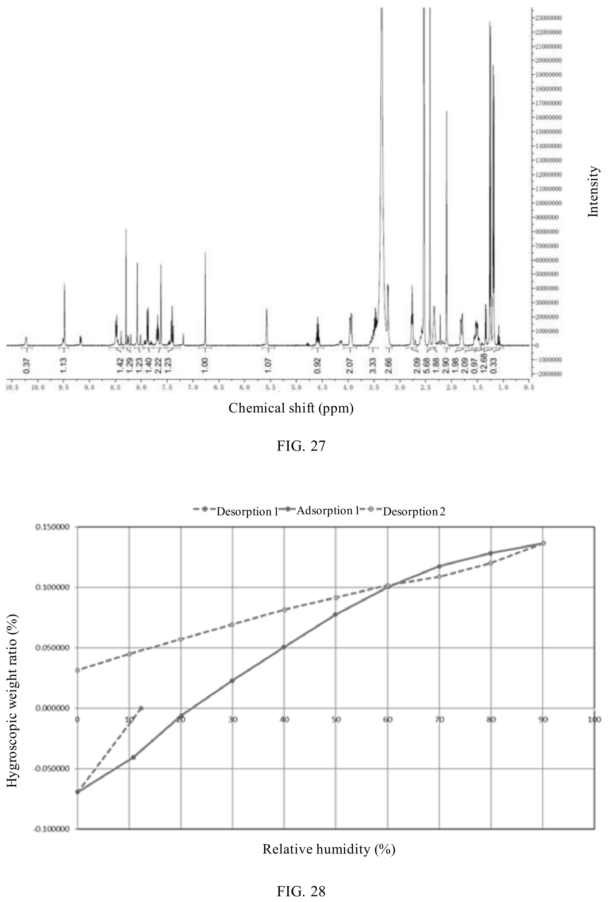

In one embodiment, a proton nuclear magnetic resonance spectrum of the one equivalent of succinate crystal form D is shown in FIG. 27.

In one embodiment, a dynamic vapor sorption (DVS) curve of the one equivalent of succinate crystal form D shows a hygroscopic weight gain of 0.19±0.005% at 25 °C and 80% RH.

In one embodiment, the dynamic vapor sorption (DVS) curve of the one equivalent of succinate crystal form D shows a hygroscopic weight gain of 0.19% at 25 °C and 80% RH.

In one embodiment, the dynamic vapor sorption curve of the one equivalent of succinate crystal form D is substantially as shown in FIG. 28.

The present disclosure further provides a preparation method for the one equivalent of succinate crystal form D of the compound represented by formula I, which comprises the following steps: stirring an amorphous form of the compound represented by formula I and succinic acid in a solvent at 50 °C to obtain a suspension, and further stirring the obtained suspension at room temperature to precipitate a solid, so as to obtain the one equivalent of succinate crystal form D of the compound represented by formula I, wherein the solvent is ethanol; the solvent and the compound represented by formula I are preferably in a mass-to-volume ratio of (10-100 mg):1 mL, and further preferably 25 mg:1 mL; succinic acid and the compound represented by formula I are preferably in a molar ratio of 1:1; further stirring the obtained suspension at room temperature is preferably performed at 25 °C; further stirring the obtained suspension at room temperature is preferably performed for a period of 3-5 days, and further preferably 4 days.

In one embodiment, the preparation method for the one equivalent of succinate crystal form D may further comprise, after the solid is precipitated, the steps of separation and drying to obtain the one equivalent of succinate crystal form D.

The present disclosure provides one equivalent of phosphate crystal form E of the compound represented by formula I, and an X-ray powder diffraction (XRPD) pattern of the crystal form has at least three, at least four, at least five, at least six, or at least seven characteristic peaks at the following 20 angles: 6.558°±0.2°, 8.841°±0.2°, 13.524°±0.2°, 18.241°±0.2°, 18.86°±0.2°, 20.246°±0.2°, and 28.756°±0.2°;

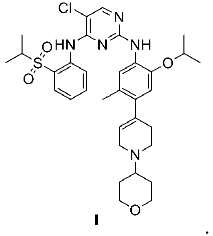

In one embodiment, the X-ray powder diffraction pattern of the one equivalent of phosphate crystal form E, measured using Cu-Kα radiation, has diffraction peaks at the following 2θ positions: 4.241°+0.2°, 6.558°+0.2°, 8.563°±0.2°, 8.841°±0.2°, 9.236°±0.2°, 9.663°±0.2°, 11.539°±0.2°, 11.8°±0.2°, 12.262°±0.2°, 12.558°±0.2°, 13.06°±0.2°, 13.524°±0.2°, 14.943°±0.2°, 17.18°±0.2°, 17.56°±0.2°, 18.084°±0.2°, 18.241°±0.2°, 18.86°+0.2°, 19.219°+0.2°, 19.484°+0.2°, 20.246°+0.2°, 23.124°+0.2°, 24.926°±0.2°, 25.221°±0.2°, 26.143°±0.2°, 28.756°±0.2°, 29.6°±0.2°, 30.543°±0.2°, 31.24°±0.2°, and 31.644°±0.2°.

In one embodiment, X-ray powder diffraction pattern analysis data of the one equivalent of phosphate crystal form E are substantially as shown in the following table:

| Position [°2θ]+0.2° | d-spacing [Å] | Relative intensity [%] | Position [°2θ]+0.2° | d-spacing [Å] | Relative intensity [%] |
|---|---|---|---|---|---|
| 4.241 | 20.8182 | 11.70% | 18.084 | 4.9013 | 38.60% |
| 6.558 | 13.4677 | 76.40% | 18.241 | 4.8596 | 49.60% |
| 8.563 | 10.3178 | 18.10% | 18.86 | 4.7014 | 85.30% |
| 8.841 | 9.9942 | 67.20% | 19.219 | 4.6144 | 15.30% |
| 9.236 | 9.5675 | 8.40% | 19.484 | 4.5522 | 14.00% |
| 9.663 | 9.1457 | 21.30% | 20.246 | 4.3825 | 100.00% |
| 11.539 | 7.6624 | 18.30% | 23.124 | 3.8433 | 24.00% |
| 11.8 | 7.4938 | 9.70% | 24.926 | 3.5693 | 17.30% |
| 12.262 | 7.2125 | 34.30% | 25.221 | 3.5282 | 32.60% |
| 12.558 | 7.0429 | 15.90% | 26.143 | 3.4058 | 14.80% |
| 13.06 | 6.7733 | 22.90% | 28.756 | 3.1021 | 79.60% |
| 13.524 | 6.5422 | 42.20% | 29.6 | 3.0155 | 10.20% |
| 14.943 | 5.9237 | 11.70% | 30.543 | 2.9246 | 13.40% |
| 17.18 | 5.1571 | 17.60% | 31.24 | 2.8608 | 9.50% |
| 17.56 | 5.0465 | 10.90% | 31.644 | 2.8252 | 7.60% |

In one embodiment, a thermogravimetric analysis (TGA) curve of the one equivalent of phosphate crystal form E shows a weight loss of 0.6794% during heating from 30.15 °C to 125.70 °C.

In one embodiment, a differential scanning calorimetry (DSC) curve of the one equivalent of phosphate crystal form E has endothermic peaks with initial temperatures of 160.90 °C and 205.05 °C, respectively.

In one embodiment, the differential scanning calorimetry (DSC) curve of the one equivalent of phosphate crystal form E has endothermic peaks with peak temperatures of 167.60 °C and 208.15 °C, respectively.

In one embodiment, ¹H-NMR results of the one equivalent of phosphate crystal form E show that residual ethanol solvent and a free base are in a molar ratio of 0.14:1 and a weight ratio of 0.86%.

The present disclosure provides one equivalent of hydrochloride crystal form A of the compound represented by formula I, and an X-ray powder diffraction (XRPD) pattern of the crystal form, measured using Cu-Kα radiation, has diffraction peaks at the following 2θ positions: 5.778°+0.2°, 8.463°+0.2°, 9.861°+0.2°, 10.416°+0.2°, 10.759°+0.2°, 12.215°+0.2°, 13.722°+0.2°, 14.12°+0.2°, 15.182°+0.2°, 15.539°+0.2°, 15.977°±0.2°, 16.326°±0.2°, 16.581°±0.2°, 16.92°±0.2°, 17.297°±0.2°, 17.621°±0.2°, 17.859°±0.2°, 18.562°±0.2°, 19.157°±0.2°, 19.742°±0.2°, 20.382°±0.2°, 20.803°±0.2°, 21.355°±0.2°, 21.618°±0.2°, 22.161°±0.2°, 22.338°+0.2°, 22.915°+0.2°, 23.14°+0.2°, 23.361°±0.2°, and 23.638°±0.2°.

In one embodiment, a thermogravimetric analysis (TGA) curve of the one equivalent of hydrochloride crystal form A shows a weight loss of 0.2462% during heating from 20.93 °C to 200.71 °C.

In one embodiment, a differential scanning calorimetry (DSC) curve of the one equivalent of hydrochloride crystal form A has endothermic peaks with initial temperatures of 267.00 °C and 272.57 °C.

In one embodiment, a dynamic vapor sorption (DVS) curve of the one equivalent of hydrochloride crystal form A shows a hygroscopic weight gain of 0.15% at 25 °C and 80% RH.

The present disclosure further provides a pharmaceutical composition comprising the aforementioned one equivalent of mesylate crystal form A of the compound represented by formula I, crystal form D of the compound represented by formula I, one equivalent of maleate crystal form A of the compound represented by formula I, one equivalent of tartrate crystal form C of the compound represented by formula I, one equivalent of citrate crystal form B of the compound represented by formula I, or one equivalent of succinate crystal form D of the compound represented by formula I, and one or more pharmaceutically acceptable excipients.

The present disclosure further provides use of a substance X in the preparation of an ALK inhibitor, wherein the substance X is the aforementioned one equivalent of mesylate crystal form A of the compound represented by formula I, crystal form D of the compound represented by formula I, one equivalent of maleate crystal form A of the compound represented by formula I, one equivalent of tartrate crystal form C of the compound represented by formula I, one equivalent of citrate crystal form B of the compound represented by formula I, or one equivalent of succinate crystal form D of the compound represented by formula I, or the aforementioned pharmaceutical composition.

The present disclosure further provides use of a substance X in the treatment or prevention of cancer, wherein the substance X is the aforementioned one equivalent of mesylate crystal form A of the compound represented by formula I, crystal form D of the compound represented by formula I, one equivalent of maleate crystal form A of the compound represented by formula I, one equivalent of tartrate crystal form C of the compound represented by formula I, one equivalent of citrate crystal form B of the compound represented by formula I, or one equivalent of succinate crystal form D of the compound represented by formula I, or the aforementioned pharmaceutical composition; preferably, the cancer is selected from the group consisting of: anaplastic large cell lymphoma, non-small cell lung cancer, diffuse large B-cell lymphoma, inflammatory myofibroblastoma, neuroblastoma, anaplastic thyroid cancer, rhabdomyosarcoma, breast cancer, colorectal cancer, esophagus squamous cell carcinoma, and renal cell carcinoma.

The above preferred conditions may be combined arbitrarily to obtain preferred embodiments of the present disclosure without departing from the general knowledge in the art.

The reagents and starting materials used in the present disclosure are commercially available.

The positive and progressive effects of the present disclosure are as follows: the present disclosure provides a crystal form of an ALK inhibitor or a salt or solvate thereof. The crystal form has one or more of the following advantages: high crystallinity, good stability, slight hygroscopicity, high solubility, and high bioavailability.

### BRIEF DESCRIPTION OF THE DRAWINGS

FIG. 1 shows an XRPD pattern of a crystal form D of the compound represented by formula I.
FIG. 2 shows a TGA profile of the crystal form D of the compound represented by formula I.
FIG. 3 shows a DSC profile of the crystal form D of the compound represented by formula I.
FIG. 4 shows a DVS profile of the crystal form D of the compound represented by formula I.
FIG. 5 shows an XRPD pattern of one equivalent of mesylate crystal form A of the compound represented by formula I.
FIG. 6 shows a TGA profile of the one equivalent of mesylate crystal form A of the compound represented by formula I.
FIG. 7 shows a DSC profile of the one equivalent of mesylate crystal form A of the compound represented by formula I.
FIG. 8 shows a ¹H-NMR spectrum of the one equivalent of mesylate crystal form A of the compound represented by formula I.
FIG. 9 shows a DVS profile of the one equivalent of mesylate crystal form A of the compound represented by formula I.
FIG. 10 shows an XRPD pattern of one equivalent of maleate crystal form A of the compound represented by formula I.
FIG. 11 shows a TGA profile of the one equivalent of maleate crystal form A of the compound represented by formula I.
FIG. 12 shows a DSC profile of the one equivalent of maleate crystal form A of the compound represented by formula I.
FIG. 13 shows a ¹H-NMR spectrum of the one equivalent of maleate crystal form A of the compound represented by formula I.
FIG. 14 shows a DVS profile of the one equivalent of maleate crystal form A of the compound represented by formula I.
FIG. 15 shows an XRPD pattern of one equivalent of tartrate crystal form C of the compound represented by formula I.
FIG. 16 shows a TGA profile of the one equivalent of tartrate crystal form C of the compound represented by formula I.
FIG. 17 shows a DSC profile of the one equivalent of tartrate crystal form C of the compound represented by formula I.
FIG. 18 shows a ¹H-NMR spectrum of the one equivalent of tartrate crystal form C of the compound represented by formula I.
FIG. 19 shows an XRPD pattern of one equivalent of citrate crystal form B of the compound represented by formula I.
FIG. 20 shows a TGA profile of the one equivalent of citrate crystal form B of the compound represented by formula I.
FIG. 21 shows a DSC profile of the one equivalent of citrate crystal form B of the compound represented by formula I.
FIG. 22 shows a ¹H-NMR spectrum of the one equivalent of citrate crystal form B of the compound represented by formula I.
FIG. 23 shows a DVS profile of the one equivalent of citrate crystal form B of the compound represented by formula I.
FIG. 24 shows an XRPD pattern of one equivalent of succinate crystal form D of the compound represented by formula I.
FIG. 25 shows a TGA profile of the one equivalent of succinate crystal form D of the compound represented by formula I.
FIG. 26 shows a DSC profile of the one equivalent of succinate crystal form D of the compound represented by formula I.
FIG. 27 shows a ¹H-NMR spectrum of the one equivalent of succinate crystal form D of the compound represented by formula I.
FIG. 28 shows a DVS profile of the one equivalent of succinate crystal form D of the compound represented by formula I.

### DETAILED DESCRIPTION

In the following examples, the experimental procedures are performed according to conventional conditions or conventional test conditions, and the compounds used in the examples are commercially available or prepared in-house. The amorphous form of the compound represented by formula I can be prepared according to the method described in WO2018044767A1.

All solvents used in the present disclosure are commercially available and can be used without further purification.

Unless otherwise stated, in the present disclosure, each example is performed at room temperature, which is generally 20-30 °C, preferably 25 °C.

Unless otherwise stated, the acid salt crystal forms in the examples are all one equivalent of acid salt crystal forms.

The full names for the English abbreviations of the solvents selected in the present disclosure are as follows:

| In English | Full name | In English | Full name |
|---|---|---|---|
| MeOH | Methanol | EtOAc | Ethyl acetate |
| EtOH | Ethanol | DCM | Dichloromethane |
| IPA | Isopropanol | MTBE | Methyl *tert*-butyl ether |
| ACN | Acetonitrile | DMSO | Dimethyl sulfoxide |
| 2-Me THF | 2-methyl-tetrahydrofuran | DMF | *N*,*N*-Dimethylformamide |
| THF | Tetrahvdrofuran | NMP | *N-*Methylpyrrolidone |
| MIBK | Methyl isobutyl ketone | FA | Methyl acetate |
| MEK | Butanone | MA | Methvl acrylate |
| iPrOAc/ IPAc | Isopropyl acetate | | |

In the present disclosure, the XRPD test parameters are set as follows:

| **Parameter** | **Instrument 1** |
|---|---|
| Model | BRUKER D8 ADVANCE |
| X-ray | Cu, kα, (λ= 1.54056 A) |
| X-ray tube settings | Voltage of X-ray tube: 40 kV, current of X-ray tube: 40 mA |
| Step length (°2θ) | 0.02° |
| Scanning speed (s/step) | 0.2 s/step |
| Scanning range (°2θ) | 3-40° |

In the present disclosure, the DVS test parameters are set as follows:

| **Parameter** | | **DVS** |
|---|---|---|
| Operating temperature (°C) | | 25 °C |
| Equilibration standard (dm/dt in 5 min) | | dm/dt=0.01%/min |
| Maximum equilibration time (min) | | 120 min |
| Hygroscopic range | | 0-90% |
| Data acquisition interval (min) | | 2 min |
| Hygroscopic gradient | 0-90% | 10% |
| | 90-95% | // |

TGA and DSC profiles were acquired on a TA Q5500 thermogravimetric analyzer and a TA Q2500 differential scanning calorimeter, respectively. The parameters are set as follows:

| **Parameter** | **TGA** | **DSC** |
|---|---|---|
| Method | Linear heating | Linear heating |
| Sample tray | Platinum tray, open | Aluminum tray, closed |
| Temperature range | Room temperature-setting endpoint temperature | Room temperature-setting endpoint temperature |
| Heating rate (°C/min) | 10 | 10 |
| Protective gas | Nitrogen | Nitrogen |

In the present disclosure, the mDSC test parameters are set as follows: amplitude: 1.0 °C; modulation period: 120 s; heating rate: 2 °C/min; and temperature range: 30 °C-setting endpoint temperature.

In the present disclosure, the ¹H-NMR test was performed using Qone-WNMR-I-AS400. The parameters are set as follows: hydrogen spectrum; solvent: dimethyl sulfoxide-d6; number of scans: 16; temperature: room temperature; and magnetic field strength: 400 MHz.

In the present disclosure, the HPLC (high-performance liquid chromatography) test parameters are set as follows:

| | | | |
|---|---|---|---|
| Chromatographic column | Waters Sunfire C18(4.6×150 mm, 3.5 µm) | | |
| Column temperature | 30 °C | | |
| Flow rate | 1.0 mL/min | | |
| Injection volume | 5 µL | | |
| Detector | UV | | |
| Detection wavelength | 277 nm | | |
| Run time | 35 min | | |
| Mobile phase A | 0.05% trifluoroacetic acid-water (V/V) | | |
| Mobile phase B | 0.05% trifluoroacetic acid-acetonitrile (V/V) | | |

| | Time | %A | %B |
|---|---|---|---|
| Elution gradient | 0 | 88 | 12 |
| | 18.0 | 52 | 48 |
| | 25.0 | 10 | 90 |
| | 28.0 | 10 | 90 |
| | 28.1 | 88 | 12 |
| | 35.0 | 88 | 12 |

### Example 1

Preparation of crystal form A of compound represented by formula I: 50 mg of an amorphous form of the compound represented by formula I was added to a 2 mL glass flask; 0.4 mL of methanol was added; the suspension was slurried at 25 °C for 3 days and centrifuged to remove the supernatant; and the resulting precipitate was dried under vacuum (25 °C, -0.1 MPa).

### Example 2

Preparation of crystal form B of compound represented by formula I: 50 mg of an amorphous form of the compound represented by formula I was added to a 2 mL glass flask; 0.4 mL of ethanol was added; the suspension was slurried at 25 °C for 3 days and centrifuged to remove the supernatant; and the resulting precipitate was dried under vacuum (25 °C, -0.1 MPa).

### Example 3

Preparation of crystal form C of compound represented by formula I: 50 mg of an amorphous form of the compound represented by formula I was added to a 2 mL glass flask; 0.4 mL of isopropanol was added; the suspension was slurried at 25 °C for 3 days and centrifuged to remove the supernatant; and the resulting precipitate was dried under vacuum (25 °C, -0.1 MPa).

### Example 4

Preparation of crystal form D of compound represented by formula I: 50 mg of an amorphous form of the compound represented by formula I was added to a 2 mL glass flask; 0.6 mL of acetonitrile was added; the suspension was slurried at 25 °C for 3 days and centrifuged to remove the supernatant; and the resulting precipitate was dried under vacuum (25 °C, -0.1 MPa).

Characterization of crystal form D of compound represented by formula I:
1) the XRPD characterization results are shown in FIG. 1;
2) the TGA characterization results are shown in FIG. 2;
3) the DSC characterization results are shown in FIG. 3; and
4) the DVS characterization results are shown in FIG. 4.

### Example 5

Preparation of crystal form E of compound represented by formula I: 50 mg of an amorphous form of the compound represented by formula I was added to a 2 mL glass flask; 0.4 mL of acetone was added; the suspension was slurried at 25 °C for 3 days and centrifuged to remove the supernatant; and the resulting precipitate was dried under vacuum (25 °C, -0.1 MPa).

### Example 6

Preparation of crystal form F of compound represented by formula I: 50 mg of an amorphous form of the compound represented by formula I was added to a 2 mL glass flask; 0.1 mL of tetrahydrofuran was added; the suspension was slurried at 25 °C for 3 days and centrifuged to remove the supernatant; and the resulting precipitate was dried under vacuum (25 °C, -0.1 MPa).

### Example 7

Preparation method for crystal form G of compound represented by formula I: 50 mg of an amorphous form of the compound represented by formula I was added to a 2 mL glass flask; 0.2 mL of 1,4-dioxane was added; the suspension was slurried at 25 °C for 3 days and centrifuged to remove the supernatant; and the resulting precipitate was dried under vacuum (25 °C, -0.1 MPa).

### Example 8

Preparation method for crystal form H of compound represented by formula I: 50 mg of an amorphous form of the compound represented by formula I was added to a 2 mL glass flask; 0.4 mL of pure water was added; the suspension was slurried at 25 °C for 3 days and centrifuged to remove the supernatant; and the resulting precipitate was dried under vacuum (25 °C, -0.1 MPa).

### Example 9

Preparation method for crystal form I of compound represented by formula I: 50 mg of an amorphous form of the compound represented by formula I was added to a 2 mL glass flask; 0.4 mL of methanol was added; the suspension was slurried at 50 °C for 3 days and centrifuged to remove the supernatant; and the resulting precipitate was dried under vacuum (25 °C, -0.1 MPa).

### Example 10

Preparation method for crystal form J of compound represented by formula I: 50 mg of an amorphous form of the compound represented by formula I was added to a 2 mL glass flask; 0.4 mL of water was added; the suspension was slurried at 50 °C for 3 days and centrifuged to remove the supernatant; and the resulting precipitate was dried under vacuum (25 °C, -0.1 MPa).

### Example 11

Preparation method for crystal form K of compound represented by formula I: 20 mg of an amorphous form of the compound represented by formula I was added to a 2 mL glass flask; 1.2 mL of methanol/dichloromethane (5:1, v/v) was added; the mixture was filtered through a 0.22 µm nylon filter; and the supernatant was placed at room temperature and volatilized naturally.

### Example 12

Preparation method for crystal form L of compound represented by formula I: 20 mg of an amorphous form of the compound represented by formula I was added to a 2 mL glass flask; 1.2 mL of ethanol/dichloromethane (5:1, v/v) was added; the mixture was filtered through a 0.22 µm nylon filter; and the supernatant was placed at room temperature and volatilized naturally.

### Example 13

Preparation method for crystal form M of compound represented by formula I: 20 mg of an amorphous form of the compound represented by formula I was added to a 2 mL glass flask; 1.2 mL of isopropanol/dichloromethane (5:1, v/v) was added; the mixture was filtered through a 0.22 µm nylon filter; and the supernatant was placed at room temperature and volatilized naturally.

### Example 14

Preparation method for crystal form N of compound represented by formula I: 20 mg of an amorphous form of the compound represented by formula I was added to a 2 mL glass flask; 0.5 mL of 1,4-dioxane was added; the mixture was filtered through a 0.22 µm nylon filter; and the supernatant was placed at room temperature and volatilized naturally.

### Example 15

Preparation method for crystal form O of compound represented by formula I: 50 mg of an amorphous form of the compound represented by formula I was added to a 2 mL glass flask; 0.4 mL of methanol/dichloromethane (3:1, v/v) was added; the mixture was stirred at 50 °C for 1 h and filtered through a 0.22 µm nylon filter to obtain a clear solution; the solution was placed in a refrigerator at 4 °C until a solid was precipitated; the supernatant was removed by centrifugation; and the resulting precipitate was dried under vacuum (25 °C, -0.1 MPa).

### Example 16

Preparation method for crystal form P of compound represented by formula I: 50 mg of an amorphous form of the compound represented by formula I was added to a 2 mL glass flask; 0.3 mL of tetrahydrofuran was added; the mixture was stirred at 25 °C until the sample was dissolved and filtered through a 0.22 µm nylon filter to obtain a clear solution; 1.2 mL of methanol was slowly added dropwise; the mixture was stirred for 5 days and centrifuged to remove the supernatant; and the resulting precipitate was dried under vacuum (25 °C, -0.1 MPa).

### Example 17

Preparation method for crystal form Q of compound represented by formula I: 50 mg of an amorphous form of the compound represented by formula I was added to a 2 mL glass flask; 0.3 mL of tetrahydrofuran was added; the mixture was stirred at 25 °C until the sample was dissolved and filtered through a 0.22 µm nylon filter to obtain a clear solution; 1.2 mL of n-heptane was slowly added dropwise; the mixture was stirred for 5 days and centrifuged to remove the supernatant; and the resulting precipitate was dried under vacuum (25 °C, -0.1 MPa).

### Example 18

Preparation method for crystal form R of compound represented by formula I: 50 mg of an amorphous form of the compound represented by formula I was added to a 2 mL glass flask; 0.3 mL of tetrahydrofuran was added; the mixture was stirred at 25 °C until the sample was dissolved and filtered through a 0.22 µm nylon filter to obtain a clear solution; 1.2 mL of pure water was slowly added dropwise; the mixture was stirred for 5 days and centrifuged to remove the supernatant; and the resulting precipitate was dried under vacuum (25 °C, -0.1 MPa).

### Example 19: Free Base Polymorph Screening Test

### Suspension method

Preparation method: 50 mg of an amorphous form of the compound represented by formula I was weighed out; a certain amount (about 0.4 mL) of the corresponding solvent was added to prepare a suspension; the suspension was stirred at 25 °C (or 50 °C) for 3 days in the dark and centrifuged to remove the supernatant; the resulting precipitate was dried under vacuum (25 °C, -0.1 MPa); and the resulting solid was collected and subjected to the XRPD test. The results are as follows:

| No. | Solvent | Crystal form (XRPD) | |
|---|---|---|---|
| | | 25 °C | 50 °C |
| 1 | Methanol | Crystal form A | Crystal form I |
| 2 | Ethanol | Crystal form B | Crystal form D |
| 3 | Isopropanol | Crystal form C | Crystal form D |
| 4 | Acetonitrile | Crystal form D | Crystal form D |
| 5 | Acetone | Crystal form E | Crystal form E |
| 6 | Ethyl acetate | Crystal form D | Crystal form D |
| 7 | Isopropyl acetate | Crystal form D | Crystal form D |
| 8 | Methyl *tert-butyl* ether | Crystal form D | Crystal form C |
| 9 | Dichloromethane | No solid sample was obtained | No solid sample was obtained |
| 10 | Tetrahydrofuran | Crystal form F | No solid sample was obtained |
| 11 | Toluene | Crystal form D | No solid sample was obtained |
| 12 | n-Heptane | Crystal forms C + D | Crystal forms C + D |
| 13 | 1,4-Dioxane | Crystal form G | Crystal form D |
| 14 | Water | Crystal form H | Crystal form J |
| 15 | MeOH/water (1:1, v/v) | Crystal form D | Crystal form D |
| 16 | ACN/water (1/1, v/v) | Crystal form D | Crystal form D |
| 17 | Acetone/water (1:1, v/v) | Crystal form D | Crystal form D |
| 18 | MA | Crystal form D | Crystal form D |
| 19 | 2-MeTHF | Crystal form D | Crystal form D |
| 20 | Cyclohexane | Crystal form D | Crystal form D |
| 21 | n-Hexane | Crystal form D | Crystal form D |
| 22 | MEK | Crystal form D | Crystal form D |
| 23 | MIBK | Crystal form D | Crystal form D |

### Volatilization method

Preparation method: about 20 mg of an amorphous form of the compound represented by formula I was weighed into a 2 mL vial; a certain amount (about 1 mL) of the corresponding solvent was added; the mixture was filtered through a 0.22 µm nylon filter; the vial was sealed with a Parafilm^{®} sealing film, and pinholes were made on the film; the resulting filtrate was placed at room temperature and volatilized slowly; and the resulting solid was collected and subjected to the XRPD test. The results are as follows:

| No. | Solvent | Crystal form (XRPD) |
|---|---|---|
| 1 | MeOH/DCM(5:1) | Crystal form K |
| 2 | EtOH/DCM(5:1)) | Crystal form L |
| 3 | IPA/DCM(5:1) | Crystal form M |
| 4 | ACN/DCM(10:3) | Crystal form D |
| 5 | Acetone | Crystal form D |
| 6 | EtOAc | Crystal form D |
| 7 | iPrOAc/DCM(5:1) | Crystal form D |
| 8 | DCM | No solid sample was obtained |
| 9 | THF | No solid sample was obtained |
| 10 | 1,4-Dioxane | Crystal form N |

### Poor solvent method

Preparation method: 50 mg of an amorphous form of the compound represented by formula I was weighed out and added to a glass flask; a certain amount (about 1 mL) of a good solvent was added; the mixture was stirred at 25 °C until the sample was dissolved to prepare a clear solution, and if the sample was not completely dissolved, the mixture was filtered through a 0.22 µm nylon filter to obtain a clear solution; 4 volumes of a poor solvent was slowly added dropwise with stirring at room temperature; the mixture was stirred for 5 days and centrifuged to remove the supernatant; the resulting precipitate was dried under vacuum (25 °C, -0.1 MPa); and the resulting solid was collected and subjected to the XRPD test. The results are as follows:

| No. | Good solvent | Poor solvent | Crystal form (XRPD) |
|---|---|---|---|
| 1 | THF | MeOH | Crystal form P |
| 2 | THF | ACN | Crystal form D |
| 3 | THF | Acetone | No solid sample was obtained |
| 4 | THF | EA | No solid sample was obtained |
| 5 | THF | MTBE | Crystal form D |
| 6 | THF | n-Heptane | Crystal form Q |
| 7 | THF | Water | Crystal form R |
| 8 | DCM | MeOH | No solid sample was obtained |
| 9 | DCM | ACN | Crystal form D |
| 10 | DCM | Acetone | Crystal form E |
| 11 | DCM | EA | Crystal form D |
| 12 | DCM | MTBE | Crystal form D |
| 13 | DCM | n-Heptane | Crystal form D |
| 14 | DCM | Water | No solid sample was obtained |
| 15 | Dioxane | MTBE | No solid sample was obtained |
| 16 | Dioxane | n-Heptane | Crystal form G |
| 17 | Dioxane | Water | Crystal form C |
| 18 | NMP | MTBE | No solid sample was obtained |
| 19 | NMP | n-Heptane | No solid sample was obtained |
| 20 | NMP | Water | Crystal form C |
| 21 | DMF | MTBE | No solid sample was obtained |
| 22 | DMF | n-Heptane | No solid sample was obtained |
| 23 | DMF | Water | Crystal form J |

### Heating and cooling method

Preparation method: about 50 mg of an amorphous form of the compound represented by formula I was weighed out and added to a glass flask; a certain amount (about 1 mL) of a solvent was added; the mixture was stirred at 50 °C for 1 h to obtain a clear solution, and if the sample was not completely dissolved, the mixture was filtered through a 0.22 µm nylon filter to obtain a clear solution; the solution was placed in a refrigerator at 4 °C until a solid was precipitated; the supernatant was removed by centrifugation; the resulting precipitate was dried under vacuum (25 °C, -0.1 MPa); and the resulting solid was collected and subjected to the XRPD test. The results are as follows:

| No. | Solvent | Crystal form (XRPD) |
|---|---|---|
| 1 | MeOH/DCM(3:1, v/v) | Crystal form O |
| 2 | EtOH/DCM(2:1, v/v) | No solid sample was obtained |
| 3 | IPA/DCM(1:1, v/v) | No solid sample was obtained |
| 4 | ACN/DCM(1:1, v/v) | Crystal form D |
| 5 | Acetone | Crystal form E |
| 6 | EtOAc | Crystal form D |
| 7 | iPrOAc/DCM(1:1, v/v) | Crystal form D |
| 8 | DCM | No solid sample was obtained |
| 9 | THF | Crystal form D |
| 10 | 1,4-Dioxane | Crystal form G |

### Example 20

Preparation method for one equivalent of mesylate crystal form A of compound represented by formula I: about 100 mg of an amorphous form of the compound represented by formula I and methanesulfonic acid (the feeding molar ratio of the acid to the base was 1:1) were stirred in 4 mL of tetrahydrofuran at room temperature for 3 days to obtain the crystal form.

Characterization of one equivalent of mesylate crystal form A of compound represented by formula I:
1) the XRPD characterization results are shown in FIG. 5;
2) the TGA characterization results are shown in FIG. 6;
3) the DSC characterization results are shown in FIG. 7;
4) the ¹H-NMR characterization results are shown in FIG. 8, showing that methanesulfonic acid and the free base in the sample were in a molar ratio of 1:1; and
5) the DVS characterization results are shown in FIG. 9.

### Example 21

Preparation method for one equivalent of mesylate crystal form B of compound represented by formula I: 50 mg of one equivalent of mesylate crystal form A was added to a 2 mL glass flask; 0.2 mL of methanol/water (50:50, v/v) was added; the suspension was slurried at 25 °C for 3 days and centrifuged to remove the supernatant; and the resulting precipitate was dried under vacuum (25 °C, -0.1 MPa).

### Example 22

Preparation method for one equivalent of mesylate crystal form C of compound represented by formula I: 50 mg of one equivalent of mesylate crystal form A was added to a 2 mL glass flask; 0.2 mL of acetonitrile was added; the suspension was slurried at 50 °C for 3 days and centrifuged to remove the supernatant; and the resulting precipitate was dried under vacuum (25 °C, -0.1 MPa).

### Example 23

Preparation method for one equivalent of mesylate crystal form D of compound represented by formula I: 50 mg of one equivalent of mesylate crystal form A was added to a 2 mL glass flask; 0.2 mL of acetone was added; the suspension was slurried at 50 °C for 3 days and centrifuged to remove the supernatant; and the resulting precipitate was dried under vacuum (25 °C, -0.1 MPa).

### Example 24

Preparation method for one equivalent of mesylate crystal form E of compound represented by formula I: 50 mg of one equivalent of mesylate crystal form A was added to a 2 mL glass flask; 0.2 mL of 1,4-dioxane was added; the suspension was slurried at 50 °C for 3 days and centrifuged to remove the supernatant; and the resulting precipitate was dried under vacuum (25 °C, -0.1 MPa).

### Example 25

Preparation method for one equivalent of mesylate crystal form F of compound represented by formula I: about 100 mg of an amorphous form of the compound represented by formula I was weighed into a glass flask; a certain amount (about 4 mL) of 1,4-dioxane was added at 50 °C; and methanesulfonic acid (the feeding molar ratio of the acid to the base was 1:1) was added. The resulting suspension was stirred at 50 °C for about 1 h, and then placed at 25 °C and stirred for 4 days. The supernatant was removed by centrifugation, and the resulting precipitate was dried under vacuum (25 °C, -0.1 MPa).

### Example 26

### Mesylate Polymorph Screening Test

Unless otherwise specified, the crystal forms referred to in Example 26 are all one equivalent of mesylate crystal forms.

### Suspension method

About 50 mg of one equivalent of mesylate crystal form A of the compound represented by formula I was weighed out; a certain amount (about 0.2 mL) of the corresponding solvent was added to prepare a suspension; the suspension was stirred at 25 °C (or 50 °C/80 °C) for 3 days in the dark and centrifuged to remove the supernatant; and the resulting precipitate was dried under vacuum (25 °C, -0.1 MPa). The results are shown in the following table.

| No. | Solvent | Crystal form (XRPD) | |
|---|---|---|---|
| | | 25 °C | 50 °C |
| 1 | Methanol | Crystal form B | Crystal form B |
| 2 | Ethanol | Crystal form A | Crystal form A |
| 3 | Isopropanol | Crystal form A | Crystal form A |
| 4 | Acetonitrile | Crystal form B | Crystal form C |
| 5 | Acetone | Crystal form A | Crystal form D |
| 6 | Ethyl acetate | Crystal form A | Crystal form A |
| 7 | Isopropyl acetate | Crystal form A | Crystal form A |
| 8 | Methyl *tert-butyl* ether | Crystal form A | Crystal form A |
| 9 | Dichloromethane | No solid sample was obtained | No solid sample was obtained |
| 10 | Tetrahydrofuran | Crystal form A | Crystal form A |
| 11 | Toluene | Crystal form A | Crystal form A |
| 12 | n-Heptane | Crystal form A | Crystal form A |
| 13 | 1,4-Dioxane | Crystal form A | Crystal form E |
| 14 | Water | Crystal form B | Crystal form B |
| 15 | Methanol-water 1:1 | Crystal form B | Clear solution |
| 16 | Acetonitrile-water 1:1 | Clear solution | Clear solution |
| 17 | Acetone-water 1:1 | Clear solution | Clear solution |
| 18 | Dichloromethane/methanol (v/v, 1:1) | Clear solution | No solid sample was obtained |
| 19 | Dichloromethane/acetone (v/v, 1:1) | Crystal form A | No solid sample was obtained |
| 20 | Dichloromethane/acetonitrile (v/v, 1:1) | Clear solution | No solid sample was obtained |
| 21 | Dichloromethane/ethyl acetate (v/v, 1:1) | Crystal form A | No solid sample was obtained |
| 22 | Dichloromethane/tetrahydrofuran (v/v, 1:1) | Crystal form A | No solid sample was obtained |
| 23 | NMP | Crystal form A | Crystal form A |
| 24 | Methyl acetate | Crystal form A | Crystal form A |
| 25 | 2-Methyltetrahydrofuran | Crystal form A | Crystal form A |
| 26 | Cyclohexane | Crystal form A | Crystal form A |
| 27 | n-Hexane | Crystal form A | Crystal form A |
| | Solvent | Crystal form (XRPD)/85 °C | |
| 28 | MIBK | Crystal form A | |
| 29 | Dioxane | Crystal form A | |
| 30 | n-Heptane | Crystal form A | |
| 31 | Toluene | Crystal form A | |

### Volatilization method

A total of 15 slow volatilization tests were set up using different solvent systems. About 20 mg of one equivalent of mesylate crystal form A of the compound represented by formula I was weighed into a 2 mL vial; a certain amount (about 1 mL) of the corresponding solvent was added; the mixture was filtered through a 0.22 µm nylon filter; the vial was sealed with a Parafilm^{®} sealing film, and pinholes were made on the film; and the resulting filtrate was placed at room temperature and volatilized slowly. The resulting solid was collected and subjected to the XRPD test. The results are shown in the following table.

| No. | Solvent | Crystal form (XRPD) |
|---|---|---|
| 1 | Methanol | Crystal form B |
| 2 | Ethanol | Glass state |
| 3 | Acetonitrile | Crystal form B |
| 4 | Acetone | Glass state |
| 5 | Dichloromethane | Crystal form B |
| 6 | Tetrahydrofuran | Colloidal |
| 7 | 1,4-Dioxane | Glass state |
| 8 | Methanol-water 1:1 | Glass state |
| 9 | Acetonitrile-water 1:1 | Glass state |
| 10 | Acetone-water 1:1 | Crystal form B |
| 11 | Dichloromethane/methanol (v/v, 1:1) | Crystal form B |
| 12 | Dichloromethane/acetone (v/v, 1:1) | Glass state |
| 13 | Dichloromethane/acetonitrile (v/v, 1:1) | Crystal form B |
| 14 | Dichloromethane/ethyl acetate (v/v, 1:1) | Crystal form B |
| 15 | Dichloromethane/tetrahydrofuran (v/v, 1:1) | Colloidal |

### Anti-solvent method

About 50 mg of one equivalent of mesylate crystal form A of the compound represented by formula I was weighed out and added to a glass flask; a certain amount (about 1 mL) of a good solvent was added; the mixture was stirred at 25 °C until the sample was dissolved to prepare a clear solution, and if the sample was not completely dissolved, the mixture was filtered through a 0.22 µm nylon filter to obtain a clear solution; 4 volumes of an anti-solvent was slowly added dropwise with stirring at room temperature; the mixture was stirred for 5 days and centrifuged to remove the supernatant; and the resulting precipitate was dried under vacuum (25 °C, -0.1 MPa).

| No. | Good solvent | Poor solvent | Crystal form (XRPD) |
|---|---|---|---|
| 1 | Methanol | Methyl *tert*-butyl ether | Crystal form B |
| 2 | Methanol | Toluene | Clear solution |
| 3 | Methanol | 1,4-Dioxane | Clear solution |
| 4 | Methanol | Water | Clear solution |
| 5 | Dichloromethane | Methyl *tert-*butyl ether | Crystal forms A + B |
| 6 | Dichloromethane | Toluene | Crystal form A |
| 7 | Dichloromethane | *n*-Heptane | Crystal forms A + B |
| 8 | Dichloromethane | 1,4-Dioxane | Crystal form F |
| 9 | Dimethyl sulfoxide | Toluene | Clear solution |
| 10 | Dimethyl sulfoxide | 1,4-Dioxane | Clear solution |
| 11 | Dimethyl sulfoxide | Water | Clear solution |
| 12 | *N,N*-Dimethylformamide | Methyl *tert-*butyl ether | Crystal form A |
| 13 | *N,N*-Dimethylformamide | Toluene | Crystal form A |
| 14 | *N,N-*Dimethylformamide | 1,4-Dioxane | Clear solution |
| 15 | *N,N-Dimethylformamide* | Water | Clear solution |

### Heating and cooling method

About 50 mg of one equivalent of mesylate crystal form A of the compound represented by formula I was weighed out and added to a glass flask; a certain amount (1 mL) of a solvent was added; the mixture was stirred at 50 °C for 1 h to obtain a clear solution, and if the sample was not completely dissolved, the mixture was filtered through a 0.22 µm nylon filter to obtain a clear solution; the solution was placed in a refrigerator at 4°C until a solid was precipitated; the supernatant was removed by centrifugation; and the resulting precipitate was dried under vacuum (25 °C, -0.1 MPa).

| No. | Solvent | Crystal form (XRPD) |
|---|---|---|
| 1 | Methanol | Crystal form B |
| 2 | Ethanol | Clear solution |
| 3 | Isopropanol | Crystal form A |
| 4 | Acetonitrile | Crystal form B |
| 5 | Acetone | Crystal form A |
| 6 | Tetrahydrofuran | Clear solution |
| 7 | 1.4-Dioxane | Clear solution |
| 8 | Water | Crystal form B |
| 9 | Methanol-water 1:1 | Clear solution |
| 10 | Acetonitrile-water 1:1 | Clear solution |
| 11 | Acetone-water 1:1 | Clear solution |

### Diffusion method

About 50 mg of one equivalent of mesylate crystal form A of the compound represented by formula I was weighed out and added to a glass flask; a certain amount (about 1 mL) of a solvent was added; the mixture was stirred at 25 °C to obtain a clear solution, and if the sample was not completely dissolved, the mixture was filtered through a 0.22 µm nylon filter to obtain a clear solution; a vial containing the clear solution was placed into a large flask containing 4 volumes of an anti-solvent (which has certain volatility and can be volatilized into a good solvent); the large flask was sealed and left to stand at room temperature; the supernatant was removed by centrifugation; and the resulting precipitate was dried under vacuum (25 °C, -0.1 MPa).

| No. | Good solvent | Poor solvent | Crystal form (XRPD) |
|---|---|---|---|
| 1 | Methanol | Acetonitrile | Clear solution |
| 2 | Methanol | Acetone | Clear solution |
| 3 | Methanol | Ethyl acetate | Clear solution |
| 4 | Methanol | Tetrahydrofuran | Clear solution |
| 5 | Dichloromethane | Ethanol | Crystal form A |
| 6 | Dichloromethane | Acetonitrile | Crystal forms A + B |
| 7 | Dichloromethane | Acetone | Crystal form A |
| 8 | Dichloromethane | Ethyl acetate | Crystal forms A + B |
| 9 | Dichloromethane | Tetrahydrofuran | Crystal forms A + B |
| 10 | Dimethyl sulfoxide | Ethanol | Clear solution |
| 11 | Dimethyl sulfoxide | Acetonitrile | Clear solution |
| 12 | Dimethyl sulfoxide | Acetone | Clear solution |
| 13 | Dimethyl sulfoxide | Ethyl acetate | Clear solution |
| 14 | Dimethyl sulfoxide | Tetrahydrofuran | Clear solution |
| 15 | *N,N-*Dimethylformamide | Ethanol | Clear solution |
| 16 | *N,N-*Dimethylformamide | Acetonitrile | Clear solution |
| 17 | *N*,*N*-Dimethylformamide | Acetone | Clear solution |
| 18 | *N,N-*Dimethylformamide | Ethyl acetate | Clear solution |
| 19 | *N,N-*Dimethylformamide | Tetrahydrofuran | Clear solution |

The results of the mesylate polymorph screening test show that in the suspension method, the one equivalent of mesylate crystal form A had relatively good stability in ethanol, isopropanol, ethyl acetate, isopropyl acetate, methyl *tert-butyl* ether, tetrahydrofuran, N-methylpyrrolidone, methyl acetate, 2-methyltetrahydrofuran, cyclohexane, n-hexane, toluene, or n-heptane. The one equivalent of mesylate crystal form A remained stable when stirred in toluene or n-heptane at 25-85 °C. The one equivalent of mesylate crystal form A remained stable when stirred in ethanol, isopropanol, ethyl acetate, isopropyl acetate, methyl *tert-butyl* ether, tetrahydrofuran, N-methylpyrrolidone, methyl acetate, 2-methyltetrahydrofuran, cyclohexane, n-hexane, toluene, or n-heptane at 25-50 °C.

### Example 27

Preparation method for one equivalent of maleate crystal form A of compound represented by formula I: about 955.9 mg of maleic acid was weighed into a 12 mL glass flask, and 7.5 mL of ethanol was added to obtain a clear solution for later use. About 5 g of an amorphous form of the compound represented by formula I was weighed into a 20 mL glass flask. 12.5 mL of ethanol and the solution of maleic acid in ethanol were added at 50 °C. The resulting suspension was stirred at 50 °C for about 1 h, and then placed at 25 °C and stirred for 7 days. The supernatant was removed by centrifugation, and the resulting precipitate was dried under vacuum (25 °C, -0.1 MPa).

Characterization of one equivalent of maleate crystal form A of compound represented by formula I:
1) the XRPD characterization results are shown in FIG. 10;
2) the TGA characterization results are shown in FIG. 11;
3) the DSC characterization results are shown in FIG. 12;
4) the ¹H-NMR characterization results are shown in FIG. 13, showing that maleic acid and the free base in the sample were in a molar ratio of 1:1, with no solvent remaining; and
5) the DVS characterization results are shown in FIG. 14, and the DVS results show that the sample exhibited a hygroscopic weight gain of 0.11% at 80% RH and had no hygroscopicity, and the crystal form did not change before and after the hygroscopic test.

### Example 28

Preparation method for one equivalent of maleate crystal form B of compound represented by formula I: 50 mg of one equivalent of maleate crystal form A was added to a 8 mL glass flask; 1 mL of dichloromethane was added; the mixture was stirred at 25 °C until the sample was dissolved and filtered through a 0.22 µm nylon filter to obtain a clear solution; 4 mL of acetone was slowly added dropwise; the mixture was stirred for 5 days and centrifuged to remove the supernatant; and the resulting precipitate was dried under vacuum (25 °C, -0.1 MPa).

### Example 29: Maleate Polymorph Screening Test

Unless otherwise specified, the crystal forms referred to in Example 29 are all one equivalent of maleate crystal forms.

### Suspension method

About 50 mg of one equivalent of maleate crystal form A of the compound represented by formula I was weighed out; a certain amount (about 0.2 mL) of the corresponding solvent was added to prepare a suspension; the suspension was stirred at 25 °C (or 50 °C) for 3 days in the dark and centrifuged to remove the supernatant; and the resulting precipitate was dried under vacuum (25 °C, -0.1 MPa). The results are shown in the following table.

**Table 86. Maleate polymorph screening test results-suspension method**

| No. | Solvent | Crystal form (XRPD) | |
|---|---|---|---|
| | | 25°C | 50°C |
| 1 | Methanol | Crystal form A | Crystal forms A + B |
| 2 | Ethanol | Crystal form A | Crystal form A |
| 3 | Isopropanol | Crystal form A | Crystal form A |
| 4 | Acetonitrile | Crystal form A | Crystal form A |
| 5 | Acetone | Crystal form A | Crystal form A |
| 6 | Ethyl acetate | Crystal form A | Crystal form A |
| 7 | Isopropyl acetate | Crystal form A | Crystal form A |
| 8 | Methyl *tert*-butyl ether | Crystal form A | Crystal form A |
| 9 | Dichloromethane | Crystal form A | Crystal form A |
| 10 | Tetrahydrofuran | Crystal form A | Crystal form A |
| 11 | Toluene | Crystal form A | Crystal form A |
| 12 | *n*-Heptane | Crystal form A | Crystal form A |
| 13 | 1,4-Dioxane | Crystal form A | Crystal form A |
| 14 | Water | Crystal form A | Crystal form A |
| 15 | Methanol-water 1:1 | Crystal form A | Crystal form A |
| 16 | Acetonitrile-water 1:1 | Crystal form A | Crystal form A |
| 17 | Acetone-water 1:1 | Crystal form A | Crystal form A |
| 18 | Methyl acetate | Crystal form A | Crystal form A |
| 19 | 2-Methyltetrahydrofuran | Crystal form A | Crystal form A |
| 20 | Cyclohexane | Crystal form A | Crystal form A |
| 21 | *n*-Hexane | Crystal form A | Crystal form A |
| 22 | Methyl ethyl ketone | Crystal form A | Crystal form A |
| 23 | Methyl isobutyl ketone | Crystal form A | Crystal form A |
| 24 | *n*-Hexane | Crystal form A | Crystal form A |

### Volatilization method

A total of 10 slow volatilization tests were set up using different solvent systems. About 20 mg of one equivalent of maleate crystal form A of the compound represented by formula I was weighed into a 2 mL vial; a certain amount (about 1 mL) of the corresponding solvent was added; the mixture was filtered through a 0.22 µm nylon filter; the vial was sealed with a Parafilm^{®} sealing film, and pinholes were made on the film; and the resulting filtrate was placed at room temperature and volatilized slowly. The resulting solid was collected and subjected to the XRPD test. The results are shown in the following table.

| No. | Solvent | Crystal form (XRPD) |
|---|---|---|
| 1 | MeOH/DCM (3:2, v/v) | Crystal form A |
| 2 | EtOH/DCM (3:3, v/v) | Crystal forms A + B |
| 3 | IPA/DCM (4:4, v/v) | Crystal forms A + B |
| 4 | ACN/DCM (5:7, v/v) | Crystal form A |
| 5 | Acetone/DCM (5:10, v/v) | Crystal form A |
| 6 | EA/DCM (5:10, v/v) | Crystal form A |
| 7 | IPAc /DCM (5:10, v/v) | Crystal form A |
| 8 | DCM | Crystal form A |
| 9 | THF/DCM (5:10, v/v) | Crystal form A |
| 10 | Dioxane/DCM (5:10, v/v) | Crystal form A |

### Anti-solvent method

About 50 mg of one equivalent of maleate crystal form A was weighed out and added to a glass flask; a certain amount (about 1 mL) of a good solvent was added; the mixture was stirred at 25 °C until the sample was dissolved to prepare a clear solution, and if the sample was not completely dissolved, the mixture was filtered through a 0.22 µm nylon filter to obtain a clear solution; 4 volumes of an anti-solvent was slowly added dropwise with stirring at room temperature; the mixture was stirred for 5 days and centrifuged to remove the supernatant; and the resulting precipitate was dried under vacuum (25 °C, -0.1 MPa).

| No. | Good solvent | Poor solvent | Crystal form (XRPD) |
|---|---|---|---|
| 1 | DCM | MeOH | No solid sample was obtained |
| 2 | DCM | ACN | Crystal form A |
| 3 | DCM | Acetone | Crystal form B |
| 4 | DCM | EA | Crystal form B |
| 5 | DCM | MTBE | Crystal form B |
| 6 | DCM | *n*-Heptane | Crystal forms A + B |
| 7 | DCM | Dioxane | Crystal form B |
| 8 | DCM | Water | No solid sample was obtained |
| 9 | DMSO | MTBE | No solid sample was obtained |
| 10 | DMSO | *n*-Heptane | No solid sample was obtained |
| 11 | DMSO | Dioxane | No solid sample was obtained |
| 12 | DMSO | Water | Crystal form A |
| 13 | NMP | MTBE | Crystal form A |
| 14 | NMP | *n*-Heptane | No solid sample was obtained |
| 15 | NMP | Dioxane | No solid sample was obtained |
| 16 | NMP | Water | Crystal form B |
| 17 | DMF | MTBE | Crystal form B |
| 18 | DMF | *n*-Heptane | No solid sample was obtained |
| 19 | DMF | Dioxane | No solid sample was obtained |
| 20 | DMF | Water | Crystal form A |

The results of the maleate polymorph screening test show that in the suspension method, the one equivalent of maleate crystal form A had relatively good stability in ethanol, isopropanol, acetone, ethyl acetate, isopropyl acetate, methyl *tert*-butyl ether, dichloromethane, tetrahydrofuran, toluene, *n*-heptane, dioxane, methyl acetate, 2-methyltetrahydrofuran, cyclohexane, *n*-hexane, methyl ethyl ketone, or methyl isobutyl ketone. The one equivalent of maleate crystal form A remained stable when stirred in the above solvents at 25-50 °C.

### Example 30

Preparation method for one equivalent of tartrate crystal form A of compound represented by formula I: about 100 mg of an amorphous form of the compound represented by formula I was weighed into a glass flask; a certain amount (4 mL) of ethanol was added at 50 °C; and tartaric acid (the feeding molar ratio of the acid to the base was 1:1) was added. The resulting suspension was stirred at 50 °C for about 1 h, and then placed at 25 °C and stirred for 4 days. The supernatant was removed by centrifugation, and the resulting precipitate was dried under vacuum (25 °C, -0.1 MPa).

### Example 31

Preparation method for one equivalent of tartrate crystal form B of compound represented by formula I: about 100 mg of an amorphous form of the compound represented by formula I was weighed into a glass flask; a certain amount (4 mL) of tetrahydrofuran was added at 50 °C; and tartaric acid (the feeding molar ratio of the acid to the base was 1:1) was added. The resulting suspension was stirred at 50 °C for about 1 h, and then placed at 25 °C and stirred for 4 days. The supernatant was removed by centrifugation, and the resulting precipitate was dried under vacuum (25 °C, -0.1 MPa).

### Example 32

Preparation method for one equivalent of tartrate crystal form C of compound represented by formula I: 50 mg of one equivalent of tartrate crystal form B was added to a 2 mL glass flask; 0.2 mL of acetonitrile was added; the suspension was slurried at 40 °C for 3 days and centrifuged to remove the supernatant; and the resulting precipitate was dried under vacuum (25 °C, -0.1 MPa).

Characterization of one equivalent of tartrate crystal form C of compound represented by formula I:
1) the XRPD characterization results are shown in FIG. 15;
2) the TGA characterization results are shown in FIG. 16;
3) the DSC characterization results are shown in FIG. 17; and
4) the ¹H-NMR characterization results are shown in FIG. 18, showing that tartaric acid and the free base in the sample were in a molar ratio of 1:1, with no solvent remaining.

### Example 33

Preparation method for one equivalent of tartrate crystal form D of compound represented by formula I: 50 mg of one equivalent of tartrate crystal form B was added to a 2 mL glass flask; 0.2 mL of ethyl acetate was added; the suspension was slurried at 40 °C for 3 days and centrifuged to remove the supernatant; and the resulting precipitate was dried under vacuum (25 °C, -0.1 MPa).

### Example 34

Preparation method for one equivalent of tartrate crystal form E of compound represented by formula I: 50 mg of one equivalent of tartrate crystal form B was added to a 2 mL glass flask; 0.2 mL of ethanol was added; the suspension was slurried at 40 °C for 3 days and centrifuged to remove the supernatant; and the resulting precipitate was dried under vacuum (25 °C, -0.1 MPa).

### Example 35

Preparation method for one equivalent of tartrate crystal form F of compound represented by formula I: 50 mg of one equivalent of tartrate crystal form B was added to a 2 mL glass flask; 0.2 mL of methanol was added; the suspension was slurried at 40 °C for 3 days and centrifuged to remove the supernatant; and the resulting precipitate was dried under vacuum (25 °C, -0.1 MPa).

### Example 36: Tartrate Polymorph Screening

Unless otherwise specified, the crystal forms referred to in Example 36 are all one equivalent of tartrate crystal forms.

### Suspension method

About 50 mg of one equivalent of tartrate crystal form B of the compound represented by formula I was weighed out; 0.2 mL of the corresponding solvent was added to prepare a suspension; the suspension was stirred at 40 °C for 3 days in the dark and centrifuged to remove the supernatant; and the resulting precipitate was dried under vacuum (25 °C, -0.1 MPa). The results are shown in the following table.

| No. | Solvent | Crystal form (XRPD) |
|---|---|---|
| 1 | Methanol | Crystal form F |
| 2 | Ethanol | Crystal form E |
| 3 | Acetonitrile | Crystal form C |
| 4 | Acetone | Crystal form B |
| 5 | Ethyl acetate | Crystal form D |
| 6 | Dichloromethane | No solid sample was obtained |
| 7 | Tetrahydrofuran | Crystal form B |
| 8 | *n*-Heptane | Crystal form B |
| 9 | 1,4-Dioxane | Crystal form B |
| 10 | Water | No solid sample was obtained |
| 11 | Methanol/water (3:1) | No solid sample was obtained |
| 12 | Acetonitrile/water (1:1) | No solid sample was obtained |
| 13 | Acetone/water (1:2) | No solid sample was obtained |

### Volatilization method

A total of 3 slow volatilization tests were set up using different solvent systems. About 20 mg of one equivalent of tartrate crystal form B of the compound represented by formula I was weighed into a 2 mL vial; a certain amount (about 1 mL) of the corresponding solvent was added; the mixture was filtered through a 0.22 µm nylon filter; the vial was sealed with a Parafilm^{®} sealing film, and pinholes were made on the film; and the resulting filtrate was placed at room temperature and volatilized slowly. The resulting solid was collected and subjected to the XRPD test. The results are shown in the following table.

| No. | Solvent | Crystal form (XRPD) |
|---|---|---|
| 1 | DCM | No solid sample was obtained |
| 2 | DCM/MeOH(1:1, v/v) | Amorphous form |
| 3 | DCM/acetone (2:1, v/v) | Amorphous form |

### Anti-solvent method

About 50 mg of one equivalent of tartrate crystal form B of the compound represented by formula I was weighed out and added to a glass flask; a certain amount (about 1 mL) of a good solvent was added; the mixture was stirred at 25 °C until the sample was dissolved to prepare a clear solution, and if the sample was not completely dissolved, the mixture was filtered through a 0.22 µm nylon filter to obtain a clear solution; 4 volumes of an anti-solvent was slowly added dropwise with stirring at room temperature; the mixture was stirred for 5 days and centrifuged to remove the supernatant; and the resulting precipitate was dried under vacuum (25 °C, -0.1 MPa).

| No. | Good solvent | Poor solvent | Crystal form (XRPD) |
|---|---|---|---|
| 1 | DCM | *n*-Heptane | Crystal form C |
| 2 | DCM | Water | No solid sample was obtained |

The results of the one equivalent of tartrate polymorph screening test show that in the suspension method, the one equivalent of tartrate crystal form B had relatively good stability in acetone, tetrahydrofuran, n-heptane, or dioxane. The one equivalent of tartrate crystal form B remained stable when stirred in the above crystal forms at 40 °C.

### Example 37

Preparation method for one equivalent of citrate crystal form A of compound represented by formula I: about 100 mg of an amorphous form of the compound represented by formula I was weighed into a glass flask; a certain amount (4 mL) of ethanol was added at 50 °C; and citric acid (the feeding molar ratio of the acid to the base was 1:1) was added. The resulting suspension was stirred at 50 °C for about 1 h, and then placed at 25 °C and stirred for 4 days. The supernatant was removed by centrifugation, and the resulting precipitate was dried under vacuum (25 °C, -0.1 MPa).

### Example 38

Preparation method for one equivalent of citrate crystal form B of compound represented by formula I: about 100 mg of an amorphous form of the compound represented by formula I was weighed into a glass flask; a certain amount (4 mL) of tetrahydrofuran was added at 50 °C; and citric acid (the feeding molar ratio of the acid to the base was 1:1) was added. The resulting suspension was stirred at 50 °C for about 1 h, and then placed at 25 °C and stirred for 4 days. The supernatant was removed by centrifugation, and the resulting precipitate was dried under vacuum (25 °C, -0.1 MPa).

Characterization of one equivalent of citrate crystal form B of compound represented by formula I:
1) the XRPD characterization results are shown in FIG. 19;
2) the TGA characterization results are shown in FIG. 20;
3) the DSC characterization results are shown in FIG. 21;
4) the ¹H-NMR characterization results are shown in FIG. 22, showing that citric acid and the free base in the sample were in a molar ratio of 1:1, with no solvent remaining; and
5) the DVS characterization results are shown in FIG. 23.

### Example 39: Citrate Polymorph Screening

Unless otherwise specified, the crystal forms referred to in Example 39 are all one equivalent of citrate crystal forms.

### Suspension method

About 50 mg of one equivalent of citrate crystal form B of the compound represented by formula I was weighed out; 0.2 mL of the corresponding solvent was added to prepare a suspension; the suspension was stirred at 40 °C for 3 days in the dark and centrifuged to remove the supernatant; and the resulting precipitate was dried under vacuum (25 °C, -0.1 MPa). The results are shown in the following table.

| No. | Solvent | Crystal form (XRPD) |
|---|---|---|
| 1 | Methanol | Crystal form B |
| 2 | Ethanol | Crystal form B |
| 3 | Acetonitrile | Crystal form B |
| 4 | Acetone | Crystal form B |
| 5 | Ethyl acetate | Crystal form B |
| 6 | Dichloromethane | Crystal form B |
| 7 | Tetrahydrofuran | Crystal form B |
| 8 | *n*-Heptane | Crystal form B |
| 9 | 1,4-Dioxane | Crystal form B |
| 10 | Water | Crystal form B |
| 11 | Methanol/water (3:1) | Crystal form B |
| 12 | Acetonitrile/water (1:1) | Crystal form B |
| 13 | Acetone/water (1:2) | Crystal form B |

### Volatilization method

A total of 3 slow volatilization tests were set up using different solvent systems. About 20 mg of one equivalent of citrate crystal form B of the compound represented by formula I was weighed into a 2 mL vial; a certain amount (about 1 mL) of the corresponding solvent was added; the mixture was filtered through a 0.22 µm nylon filter; the vial was sealed with a Parafilm^{®} sealing film, and pinholes were made on the film; and the resulting filtrate was placed at room temperature and volatilized slowly. The resulting solid was collected and subjected to the XRPD test. The results are shown in the following table.

| No. | Solvent | Crystal form (XRPD) |
|---|---|---|
| 1 | DCM | No solid sample was obtained |
| 2 | DCM/MeOH(1:1, v/v) | Crystal form B |
| 3 | DCM/acetone (1:1, v/v) | No solid sample was obtained |

### Anti-solvent method

About 50 mg of one equivalent of citrate crystal form B of the compound represented by formula I was weighed out and added to a glass flask; a certain amount (about 1 mL) of a good solvent was added; the mixture was stirred at 25 °C until the sample was dissolved to prepare a clear solution, and if the sample was not completely dissolved, the mixture was filtered through a 0.22 µm nylon filter to obtain a clear solution; 4 volumes of an anti-solvent was slowly added dropwise with stirring at room temperature; the mixture was stirred for 5 days and centrifuged to remove the supernatant; and the resulting precipitate was dried under vacuum (25 °C, -0.1 MPa).

| No. | Good solvent | Poor solvent | Crystal form (XRPD) |
|---|---|---|---|
| 1 | DMSO | *n*-Heptane | No solid sample was obtained |
| 2 | DMSO | Water | Crystal form B |

The results of the one equivalent of citrate polymorph screening test show that the one equivalent of citrate crystal form B had relatively good stability in solvents. For example, in the suspension method, when the solvent was methanol, ethanol, acetonitrile, acetone, ethyl acetate, dichloromethane, tetrahydrofuran, n-heptane, dioxane, or a mixed solvent of methanol, acetonitrile, or acetone with water, the one equivalent of citrate crystal form B remained stable when stirred at 40 °C.

### Example 40

Preparation method for one equivalent of succinate crystal form A of compound represented by formula I: about 100 mg of an amorphous form of the compound represented by formula I was weighed into a glass flask; a certain amount (4 mL) of ethanol was added at 50 °C; and succinic acid (the feeding molar ratio of the acid to the base was 1:1) was added. The resulting suspension was stirred at 50 °C for about 1 h, and then placed at 25 °C and stirred for 4 days. The supernatant was removed by centrifugation, and the resulting precipitate was dried under vacuum (25 °C, -0.1 MPa).

### Example 41

Preparation method for one equivalent of succinate crystal form B of compound represented by formula I: about 100 mg of an amorphous form of the compound represented by formula I was weighed into a glass flask; a certain amount (4 mL) of tetrahydrofuran was added at 50 °C; and succinic acid (the feeding molar ratio of the acid to the base was 1:1) was added. The resulting suspension was stirred at 50 °C for about 1 h, and then placed at 25 °C and stirred for 4 days. The supernatant was removed by centrifugation, and the resulting precipitate was dried under vacuum (25 °C, -0.1 MPa).

### Example 42

Preparation method for one equivalent of succinate crystal form C of compound represented by formula I: about 100 mg of an amorphous form of the compound represented by formula I was weighed into a glass flask; a certain amount (4 mL) of 1,4-dioxane was added at 50 °C; and succinic acid (the feeding molar ratio of the acid to the base was 1:1) was added. The resulting suspension was stirred at 50 °C for about 1 h, and then placed at 25 °C and stirred for 4 days. The supernatant was removed by centrifugation, and the resulting precipitate was dried under vacuum (25 °C, -0.1 MPa).

### Example 43

Preparation method for one equivalent of succinate crystal form D of compound represented by formula I: about 1.5 g of an amorphous form of the compound represented by formula I was weighed into a glass flask; a certain amount (about 60 mL) of ethanol was added at 50 °C; and succinic acid (the feeding molar ratio of the acid to the base was 1:1) was added. The resulting suspension was stirred at 50 °C for about 1 h, and then placed at 25 °C and stirred for 4 days. The supernatant was removed by centrifugation, and the resulting precipitate was dried under vacuum (25 °C, -0.1 MPa).

Characterization of one equivalent of succinate crystal form D of compound represented by formula I:
1) the XRPD characterization results are shown in FIG. 24;
2) the TGA characterization results are shown in FIG. 25;
3) the DSC characterization results are shown in FIG. 26;
4) the ¹H-NMR characterization results are shown in FIG. 27, showing that succinic acid and the free base in the sample were in a molar ratio of 1:1, with no solvent remaining; and
5) the DVS characterization results are shown in FIG. 28.

### Example 44: Succinate Polymorph Screening

Unless otherwise specified, the crystal forms referred to in Example 44 are all one equivalent of succinate crystal forms.

### Suspension method

About 50 mg of one equivalent of succinate crystal form D of the compound represented by formula I was weighed out; 0.2 mL of the corresponding solvent was added to prepare a suspension; the suspension was stirred at 40 °C for 3 days in the dark and centrifuged to remove the supernatant; and the resulting precipitate was dried under vacuum (25 °C, -0.1 MPa). The results are shown in the following table.

| No. | Solvent | Crystal form (XRPD) |
|---|---|---|
| 1 | Methanol | Crystal form D |
| 2 | Ethanol | Crystal form D |
| 3 | Acetonitrile | Crystal form D |
| 4 | Acetone | Crystal form D |
| 5 | Ethyl acetate | Crystal form D |
| 6 | Dichloromethane | Crystal form D |
| 7 | Tetrahydrofuran | Crystal form B |
| 8 | n-Heptane | Crystal form D |
| 9 | 1,4-Dioxane | Crystal form D |
| 10 | Water | Crystal form D |
| 11 | Methanol/water (3:1) | Crystal form D |
| 12 | Acetonitrile/water (1:1) | Crystal form D |
| 13 | Acetone/water (1:2) | Crystal form D |

### Volatilization method

A total of 3 slow volatilization tests were set up using different solvent systems. About 20 mg of one equivalent of succinate crystal form D of the compound represented by formula I was weighed into a 2 mL vial; a certain amount (about 1 mL) of the corresponding solvent was added; the mixture was filtered through a 0.22 µm nylon filter; the vial was sealed with a Parafilm^{®} sealing film, and pinholes were made on the film; and the resulting filtrate was placed at room temperature and volatilized slowly. The resulting solid was collected and subjected to the XRPD test. The results are shown in the following table.

| No. | Solvent | Crystal form (XRPD) |
|---|---|---|
| 1 | DCM | Crystal form D |
| 2 | DCM/MeOH(1:1, v/v) | Crystal form D |
| 3 | DCM/acetone (1:1, v/v) | Crystal forms A + D |

### Anti-solvent method

About 50 mg of one equivalent of succinate crystal form D of the compound represented by formula I was weighed out and added to a glass flask; a certain amount (about 1 mL) of a good solvent was added; the mixture was stirred at 25 °C until the sample was dissolved to prepare a clear solution, and if the sample was not completely dissolved, the mixture was filtered through a 0.22 µm nylon filter to obtain a clear solution; 4 volumes of an anti-solvent was slowly added dropwise with stirring at room temperature; the mixture was stirred for 5 days and centrifuged to remove the supernatant; and the resulting precipitate was dried under vacuum (25 °C, -0.1 MPa).

| No. | Good solvent | Poor solvent | Crystal form (XRPD) |
|---|---|---|---|
| 1 | DMSO | n-Heptane | Crystal form D |
| 2 | DMSO | Water | No solid sample was obtained |

The results of the one equivalent of succinate polymorph screening test show that the one equivalent of succinate crystal form D had relatively good stability in solvents. For example, in the suspension method, when the solvent was methanol, ethanol, acetonitrile, acetone, ethyl acetate, dichloromethane, water, n-heptane, dioxane, or a mixed solvent of methanol, acetonitrile, or acetone with water, the one equivalent of succinate crystal form D remained stable when stirred at 40 °C.

### Example 45

Preparation method for one equivalent of phosphate crystal form A of compound represented by formula I: about 100 mg of an amorphous form of the compound represented by formula I was weighed into a glass flask; a certain amount (about 4 mL) of ethanol was added at 50 °C; and phosphoric acid (the feeding molar ratio of the acid to the base was 1:1) was added. The resulting suspension was stirred at 50 °C for about 1 h, and then placed at 25 °C and stirred for 4 days. The supernatant was removed by centrifugation, and the resulting precipitate was dried under vacuum (25 °C, -0.1 MPa).

Preparation method for one equivalent of phosphate crystal form E of compound represented by formula I: 50 mg of one equivalent of phosphate crystal form A was added to a 2 mL glass flask; 0.2 mL of ethyl acetate was added; the suspension was slurried at 40 °C for 3 days and centrifuged to remove the supernatant; and the resulting precipitate was dried under vacuum (25 °C, -0.1 MPa).

### Example 46

Preparation method for one equivalent of hydrochloride crystal form A of compound represented by formula I: about 2 g of an amorphous form of the compound represented by formula I was weighed into a 20 mL glass flask; and 5 mL of ethanol and about 3.12 mL of a 1 M diluted solution of hydrochloric acid in ethanol were added at 50 °C. The resulting suspension was stirred at 50 °C for about 1 h, and then placed at 25 °C and stirred for 2 days. The supernatant was removed by centrifugation, and the resulting precipitate was dried under vacuum (25 °C, -0.1 MPa).

### Example 47: Stability of Crystal Form D of Compound Represented by Formula I

About 30 mg of samples of the crystal form D of the compound represented by formula I were weighed into 4 mL transparent glass flasks. The samples were placed under the stability conditions shown in the following table, sampling was performed at the corresponding time points, and the samples were subjected to appearance and XRPD tests.

5 mg of stability test samples at different time points were weighed out, 10 mL of a diluent was added for dissolution, and the resulting clear solutions were subjected to the HPLC test. The stability results obtained are recorded in the following table.

| **Sample** | **Stability condition** | | **Appearance** | **XRPD** | **Total impurities** |
|---|---|---|---|---|---|
| Crystal form D of the compound represented by formula I | // | 0d | No significant change | No significant change | 0.14% |
| | 60 °C | 10d | No significant change | No significant change | 0.17% |
| | | 1M | | | 0.20% |
| | 90% RH | 10d | No significant change | No significant change | 0.17% |
| | | 1M | | | 0.17% |
| | Illumination | 5d | Color deepening | No significant change | 0.17% |
| | | 10d | | | 2.43% |
| | In the dark | 5d | No significant change | No significant change | 0.17% |
| | | 10d | | | 0.14% |
| | 40 °C/75% RH | 10d | No significant change | No significant change | 0.17% |
| | | 1M | | | 0.17% |
| | 25 °C/60% RH | 10d | No significant change | No significant change | 0.14% |
| | | 1M | | | 0.17% |

### Example 48: Stability of Mesylate Crystal Form A

About 30 mg of samples of the mesylate crystal form A were weighed into 4 mL transparent glass flasks. The samples were placed under the stability test conditions shown in the following table, sampling was performed at different time points, and the samples were subjected to the XRPD test.

| **Sample** | **Stability condition** | | **Crystal form result by XRPD test** |
|---|---|---|---|
| Mesylate crystal form A | 60 °C | 5 days | Crystal form A |
| | | 10 days | Crystal form A |
| | | 1 month | Crystal form A |
| | | 2 months | Crystal form A |
| | | 3 months | Crystal form A |
| | | 6 months | Crystal form A |
| | 90% RH | 5 days | Crystal form A |
| | | 10 days | Crystal form A |
| | | 1 month | Crystal form A |
| | | 2 months | Crystal form A |
| | | 3 months | Crystal form A |
| | | 6 months | Crystal form A + crystal form B |
| | 40 °C/75% RH | 5 days | Crystal form A |
| | | 10 days | Crystal form A |
| | | 1 month | Crystal form A |
| | | 2 months | Crystal form A |
| | | 3 months | Crystal form A |
| | | 6 months | Crystal form A |
| | 25 °C/60% RH | 5 days | Crystal form A |
| | | 10 days | Crystal form A |
| | | 1 month | Crystal form A |
| | | 2 months | Crystal form A |
| | | 3 months | Crystal form A |
| | | 6 months | Crystal form A |

### Example 49: Stability of Maleate Crystal Form A and Hydrochloride Crystal Form A

About 30 mg of different samples (hydrochloride and maleate) were weighed into 4 mL transparent glass flasks. The samples were placed in an open state under the stability test conditions of 40 °C/75% RH and 90% RH; and the samples were placed in a closed state at 60 °C in a light box (the test samples in the dark were placed in the same manner as the illumination test samples, and the treatment was performed in the dark). Sampling was performed at different time points, and the samples were subjected to the XRPD test.

5.28 mg of hydrochloride and 5.91 mg of maleate (equivalent to 5 mg of the free base) were weighed out and placed under different stability test conditions at the same time as the above samples. Sampling was performed at different time points, 10 mL of a diluent was added for dissolution, and the resulting clear solutions were subjected to the HPLC test.

| Sample | Stability condition | | Appearance | Crystal form (XRPD) | Total impurities, % |
|---|---|---|---|---|---|
| Hydrochloride crystal form A | // | 0d | Pale yellow powder | Hydrochloride crystal form A | 0.10 |
| | 60 °C | 5d | No significant change | Hydrochloride crystal form A | 0.15 |
| | | 10d | | | 0.23 |
| | | 30d | | | 0.21 |
| | 90% RH | 5d | No significant change | Hydrochloride crystal form A | 0.17 |
| | | 10d | | | 0.28 |
| | | 30d | Color deepening | | 0.41 |
| | Illumination (1 ICH) | 5d | Color deepening | Hydrochloride crystal form A | 0.39 |
| | | 10d | | | 0.51 |
| | In the dark | 5d | No significant change | Hydrochloride crystal form A | 0.07 |
| | | 10d | | | 0.16 |
| | 40 °C/75% RH | 5d | No significant change | Hydrochloride crystal form A | 0.18 |
| | | 10d | | | 0.53 |
| | | 30d | Color deepening | | 1.09 |
| Maleate crystal form A | // | 0d | Pale yellow powder | Maleate crystal form A | 0.12 |
| | 60°C | 5d | No significant change | Maleate crystal form A | 0.16 |
| | | 10d | | | 0.21 |
| | | 30d | | | 0.16 |
| | 90% RH | 5d | No significant change | Maleate crystal form A | 0.14 |
| | | 10d | | | 0.25 |
| | | 30d | | | 0.26 |
| | Illumination (1 ICH) | 5d | Color deepening | Maleate crystal form A | 0.26 |
| | | 10d | | | 0.65 |
| | In the dark | 5d | No significant change | Maleate crystal form A | 0.12 |
| | | 10d | | | 0.18 |
| | 40 °C/75% RH | 5d | No significant change | Maleate crystal form A | 0.16 |
| | | 10d | | | 0.14 |
| | | 30d | | | 0.29 |

### Example 50: Stability of Phosphate Crystal Form E, Tartrate Crystal Form C, Citrate Crystal Form B, and Succinate Crystal Form D

About 30 mg of the phosphate crystal form E, the tartrate crystal form C, the citrate crystal form B, and the succinate crystal form D were weighed into 4 mL transparent glass flasks. The samples were placed under the stability conditions shown in the following table, sampling was performed at the corresponding time points, and the samples were subjected to appearance and XRPD tests. A certain amount of the stability samples (equivalent to 5 mg of the free base) was weighed out, 10 mL of a diluent was added for dissolution, and the resulting clear solutions were subjected to the HPLC test. The stability results obtained are recorded in the following table.

| **Sample** | **Stability condition** | | **Appearance** | **Crystal form (XRPD)** | **Total impurities, %** |
|---|---|---|---|---|---|
| Phosphate crystal form E | // | 0d | No significant change | Significant change | 1.24 |
| | 60 °C | 10d | No significant change | Significant change | 3.26 |
| | | 1M | | | 2.74 |
| | 90% RH | 10d | No significant change | Significant change | 2.13 |
| | | 1M | | | 3.03 |
| | Illumination | 5d | Color deepening | Significant change | 3.02 |
| | | 10d | | | 5.00 |
| | 40 °C/75% RH | 10d | No significant change | Significant change | 1.79 |
| | | 1M | | | 1.69 |

| **Sample** | **Stability condition** | | **Appearance** | **Crystal form (XRPD)** | **Total impurities, %** |
|---|---|---|---|---|---|
| Tartrate crystal form C | // | 0d | No significant change | No significant change | 0.28 |
| | 60 °C | 10d | No significant change | No significant change | 0.41 |
| | | 1M | | | 0.47 |
| | 90% RH | 10d | No significant change | No significant change | 0.48 |
| | | 1M | | | 0.59 |
| | Illumination | 5d | Color deepening | No significant change | 1.01 |
| | | 10d | | | 1.04 |
| | 40 °C/75% RH | 10d | No significant change | No significant change | 0.37 |
| | | 1M | | | 0.38 |

| **Sample** | **Stability condition** | | **Appearance** | **Crystal form (XRPD)** | **Total impurities, %** |
|---|---|---|---|---|---|
| Citrate crystal form B | // | 0d | No significant change | No significant change | 0.18 |
| | 60 °C | 10d | No significant change | No significant change | 0.19 |
| | | 1M | | | 0.22 |
| | 90% RH | 10d | No significant change | No significant change | 0.18 |
| | | 1M | | | 0.19 |
| | Illumination | 5d | Color deepening | No significant change | 0.48 |
| | | 10d | | | 0.55 |
| | 40 °C/75% RH | 10d | No significant change | No significant change | 0.19 |
| | | 1M | | | 0.19 |

| **Sample** | **Stability condition** | | **Appearance** | **Crystal form (XRPD)** | **Total impurities, %** |
|---|---|---|---|---|---|
| | // | 0d | No significant change | No significant change | 0.73 |
| | 60 °C | 10d | No significant change | No significant change | 0.69 |
| | | 1M | | | 0.71 |
| Succinate crystal form D | 90% RH | 10d | No significant change | No significant change | 0.73 |
| | | 1M | | | 0.96 |
| | Illumination | 5d | Color deepening | No significant change | 0.86 |
| | | 10d | | | 0.98 |
| | 40 °C/75% RH | 10d | No significant change | No significant change | 0.74 |
| | | 1M | | | 0.74 |

### Example 51: Solubility of Mesylate Crystal Form A

30 mg of the mesylate crystal form A was weighed out, and for each medium and each time point, 3 parts were weighed out and added to 15 mL centrifuge tubes; 5 mL of buffers with different pH values were added; the resulting suspensions were separately shaken (at 100 rpm) at 37 °C, and pH values were tested and recorded; and if the pH change △pH > 0.1, the pH was adjusted to the initial value with 1 M NaOH, and the suspensions were equilibrated for 1 h and then subjected to solubility tests at 15 min, 2 h, 4 h, and 24 h, each test being performed in parallel 3 times. Suspension states at 15 min, 2 h, 4 h, and 24 h were recorded; and different sample solutions at 15 min, 2 h, 4 h, and 24 h were collected and added to 1.5 mL centrifuge tubes. The suspensions were centrifuged at 12000 rpm for 2 min to obtain supernatants, and the supernatants were subjected to 2-fold dilution to obtain the sample solutions (the solutions were subjected to 10-fold dilution if the samples were dissolved completely). The residual substrates were centrifuged and subjected to the XRPD test.

| Time | pH1.2 | | 0.01 N hydrochloric acid | | pH4.5 | | pH6.8 | | Pure water | |
|---|---|---|---|---|---|---|---|---|---|---|
| | Concentr ation | Appear ance | Concentr ation | Appear ance | Concentr ation | Appear ance | Concentr ation | Appear ance | Concentr ation | Appear ance |
| 15m in | >6 | Clear solution | >6 | Clear solution | >6 | Clear solution | 0.003 | Yellow suspens ion | 0.747 | Yellow suspens ion |
| 2h | >6 | Clear solution | >6 | Clear solution | 0.044 | White turbid liquid | 0.005 | Yellow suspens ion | 4.930 | White turbid liquid |
| 4h | >6 | Clear solution | >6 | Clear solution | 0.046 | White turbid liquid | 0.005 | Yellow suspens ion | 4.657 | White turbid liquid |
| 24h | >6 | Clear solution | >6 | Clear solution | 0.043 | White turbid liquid | 0.015 | Yellow suspens ion | 4.567 | White turbid liquid |
| XRP D | // | | // | | Crystal form C of compound represented by formula I | | Mesylate crystal form A + crystal form C of compound represented by formula I | | Crystal form C of compound represented by formula I | |

### Example 52: Solubility of Mesylate Crystal Form A, Maleate Crystal Form A, and Hydrochloride Crystal Form A in Biological Media

About 20 mg of samples (mesylate, hydrochloride, and maleate) were weighted into glass flasks, and 10 mL of different media were added; the resulting suspensions were stirred at 37 °C, and about 3 mL of different sample solutions were collected at 2 h (ensuring that a certain amount of sample suspensions still existed in the glass flasks); the suspensions were filtered through a 0.22 µm filter membrane, and the subsequent filtrates were collected to obtain the sample solutions; the filtrates were diluted in a certain factor and subjected to the HPLC test (if the samples were completely dissolved, this phenomenon was recorded, and if the amount of the dissolved sample was relatively small, the filtrates were subjected to 2-fold dilution for test); sampling was performed at 24 h according to the same steps, and pH and HPLC tests were performed; and the undissolved solids in the flasks were subjected to the XRPD test.

Solubility test results (37 °C, mg/mL) of mesylate crystal form A, maleate crystal form A, and hydrochloride crystal form A in biological media

| **Medium** | **Mesylate** | | **Hydrochloride** | | **Maleate** | |
|---|---|---|---|---|---|---|
| | **2h** | **24h** | **2h** | **24h** | **2h** | **24h** |
| **SGF** | 1.86 | 1.92 | 0.87 | 1.00 | 1.39 | 1.92 |
| **FaSSIF-V2** | 0.04 | <LOQ | <LOQ | <LOQ | 0.02 | <LOQ |
| **FeSSIF-V2** | 0.90 | 1.19 | 1.58 | 1.73 | 0.22 | 0.22 |
| **Water** | 1.25 | 1.25 | 0.43 | 0.49 | 0.02 | 0.02 |

XRPD results of residual solids in solubility test of mesylate crystal form A, maleate crystal form A, and hydrochloride crystal form A in biological media

| **Medium** | **Mesylate** | **Hydrochloride** | **Maleate** |
|---|---|---|---|
| **SGF** | // | Crystal form B of compound represented by formula I + crystal form A | // |
| **FaSSIF-V2** | Crystal form C of compound represented by formula I | Crystal form B of compound represented by formula I | Crystal form C of compound represented by formula I + crystal form A |
| **FeSSIF-V2** | // | // | Crystal form C of compound represented by formula I + crystal form A |
| **Water** | Crystal form C of compound represented by formula I | Crystal form B of compound represented by formula I + crystal form A | Crystal form A |

The crystal form provided by the present disclosure has the advantages of high crystallinity, good stability, slight hygroscopicity, high solubility, and high bioavailability.

## Claims

1. A crystal form of a compound represented by formula I or a salt thereof, being one equivalent of mesylate crystal form A of the compound represented by formula I, crystal form D of the compound represented by formula I, one equivalent of maleate crystal form A of the compound represented by formula I, one equivalent of tartrate crystal form C of the compound represented by formula I, one equivalent of citrate crystal form B of the compound represented by formula I, or one equivalent of succinate crystal form D of the compound represented by formula I, 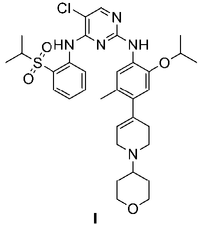
wherein an X-ray powder diffraction pattern of the one equivalent of mesylate crystal form A of the compound represented by formula I, measured using Cu-Kα radiation, has at least three, at least four, at least five, at least six, or at least seven characteristic peaks at the following 2θ angles: 10.2973°±0.2°, 14.7185°±0.2°, 15.1407°±0.2°, 17.8003°±0.2°, 18.3418°±0.2°, 20.6191°±0.2°, and 20.7584°±0.2°;
an X-ray powder diffraction pattern of the crystal form D of the compound represented by formula I, measured using Cu-Kα radiation, has at least three, at least four, at least five, at least six, or at least seven characteristic peaks at the following 2θ angles: 11.539°+0.2°, 12.658°+0.2°, 13.558°+0.2°, 18.081°±0.2°, 18.859°+0.2°, 20.278°±0.2°, and 23.157°±0.2°;
an X-ray powder diffraction pattern of the one equivalent of maleate crystal form A of the compound represented by formula I, measured using Cu-Kα radiation, has at least three, at least four, at least five, at least six, or at least seven characteristic peaks at the following 20 angles: 5.2344°±0.2°, 10.8375°±0.2°, 12.9364°±0.2°, 17.781°±0.2°, 18.6613°±0.2°, 18.9188°±0.2°, and 20.1186°±0.2°;
an X-ray powder diffraction pattern of the one equivalent of tartrate crystal form C of the compound represented by formula I, measured using Cu-Kα radiation, has at least three, at least four, at least five, at least six, or at least seven characteristic peaks at the following 2θ angles: 10.196°±0.2°, 15.543°±0.2°, 15.757°±0.2°, 17.004°±0.2°, 18.196°±0.2°, 20.699°±0.2°, and 21.665°±0.2°;
an X-ray powder diffraction pattern of the one equivalent of citrate crystal form B of the compound represented by formula I, measured using Cu-Kα radiation, has at least three, at least four, at least five, at least six, or at least seven characteristic peaks at the following 2θ angles: 10.196°±0.2°, 15.543°±0.2°, 15.757°±0.2°, 17.004°±0.2°, 18.196°±0.2°, 20.699°+0.2°, and 21.665°±0.2°;
an X-ray powder diffraction pattern of the one equivalent of succinate crystal form D of the compound represented by formula I, measured using Cu-Kα radiation, has at least three, at least four, at least five, at least six, or at least seven characteristic peaks at the following 2θ angles: 15.715°±0.2°, 17.778°±0.2°, 18.44°±0.2°, 18.698°±0.2°, 18.86°±0.2°, 20.079°±0.2°, and 20.218°±0.2°.

2. The crystal form according to claim 1, wherein one or more of the following conditions are met:
(1) the X-ray powder diffraction pattern of the one equivalent of mesylate crystal form A, measured using Cu-Kα radiation, has diffraction peaks at the following 2θ positions: 10.2973°±0.2°, 15.1407°±0.2°, and 17.8003°±0.2°; for example, 10.2973°±0.2°, 15.1407°±0.2°, 17.8003°±0.2°, and 20.6191°±0.2°; for another example, 10.2973°±0.2°, 15.1407°±0.2°, 17.8003°±0.2°, 18.3418°±0.2°, and 20.6191°±0.2°; for another example, 10.2973°±0.2°, 15.1407°±0.2°, 17.8003°±0.2°, 18.3418°±0.2°, 20.6191°±0.2°, and 20.7584°±0.2°; for another example, 10.2973°±0.2°, 14.7185°±0.2°, 15.1407°±0.2°, 17.8003°±0.2°, 18.3418°±0.2°, 20.6191°±0.2°, and 20.7584°±0.2°; for another example, 10.2973°±0.2°, 10.7003°±0.2°, 11.5381°±0.2°, 12.618°±0.2°, 12.918°±0.2°, 13.995°±0.2°, 14.7185°±0.2°, 15.1407°±0.2°, 15.6419°±0.2°, 17.8003°±0.2°, 18.1003°±0.2°, 18.3418°±0.2°, 18.7198°±0.2°, 19.281°±0.2°, 19.8025°±0.2°, 20.3791°±0.2°, 20.6191°±0.2°, 20.7584°+0.2°, 21.2626°±0.2°, 21.4387°±0.2°, 21.7211°±0.2°, 22.0791°±0.2°, 22.442°±0.2°, 22.8785°±0.2°, 23.1402°±0.2°, 23.4006°±0.2°, 23.739°±0.2°, 24.2791°±0.2°, 24.6595°±0.2°, and 25.2227°±0.2°; preferably, X-ray powder diffraction pattern analysis data of the one equivalent of mesylate crystal form A are substantially as shown in the following table:
| Position [°2θ]+0.2° | d-spacing [Å] | Relative intensity [%] | Position [°2θ]±0.2° | d-spacing [Å] | Relative intensity [%] |
|---|---|---|---|---|---|
| 10.2973 | 8.58364 | 100.00% | 20.3791 | 4.35427 | 9.60% |
| 10.7003 | 8.2612 | 3.40% | 20.6191 | 4.30414 | 65.00% |
| 11.5381 | 7.66317 | 11.30% | 20.7584 | 4.27556 | 44.80% |
| 12.618 | 7.00966 | 35.70% | 21.2626 | 4.17531 | 32.20% |
| 12.918 | 6.84756 | 24.00% | 21.4387 | 4.14141 | 13.80% |
| 13.995 | 6.32292 | 4.70% | 21.7211 | 4.0882 | 33.20% |
| 14.7185 | 6.01369 | 44.60% | 22.0791 | 4.02272 | 5.20% |
| 15.1407 | 5.84694 | 69.20% | 22.442 | 3.95847 | 27.40% |
| 15.6419 | 5.6607 | 20.60% | 22.8785 | 3.88393 | 20.60% |
| 17.8003 | 4.97888 | 87.70% | 23.1402 | 3.84059 | 26.00% |
| 18.1003 | 4.89702 | 31.00% | 23.4006 | 3.79845 | 11.10% |
| 18.3418 | 4.83307 | 52.80% | 23.739 | 3.74505 | 10.70% |
| 18.7198 | 4.73634 | 35.50% | 24.2791 | 3.66295 | 5.30% |
| 19.281 | 4.59973 | 18.00% | 24.6595 | 3.60731 | 30.50% |
| 19.8025 | 4.47975 | 6.90% | 25.2227 | 3.52802 | 10.10% |
;
(2) a thermogravimetric analysis curve of the one equivalent of mesylate crystal form A shows a weight loss of 0.02143±0.005% during heating from 39.18±3 °C to 150.63±3 °C;
for example, the thermogravimetric analysis curve of the one equivalent of mesylate crystal form A shows a weight loss of 0.02143% during heating from 39.18 °C to 150.63 °C;
(3) a differential scanning calorimetry (DSC) curve of the one equivalent of mesylate crystal form A has endothermic peaks with initial temperatures of 85.22±3 °C and 220.46±3 °C, respectively;
for example, the differential scanning calorimetry (DSC) curve of the one equivalent of mesylate crystal form A has endothermic peaks with initial temperatures of 85.22 °C and 220.46 °C, respectively;
(4) the differential scanning calorimetry (DSC) curve of the one equivalent of mesylate crystal form A has endothermic peaks with peak temperatures of 86.90+3 °C and 222.22+3 °C, respectively;
for example, the differential scanning calorimetry (DSC) curve of the one equivalent of mesylate crystal form A has endothermic peaks with peak temperatures of 86.90 °C and 222.22 °C, respectively;
(5) a dynamic vapor sorption (DVS) curve of the one equivalent of mesylate crystal form A shows a hygroscopic weight gain of 0.31±0.005% at 25 °C and 80% RH;
for example, the dynamic vapor sorption (DVS) curve of the one equivalent of mesylate crystal form A shows a hygroscopic weight gain of 0.31% at 25 °C and 80% RH;
(6) the X-ray powder diffraction pattern of the crystal form D, measured using Cu-Kα radiation, has diffraction peaks at the following 2θ positions: 18.081°±0.2°, 20.278°±0.2°, and 23.157°±0.2°; for example, 18.081°±0.2°, 20.278°±0.2°, and 23.157°±0.2°; for another example, 13.558°±0.2°, 18.081°±0.2°, 20.278°±0.2°, and 23.157°+0.2°; for another example, 13.558°+0.2°, 18.081°±0.2°, 18.859°+0.2°, 20.278°±0.2°, and 23.157°±0.2°; for another example, 12.658°±0.2°, 13.558°±0.2°, 18.081°±0.2°, 18.859°±0.2°, 20.278°±0.2°, and 23.157°±0.2°; for another example, 11.539°±0.2°, 12.658°±0.2°, 13.558°±0.2°, 18.081°±0.2°,18.859°+0.2°, 20.278°±0.2°, and 23.157°+0.2°; for another example, 6.297°±0.2°, 6.737°±0.2°, 11.097°±0.2°, 11.539°±0.2°, 12.381°±0.2°, 12.658°±0.2°, 12.837°±0.2°, 13.558°±0.2°, 14.102°±0.2°, 14.996°±0.2°, 15.66°±0.2°, 16.359°±0.2°, 16.522°±0.2°, 17.04°±0.2°, 17.2°±0.2°, 17.416°±0.2°, 18.081°±0.2°,18.32°±0.2°, 18.515°±0.2°, 18.859°±0.2°, 19.501°±0.2°, 20.278°±0.2°, 20.541°±0.2°, 20.7°±0.2°, 20.937°±0.2°, 21.179°+0.2°, 21.801°±0.2°, 22.057°+0.2°, 22.543°±0.2°, and 23.157°±0.2°; preferably, X-ray powder diffraction pattern analysis data of the crystal form D are substantially as shown in the following table:
| Position [°2θ]±0.2° | d-spacing [Å] | Relative intensity [%] | Position [°2θ]±0.2° | d-spacing [Å] | Relative intensity [%] |
|---|---|---|---|---|---|
| 6.297 | 14.0244 | 2.10% | 17.416 | 5.0879 | 18.30% |
| 6.737 | 13.1091 | 14.50% | 18.081 | 4.9022 | 56.10% |
| 11.097 | 7.9668 | 5.70% | 18.32 | 4.8387 | 13.80% |
| 11.539 | 7.6629 | 23.70% | 18.515 | 4.7882 | 17.30% |
| 12.381 | 7.1434 | 3.70% | 18.859 | 4.7018 | 29.30% |
| 12.658 | 6.9878 | 25.90% | 19.501 | 4.5483 | 7.80% |
| 12.837 | 6.8905 | 16.40% | 20.278 | 4.3758 | 100.00% |
| 13.558 | 6.5255 | 40.90% | 20.541 | 4.3203 | 7.50% |
| 14.102 | 6.2751 | 8.90% | 20.7 | 4.2876 | 5.10% |
| 14.996 | 5.9031 | 1.20% | 20.937 | 4.2394 | 5.10% |
| 15.66 | 5.6541 | 17.90% | 21.179 | 4.1916 | 7.40% |
| 16.359 | 5.4142 | 19.70% | 21.801 | 4.0734 | 12.00% |
| 16.522 | 5.361 | 15.40% | 22.057 | 4.0267 | 5.70% |
| 17.04 | 5.1992 | 17.90% | 22.543 | 3.9409 | 2.20% |
| 17.2 | 5.1513 | 15.30% | 23.157 | 3.8379 | 41.80% |
(7) a thermogravimetric analysis (TGA) curve of the crystal form D shows a weight loss of 0.2139±0.005% during heating from 37.69±3 °C to 150.63±3 °C;
for example, the thermogravimetric analysis (TGA) curve of the crystal form D shows a weight loss of 0.2139% during heating from 37.69 °C to 150.63 °C;
(8) a differential scanning calorimetry (DSC) curve of the crystal form D has an endothermic peak with an initial temperature of 165.58±3 °C;
for example, the differential scanning calorimetry (DSC) curve of the crystal form D has an endothermic peak with an initial temperature of 165.58 °C;
(9) the differential scanning calorimetry (DSC) curve of the crystal form D has an endothermic peak with a peak temperature of 167.41±3 °C;
for example, the differential scanning calorimetry (DSC) curve of the crystal form D has an endothermic peak with a peak temperature of 167.41 °C;
(10) a dynamic vapor sorption (DVS) curve of the crystal form D shows a hygroscopic weight gain of 0.14±0.005% at 25 °C and 80% RH;
for example, the dynamic vapor sorption (DVS) curve of the crystal form D shows a hygroscopic weight gain of 0.14% at 25 °C and 80% RH;
(11) the X-ray powder diffraction pattern of the one equivalent of maleate crystal form A, measured using Cu-Kα radiation, has diffraction peaks at the following 2θ positions: 17.781°±0.2°, 18.6613°±0.2°, and 20.1186°±0.2°; for example, 10.8375°±0.2°, 17.781°±0.2°, 18.6613°±0.2°, and 20.1186°±0.2°; for another example, 10.8375°±0.2°, 17.781°±0.2°, 18.6613°±0.2°, 18.9188°±0.2°, and 20.1186°±0.2°; for another example, 5.2344°+0.2°, 10.8375°±0.2°, 17.781°+0.2°, 18.6613°±0.2°, 18.9188°+0.2°, and 20.1186°+0.2°; for another example, 5.2344°±0.2°, 10.8375°±0.2°, 12.9364°±0.2°, 17.781°±0.2°, 18.6613°±0.2°, 18.9188°±0.2°, and 20.1186°±0.2°; for another example, 5.2344°±0.2°, 8.0562°±0.2°, 9.3187°±0.2°, 9.981°±0.2°, 10.4404°+0.2°, 10.8375°+0.2°, 12.5202°±0.2°, 12.9364°+0.2°, 13.9614°+0.2°, 14.9983°+0.2°, 15.6783°±0.2°, 17.781°±0.2°, 18.3794°±0.2°, 18.6613°±0.2°, 18.9188°±0.2°, 19.4195°±0.2°, 19.701°±0.2°, 20.1186°±0.2°, 20.5991°±0.2°, 20.9388°±0.2°, 22.6192°±0.2°, 22.739°±0.2°, 23.3011°±0.2°, 24.6412°±0.2°, 25.1802°±0.2°, 26.1581°±0.2°, 28.061°±0.2°, 29.7621°±0.2°, 30.2013°±0.2°, and 30.9209°±0.2°; preferably, X-ray powder diffraction pattern analysis data of the one equivalent of maleate crystal form A are substantially as shown in the following table:
| Position [°2θ]+0.2° | d-spacing [Å] | Relative intensity [%] | Position [°2θ]±0.2° | d-spacing [Å] | Relative intensity [%] |
|---|---|---|---|---|---|
| 5.2344 | 16.86931 | 34.50% | 19.4195 | 4.56722 | 7.60% |
| 8.0562 | 10.96576 | 33.60% | 19.701 | 4.50259 | 19.40% |
| 9.3187 | 9.48276 | 12.10% | 20.1186 | 4.41008 | 60.80% |
| 9.981 | 8.85495 | 23.50% | 20.5991 | 4.30827 | 20.20% |
| 10.4404 | 8.4663 | 15.80% | 20.9388 | 4.23914 | 12.20% |
| 10.8375 | 8.15697 | 44.70% | 22.6192 | 3.92786 | 19.90% |
| 12.5202 | 7.06419 | 12.20% | 22.739 | 3.90744 | 22.70% |
| 12.9364 | 6.83786 | 34.10% | 23.3011 | 3.81444 | 23.60% |
| 13.9614 | 6.33807 | 23.10% | 24.6412 | 3.60994 | 11.20% |
| 14.9983 | 5.90214 | 15.70% | 25.1802 | 3.53388 | 26.50% |
| 15.6783 | 5.64762 | 27.00% | 26.1581 | 3.40394 | 27.50% |
| 17.781 | 4.98423 | 50.50% | 28.061 | 3.17728 | 16.40% |
| 18.3794 | 4.82328 | 31.40% | 29.7621 | 2.99944 | 20.70% |
| 18.6613 | 4.75104 | 100.00% | 30.2013 | 2.95681 | 4.20% |
| 18.9188 | 4.68697 | 40.80% | 30.9209 | 2.88962 | 9.30% |
;
(12) a thermogravimetric analysis (TGA) curve of the one equivalent of maleate crystal form A shows a weight loss of 0.1441±0.005% during heating from 30.24±3 °C to 150.15±3 °C;
for example, the thermogravimetric analysis (TGA) curve of the one equivalent of maleate crystal form A shows a weight loss of 0.1441% during heating from 30.24 °C to 150.15 °C;
(13) a differential scanning calorimetry (DSC) curve of the one equivalent of maleate crystal form A has an endothermic peak with an initial temperature of 212.82±3 °C;
for example, the differential scanning calorimetry (DSC) curve of the one equivalent of maleate crystal form A has an endothermic peak with an initial temperature of 212.82 °C;
**(14)** the differential scanning calorimetry (DSC) curve of the one equivalent of maleate crystal form A has an endothermic peak with a peak temperature of 213.55±3 °C;
for example, the differential scanning calorimetry (DSC) curve of the one equivalent of maleate crystal form A has an endothermic peak with a peak temperature of 213.55 °C;
(15) a dynamic vapor sorption (DVS) curve of the one equivalent of maleate crystal form A shows a hygroscopic weight gain of 0.11±0.005% at 25 °C and 80% RH;
for example, the dynamic vapor sorption (DVS) curve of the one equivalent of maleate crystal form A shows a hygroscopic weight gain of **0.11%** at 25 °C and 80% RH;
(16) the X-ray powder diffraction pattern of the one equivalent of tartrate crystal form C, measured using Cu-Kα radiation, has diffraction peaks at the following 2θ positions: 17.781°±0.2°, 18.6613°+0.2°, and 20.1186°±0.2°; for example, 10.196°±0.2°, 15.543°±0.2°, 17.004°±0.2°, and 18.196°±0.2°; for another example, 10.196°±0.2°, 15.543°±0.2°, 17.004°±0.2°, 18.196°±0.2°, and 20.699°±0.2°; for another example, 10.196°±0.2°, 15.543°+0.2°, 15.757°+0.2°, 17.004°+0.2°, 18.196°±0.2°, and 20.699°+0.2°; for another example, 10.196°±0.2°, 15.543°±0.2°, 15.757°±0.2°, 17.004°±0.2°, 18.196°±0.2°, 20.699°±0.2°, and 21.665°±0.2°; for another example, 8.48°±0.2°, 10.196°±0.2°, 14.537°±0.2°, 15.052°±0.2°, 15.277°±0.2°, 15.543°±0.2°, 15.757°+0.2°, 16.003°+0.2°, 16.377°+0.2°, 17.004°+0.2°, 18.196°+0.2°, 18.782°+0.2°, 19.226°±0.2°, 19.608°±0.2°, 20.001°±0.2°, 20.699°±0.2°, 21.665°±0.2°, 22.058°±0.2°, 22.545°±0.2°, 22.875°±0.2°, 23.097°±0.2°, 23.505°±0.2°, 23.819°±0.2°, 24.313°±0.2°, 24.642°±0.2°, 25.027°±0.2°, 25.699°±0.2°, 26.666°±0.2°, 26.867°±0.2°, and 27.857°±0.2°; preferably, X-ray powder diffraction pattem analysis data of the one equivalent of tartrate crystal form C are substantially as shown in the following table:
| Position [°2θ]±0.2° | d-spacing [Å] | Relative intensity [%] | Position [°2θ]±0.2° | d-spacing [Å] | Relative intensity [%] |
|---|---|---|---|---|---|
| 8.48 | 10.419 | 1.60% | 20.699 | 4.2877 | 37.10% |
| 10.196 | 8.669 | 44.50% | 21.665 | 4.0987 | 30.00% |
| 14.537 | 6.0882 | 14.50% | 22.058 | 4.0264 | 25.80% |
| 15.052 | 5.8813 | 1.30% | 22.545 | 3.9407 | 25.90% |
| 15.277 | 5.7951 | 6.50% | 22.875 | 3.8845 | 24.30% |
| 15.543 | 5.6965 | 48.50% | 23.097 | 3.8477 | 2.90% |
| 15.757 | 5.6195 | 32.10% | 23.505 | 3.7818 | 15.00% |
| 16.003 | 5.5339 | 16.80% | 23.819 | 3.7327 | 18.50% |
| 16.377 | 5.4083 | 24.60% | 24.313 | 3.6578 | 2.40% |
| 17.004 | 5.2103 | 39.50% | 24.642 | 3.6098 | 3.50% |
| 18.196 | 4.8714 | 100.00% | 25.027 | 3.5552 | 2.70% |
| 18.782 | 4.7208 | 16.10% | 25.699 | 3.4637 | 21.30% |
| 19.226 | 4.6128 | 2.70% | 26.666 | 3.3402 | 4.80% |
| 19.608 | 4.5238 | 3.20% | 26.867 | 3.3157 | 4.00% |
| 20.001 | 4.4357 | 29.00% | 27.857 | 3.2001 | 1.30% |
(17) a thermogravimetric analysis (TGA) curve of the one equivalent of tartrate crystal form C shows a weight loss of 0.04074±0.005% during heating from 29.99±3 °C to 150.15±3 °C;
for example, the thermogravimetric analysis (TGA) curve of the one equivalent of tartrate crystal form C shows a weight loss of 0.04074% during heating from 29.99 °C to 150.15 °C;
(18) a differential scanning calorimetry (DSC) curve of the one equivalent of tartrate crystal form C has an endothermic peak with an initial temperature of 188.73±3 °C;
for example, the differential scanning calorimetry (DSC) curve of the one equivalent of tartrate crystal form C has an endothermic peak with an initial temperature of 188.73 °C;
(19) the differential scanning calorimetry (DSC) curve of the one equivalent of tartrate crystal form C has an endothermic peak with a peak temperature of 190.42±3 °C;
for example, the differential scanning calorimetry (DSC) curve of the one equivalent of tartrate crystal form C has an endothermic peak with a peak temperature of 190.42 °C;
(20) ¹H-NMR results of the one equivalent of tartrate crystal form C show that tartaric acid and a free base in a sample are in a molar ratio of 1:1, with no solvent remaining;
(21) the X-ray powder diffraction pattern of the one equivalent of citrate crystal form B, measured using Cu-Kα radiation, has diffraction peaks at the following 2θ positions: 16.8372°±0.2°, 21.4798°±0.2°, and 23.6411°±0.2°; for example, 15.0928°±0.2°, 16.8372°±0.2°, 21.4798°±0.2°, and 23.6411°±0.2°; for another example, 15.0928°±0.2°, 16.8372°±0.2°, 19.194°+0.2°, 21.4798°+0.2°, and 23.6411°±0.2°; for another example, 15.0928°±0.2°, 16.8372°±0.2°, 18.5382°±0.2°, 19.194°±0.2°, 21.4798°±0.2°, and 23.6411°±0.2°; for another example, 15.0928°±0.2°, 16.8372°±0.2°, 18.5382°±0.2°, 19.194°±0.2°, 21.4798°±0.2°, 23.6411°±0.2°, and 26.9181°+0.2°; for another example, 7.5584°+0.2°, 9.2726°+0.2°, 10.6593°+0.2°, 11.3352°±0.2°, 12.54°±0.2°, 13.5438°±0.2°, 13.7735°±0.2°, 14.0152°±0.2°, 15.0928°±0.2°, 15.4425°±0.2°, 15.628°±0.2°, 16.8372°±0.2°, 18.5382°±0.2°, 19.194°±0.2°, 21.1607°±0.2°, 21.4798°±0.2°, 22.0822°±0.2°, 22.7152°±0.2°, 23.6411°±0.2°, 24.0169°±0.2°, 24.6133°±0.2°, 26.9181°±0.2°, 27.5774°±0.2°, 27.9026°±0.2°, 30.3368°±0.2°, 32.439°±0.2°, 33.9973°±0.2°, 34.2994°±0.2°, 34.7631°±0.2°, and 35.1435°±0.2°; preferably, X-ray powder diffraction pattern analysis data of the one equivalent of citrate crystal form B are substantially as shown in the following table:
| Position [°2θ]±0.2° | d-spacing [Å] | Relative intensity [%] | Position [°2θ]+0.2° | d-spacing [Å] | Relative intensity [%] |
|---|---|---|---|---|---|
| 7.5584 | 11.68671 | 5.80% | 21.4798 | 4.13358 | 55.30% |
| 9.2726 | 9.52976 | 13.70% | 22.0822 | 4.02216 | 10.30% |
| 10.6593 | 8.29289 | 30.00% | 22.7152 | 3.91149 | 11.80% |
| 11.3352 | 7.79989 | 13.90% | 23.6411 | 3.76034 | 100.00% |
| 12.54 | 7.05311 | 12.70% | 24.0169 | 3.70234 | 23.00% |
| 13.5438 | 6.53253 | 4.80% | 24.6133 | 3.61397 | 14.70% |
| 13.7735 | 6.42407 | 20.20% | 26.9181 | 3.30954 | 33.50% |
| 14.0152 | 6.31383 | 11.10% | 27.5774 | 3.23189 | 15.00% |
| 15.0928 | 5.8654 | 45.40% | 27.9026 | 3.19496 | 23.90% |
| 15.4425 | 5.73333 | 14.50% | 30.3368 | 2.94392 | 10.80% |
| 15.628 | 5.66571 | 15.80% | 32.439 | 2.75778 | 13.40% |
| 16.8372 | 5.26142 | 80.80% | 33.9973 | 2.63484 | 10.20% |
| 18.5382 | 4.78231 | 38.10% | 34.2994 | 2.61233 | 1.90% |
| 19.194 | 4.62037 | 41.20% | 34.7631 | 2.57854 | 0.90% |
| 21.1607 | 4.19519 | 15.10% | 35.1435 | 2.55149 | 2.80% |
(22) a thermogravimetric analysis (TGA) curve of the one equivalent of citrate crystal form B shows a weight loss of 19.64±0.005% during heating from 35.86±3 °C to 212.99±3 °C;
for example, the thermogravimetric analysis (TGA) curve of the one equivalent of citrate crystal form B shows a weight loss of 19.64% during heating from 35.86 °C to 212.99 °C;
(23) a differential scanning calorimetry (DSC) curve of the one equivalent of citrate crystal form B has an endothermic peak with an initial temperature of 178.23±3 °C;
for example, the differential scanning calorimetry (DSC) curve of the one equivalent of citrate crystal form B has an endothermic peak with an initial temperature of 178.23 °C;
(24) the differential scanning calorimetry (DSC) curve of the one equivalent of citrate crystal form B has an endothermic peak with a peak temperature of 180.38±3 °C;
for example, the differential scanning calorimetry (DSC) curve of the one equivalent of citrate crystal form B has an endothermic peak with a peak temperature of 180.38 °C;
(25) ¹H-NMR results of the one equivalent of citrate crystal form B show that citric acid and a free base in a sample are in a molar ratio of 1:1, with no solvent remaining;
(26) a dynamic vapor sorption (DVS) curve of the one equivalent of citrate crystal form B shows a hygroscopic weight gain of 0. 19±0.005% at 25 °C and 80% RH;
for example, the dynamic vapor sorption (DVS) curve of the one equivalent of citrate crystal form B shows a hygroscopic weight gain of 0.19% at 25 °C and 80% RH;
(27) the X-ray powder diffraction pattern of the one equivalent of succinate crystal form D, measured using Cu-Kα radiation, has diffraction peaks at the following 2θ positions: 17.778°±0.2°, 18.698°±0.2°, and 20.079°±0.2°; for example, 17.778°±0.2°, 18.698°±0.2°, 18.86°±0.2°, and 20.079°±0.2°; for another example, 17.778°±0.2°, 18.44°±0.2°, 18.698°±0.2°, 18.86°±0.2°, and 20.079°±0.2°; for another example, 17.778°±0.2°, 18.44°±0.2°, 18.698°±0.2°, 18.86°±0.2°, 20.079°±0.2°, and 20.218°±0.2°; for another example, 15.715°±0.2°, 17.778°±0.2°, 18.44°±0.2°, 18.698°±0.2°, 18.86°±0.2°, 20.079°±0.2°, and 20.218°±0.2°; for another example, 5.255°±0.2°, 8.059°±0.2°, 9.338°±0.2°, 9.941°±0.2°, 10.472°±0.2°, 11.401°±0.2°, 12.483°±0.2°, 12.86°±0.2°, 13.203°±0.2°, 13.901°±0.2°, 14.72°±0.2°, 15.038°±0.2°, 15.715°±0.2°, 16.36°±0.2°, 17.778°±0.2°, 18.44°±0.2°, 18.698°±0.2°, 18.86°±0.2°, 19.421°±0.2°, 19.719°±0.2°, 20.079°±0.2°, 20.218°±0.2°, 20.542°±0.2°, 20.862°±0.2°, 20.997°±0.2°, 21.579°±0.2°, 22.461°±0.2°, 22.679°±0.2°, 22.899°±0.2°, and 23.222°±0.2°; preferably, X-ray powder diffraction pattern analysis data of the one equivalent of succinate crystal form D are substantially as shown in the following table:
| Position [°2θ]+0.2° | d-spacing [Å] | Relative intensity [%] | Position [°2θ]±0.2° | d-spacing [Å] | Relative intensity [%] |
|---|---|---|---|---|---|
| 5.255 | 16.8041 | 38.10% | 18.44 | 4.8076 | 58.90% |
| 8.059 | 10.962 | 37.20% | 18.698 | 4.7418 | 79.40% |
| 9.338 | 9.4636 | 13.00% | 18.86 | 4.7013 | 59.20% |
| 9.941 | 8.8906 | 23.60% | 19.421 | 4.5669 | 17.20% |
| 10.472 | 8.4405 | 23.50% | 19.719 | 4.4985 | 21.40% |
| 11.401 | 7.7552 | 7.80% | 20.079 | 4.4186 | 99.10% |
| 12.483 | 7.0851 | 13.20% | 20.218 | 4.3886 | 46.50% |
| 12.86 | 6.8785 | 40.80% | 20.542 | 4.32 | 18.10% |
| 13.203 | 6.7002 | 7.30% | 20.862 | 4.2546 | 10.60% |
| 13.901 | 6.3655 | 31.20% | 20.997 | 4.2276 | 13.80% |
| 14.72 | 6.0132 | 5.90% | 21.579 | 4.1148 | 12.80% |
| 15.038 | 5.8865 | 20.80% | 22.461 | 3.9552 | 23.00% |
| 15.715 | 5.6345 | 41.90% | 22.679 | 3.9176 | 13.20% |
| 16.36 | 5.414 | 11.40% | 22.899 | 3.8806 | 23.40% |
| 17.778 | 4.985 | 100.00% | 23.222 | 3.8272 | 30.80% |
(28) a thermogravimetric analysis (TGA) curve of the one equivalent of succinate crystal form D shows a weight loss of 0.1218±0.005% during heating from 30.10±3 °C to 150.15±3 °C;
for example, the thermogravimetric analysis (TGA) curve of the one equivalent of succinate crystal form D shows a weight loss of 0.1218% during heating from 30.10 °C to 150.15 °C;
(29) a differential scanning calorimetry (DSC) curve of the one equivalent of succinate crystal form D has an endothermic peak with an initial temperature of 184.29±3 °C;
for example, the differential scanning calorimetry (DSC) curve of the one equivalent of succinate crystal form D has an endothermic peak with an initial temperature of 184.29 °C;
(30) the differential scanning calorimetry (DSC) curve of the one equivalent of succinate crystal form D has an endothermic peak with a peak temperature of 185.23±3 °C;
for example, the differential scanning calorimetry (DSC) curve of the one equivalent of succinate crystal form D has an endothermic peak with a peak temperature of 185.23 °C;
(31) ¹H-NMR results of the one equivalent of succinate crystal form D show that succinic acid and a free base in a sample are in a molar ratio of 1:1, with no solvent remaining; and
(32) a dynamic vapor sorption (DVS) curve of the one equivalent of succinate crystal form D shows a hygroscopic weight gain of 0.19±0.005% at 25 °C and 80% RH;
for example, the dynamic vapor sorption (DVS) curve of the one equivalent of succinate crystal form D shows a hygroscopic weight gain of 0.19% at 25 °C and 80% RH.

3. The crystal form according to claim 1, wherein one or more of the following conditions are met:
(1) the X-ray powder diffraction pattern of the one equivalent of mesylate crystal form A is substantially as shown in FIG. 5;
(2) a thermogravimetric analysis curve of the one equivalent of mesylate crystal form A is substantially as shown in FIG. 6;
(3) a differential scanning calorimetry curve of the one equivalent of mesylate crystal form A is substantially as shown in FIG. 7;
(4) a proton nuclear magnetic resonance spectrum of the one equivalent of mesylate crystal form A is shown in FIG. 8;
(5) a dynamic vapor sorption curve of the one equivalent of mesylate crystal form A is as shown in FIG. 9;
(6) the X-ray powder diffraction pattern of the crystal form D is substantially as shown in FIG. 1;
(7) a thermogravimetric analysis curve of the crystal form D is substantially as shown in FIG. 2;
(8) a differential scanning calorimetry curve of the crystal form D is substantially as shown in FIG. 3;
(9) a dynamic vapor sorption curve of the crystal form D is substantially as shown in FIG. 4;
(10) the X-ray powder diffraction pattern of the one equivalent of maleate crystal form A is substantially as shown in FIG. 10;
(11) a thermogravimetric analysis curve of the one equivalent of maleate crystal form A is substantially as shown in FIG. 11;
(12) a differential scanning calorimetry curve of the one equivalent of maleate crystal form A is substantially as shown in FIG. 12;
(13) a proton nuclear magnetic resonance spectrum of the one equivalent of maleate crystal form A is shown in FIG. 13;
(14) a dynamic vapor sorption curve of the one equivalent of maleate crystal form A is as shown in FIG. 14;
(15) the X-ray powder diffraction pattern of the one equivalent of tartrate crystal form C is substantially as shown in FIG. 15;
(16) a thermogravimetric analysis curve of the one equivalent of tartrate crystal form C is substantially as shown in FIG. 16;
(17) a differential scanning calorimetry curve of the one equivalent of tartrate crystal form C is substantially as shown in FIG. 17;
(18) a proton nuclear magnetic resonance spectrum of the one equivalent of tartrate crystal form C is shown in FIG. 18;
(19) the X-ray powder diffraction pattern of the one equivalent of citrate crystal form B is substantially as shown in FIG. 19;
(20) a thermogravimetric analysis curve of the one equivalent of citrate crystal form B is substantially as shown in FIG. 20;
(21) a differential scanning calorimetry curve of the one equivalent of citrate crystal form B is substantially as shown in FIG. 21;
(22) a proton nuclear magnetic resonance spectrum of the one equivalent of citrate crystal form B is shown in FIG. 22;
(23) a dynamic vapor sorption curve of the one equivalent of citrate crystal form B is substantially as shown in FIG. 23;
(24) the X-ray powder diffraction pattern of the one equivalent of succinate crystal form D is substantially as shown in FIG. 24;
(25) a thermogravimetric analysis curve of the one equivalent of succinate crystal form D is substantially as shown in FIG. 25;
(26) a differential scanning calorimetry curve of the one equivalent of succinate crystal form D is substantially as shown in FIG. 26;
(27) a proton nuclear magnetic resonance spectrum of the one equivalent of succinate crystal form D is shown in FIG. 27; and
(28) a dynamic vapor sorption curve of the one equivalent of succinate crystal form D is substantially as shown in FIG. 28.

4. A pharmaceutical composition, comprising the one equivalent of mesylate crystal form A of the compound represented by formula I, crystal form D of the compound represented by formula I, one equivalent of maleate crystal form A of the compound represented by formula I, one equivalent of tartrate crystal form C of the compound represented by formula I, one equivalent of citrate crystal form B of the compound represented by formula I, or one equivalent of succinate crystal form D of the compound represented by formula I according to any one of claims 1-3, and one or more pharmaceutically acceptable excipients.

5. Use of a substance X in the preparation of an ALK inhibitor, wherein the substance X is the one equivalent of mesylate crystal form A of the compound represented by formula I, crystal form D of the compound represented by formula I, one equivalent of maleate crystal form A of the compound represented by formula I, one equivalent of tartrate crystal form C of the compound represented by formula I, one equivalent of citrate crystal form B of the compound represented by formula I, or one equivalent of succinate crystal form D of the compound represented by formula I according to any one of claims 1-3, or the pharmaceutical composition according to claim 4.

6. Use of a substance X in the treatment or prevention of cancer, wherein the substance X is the one equivalent of mesylate crystal form A of the compound represented by formula I, crystal form D of the compound represented by formula I, one equivalent of maleate crystal form A of the compound represented by formula I, one equivalent of tartrate crystal form C of the compound represented by formula I, one equivalent of citrate crystal form B of the compound represented by formula I, or one equivalent of succinate crystal form D of the compound represented by formula I according to any one of claims 1-3, or the pharmaceutical composition according to claim 4; preferably, the cancer is selected from the group consisting of: anaplastic large cell lymphoma, non-small cell lung cancer, diffuse large B-cell lymphoma, inflammatory myofibroblastoma, neuroblastoma, anaplastic thyroid cancer, rhabdomyosarcoma, breast cancer, colorectal cancer, esophagus squamous cell carcinoma, and renal cell carcinoma.
